# EUROPEAN PATENT APPLICATION

(11) **EP 3 342 854 A1**
(43) Date of publication of application: **04.07.2018**
(21) Application number: 15790164.6
(22) Date of filing: 26.08.2015
(51) Int. Cl.: C12N 1/36, A61K 39/085

(54) **MUTANT STRAINS OF STAPHYLOCOCCUS AUREUS WITH MULTIPLE INACTIVATED TCS SYSTEMS**

(71) Applicant: Universidad Pública De Navarra, 31006 Pamplona, Navarra (ES); Consejo Superior de Investigaciones Científicas (CSIC), 28006 Madrid (ES)
(72) Inventor: VILLANUEVA SAN MARTÍN, Maite, 31192 Mutilva (Navarra) (ES); GARCÍA MARTÍNEZ, Begoña, 31192 Mutilva (Navarra) (ES); VALLE TURRILLAS, Jaione, 31192 Mutilva (Navarra) (ES); PENADÉS CASANOVA, José R., 31192 Mutilva (Navarra) (ES); MARTÍ JIMÉNEZ, Miguel, 31192 Mutilva (Navarra) (ES); TOLEDO ARANA, Alejandro, 31192 Mutilva (Navarra) (EE); LASA UZCUDUN, Iñigo, 31192 Mutilva (Navarra) (ES)
(74) Representative: Elzaburu S.L.P.
(86) International application number: PCT/ES2015/070636
(87) International publication number: WO 2017/032909

(57) **Abstract**

Novel strains of Staphylococcus aureus and uses thereof. The invention provides mutant strains of *S. aureus* devoid of all non-essential two-component signal systems (TCSs) and even the essential walKR system, the intermediate mutants devoid of a number of TCSs and their uses as research and biotechnological platform. The mutants devoid of all non-essential TCSs are viable, stable under laboratory growing conditions, genetically manageable and capable to express heterologous proteins. Having them allows individual analysis and targeting of each TCS, its individual complementation and obtaining information about the sensing systems that can be useful for biotechnological applications based or directed to S. *aureus,* such as the identification of novel antimicrobials.

## Description

### FIELD OF THE INVENTION

The present invention relates to genetically modified strains of *Staphylococcus aureus (S. aureus).* More particularly, the invention relates to novel strains wherein all the genes encoding two-component systems (TCSs) have been sequentially mutated (by deletion of their corresponding genes and the conditional expression of the only essential two-component system) so that all TCS can be inactive simultaneously, to strains maintaining only the essential two-component system and to the intermediate deletion mutants generated for obtaining the previously mentioned mutant strains, as well as to the applications of said strains for research and biotechnological uses.

### BACKGROUND OF THE INVENTION

Bacteria depends on its capacity to sense environmental conditions and transmit this information to the intracellular regulatory machinery to respond to external signals such as nutrient availability (especially, iron), inhabit in different ecological niches and survive under different conditions (particularly, those related to osmolarity of high density of population) or in the presence of various stresses (pH, oxygen reactive radicals, nitrogen pressure...). Gene expression, enzyme activity or growth rate must be adapted to such conditions in order to survive to environmental changes.

The advantages and selective pressures that influence the number of sensorial systems present in bacteria remains poorly understood. The genome of a single bacterial species usually encodes for multiple signal transducers, being this number proportional to the genome size, the diversity of environments in which organisms live and the complexity in cellular differentiation. Bacteria inhabiting relatively stable host environments, such as obligate intracellular parasites, encode for few or even none signalling systems, while bacteria able to live ubiquitously in varied of environments such as *Pseudomonas* and bacteria with a complex lifestyles such as *Myxococcus xanthus* and *Nostoc punctiforme* encode high numbers. A question that arises from these data is whether a minimum number of signalling systems is necessary to maintain growth in free-living bacteria.

Most signalling proteins can be mutated individually under laboratory conditions, and only few of them have been shown to be essential for the maintenance of basic cellular functions (Skerker *et al.,* 2005; Toledo-Arana *et al.,* 2005). It is unknown how dependent on the signalling machinery bacteria might be if they are grown in an environment where conditions remain unchanged.

The simplest signalling module in bacteria consists of a multidomain protein that contains both the sensing and the output regulatory domains. A more complex design involves a membrane-embedded sensor protein and a cytoplasmic response regulator, which together constitute a two-component system (TCS) (Kofoid and Parkinson, 1988). Two-component signal transduction proteins are the prevalent mechanism for transmitting changes in environmental conditions to the proteins and other components that respond to signals. In a typical TCS, detection of the environmental signal by the membrane-embedded sensor domain, which is a histidine kinase (HK), results in the auto-phosphorylation of a conserved histidine residue. Subsequently, the phosphoryl group is transferred to a conserved aspartate residue in a cytoplasmic protein, the cytoplasmic regulator partner (RR), resulting in conformational changes that influence its regulatory activity (Stock *et al.,* 2000). Thus, phosphorylation of the regulator partner activates said protein and results in a modified enzymatic activity or different interaction possibilities with other proteins or DNA.

TCSs are widely distributed in the bacterial kingdom and constitute the most common and predominant means by which bacteria sense and respond to environmental signals. A variety of other different sensing systems have been described in bacteria, including quorum sensing systems, c-di-GMP-mediated signal transduction systems and redox sensors.

The term 'signal transduction' has been typically reserved for the TCSs, however, according to the census of signal transduction proteins encoded in bacterial genomes at the NCBI (http://www.ncbi.ntm.nih.gov/Complete Genomes/SianalCensus.html) (Galperin, 2010), S. *aureus* contains additional signal transduction systems that included: (i) GdpS, the only GGDEF domain protein with a conserved GGEEF motif; (ii) a c-di-AMP cyclase (Dac) and phosphodiesterase (GpdP) and (iii) a Ser/Thr protein-kinase (Stk1) and two Ser/Thr protein-phosphatases (Stp 1 and RsbU). Among these signal transducers, only GdpS and Stk1 contain transmembrane sensor domain capable to respond to extracelular signals. Biochemical analysis showed that GdpS does not synthesize c-di-GMP (Holland *et al.,* 2008). Thus, S. *aureus* lacks the c-di-GMP signaling pathway and therefore, the role of this protein in signal transduction remains unknown. In contrast, stk1 contains three PASTA domains in the carboxy-terminal part that are suggested to interact with the cell-wall (Maestro *et al.,* 2011). Recent studies have indicated that Stk1 phosphorylates GraR to modulate D-Ala content in the teichoic acids and the cell-wall charge (Fridman *et al.,* 2013). In the absence of GraR, Stk1 dependent remodelation of cell-wall is compromised. Hence, Stk1 signaling pathway depends on the presence of the TCS.

According to gene evolution studies, the collection of TCS contained in a single bacterium derives from horizontal gene transfer or gene duplication (Aim *et al.,* 2006). The newly acquired TCS subsequently diverge into the recognition of a different pathway to provide a substantial selective advantage (Capra *et al.,* 2012; Podgornaia and Laub, 2013). Detailed system-level analysis with purified proteins have shown that this adaptive differentiation occurs through changes in small number of residues in the protein-protein interaction interface that provide strong kinetic preference to the HK for transferring or removing the phosphate from the cognate RR pair depending on its activation state. General interest in determining whether a similar specificity in the TCS signaling exists *in vivo* is extremely high. Indeed, the similarity of TCS signaling proteins raises the possibility of cross-talk between different pathways, which is defined as the communication between two pathways that, if eliminated, would leave two distinct, functioning pathways. Although cross-talk between distinct pathways, in general, must be kept to a minimum, under some conditions, it may be advantageous to an organism to permit or use cross-talk as a means of either integrating multiple signals or diversifying the response to a single input, a benefitial situation which is usually known as cross-regulation and that cannot be discarded in bacterial systems. However, technical difficulties due to the presence of dozens of TCS in the same bacterial cell have impaired to answer this question through global system-level approaches. Instead, cross regulation studies have been focused on specific TCS partners (some examples are described in Siryaporn and Goulian, 2008; Guckes *et al.,* 2013). The general conclusion of these studies is that cross talk between non-cognate HK-RR pairs is possible, but rare. Still, additional studies using system-level analysis are necessary to confirm the scarcity of cross talk between non-cognate HK and RR *in vivo.*

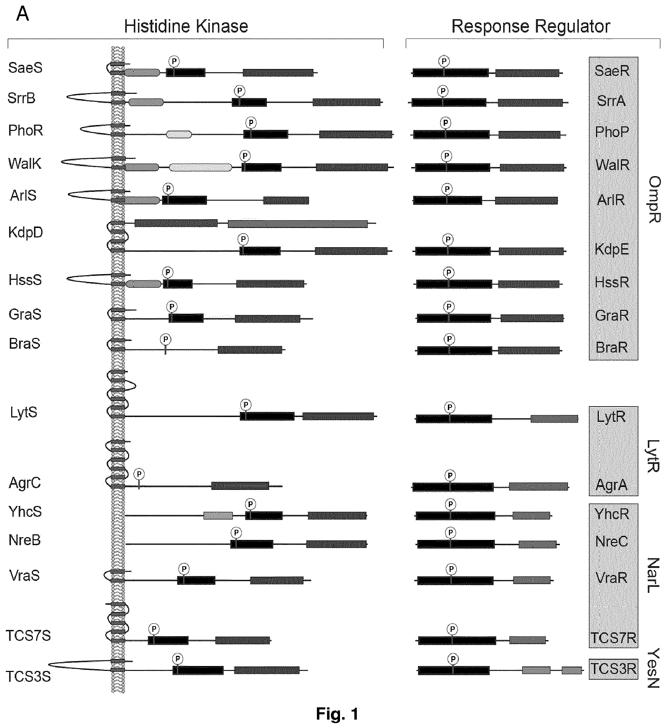

More than 4000 genomes of S. *aureus* strains have been completely sequenced. Among them, the genome sequence of 70 strains can be considered established (as can be seen, for instance, in the section of the web site of PATRIC (Pathosystems Resource Integration Center) corresponding to S. *aureus*: https://www.patricbrc.org/portal/portal/patric/GenomeList?cType=taxon&cId=1279& displayMode=Complete&dataSource=PATRIC&pk=. This allows to conclude that the core genome of *S*. *aureus* strains contains 16 TCSs, which are very well conserved among different strains. All of them are HK-RR pairs that are located together and transcribed together in the same operon without "orphan" HK or RR proteins (Lasa *et al.,* 2011; Ruiz de Los Mozos *et al*., 2013). One of them, VicRK (also known as *yycGF* or *walKR),* is considered to be essential for *S*. *aureus* viability. The domain organization and topology of the 16 *S*. *aureus* HKs and RRs is represented in Fig.1. A list of the TCSs present in *S*. *aureus* genome and their function can be found in Table 1 below.

**Table 1: Description of the sixteen TCSs present in S. aureus genome plus TCSs of the SCC-mec type element**

| **TCS (synonimous)** | **Histidine Kinase** | **Response Regulator** | **Function** | **References** |
|---|---|---|---|---|
| *walKR (yycGF*/ *vicKR)* | WalK | WalR | Viability and virulence | (Martin *et al*., 1999) (Dubrac and Msadek, 2004) (Delaune *et al.*, 2012) |
| *tcs3 (MW0199-MW0198)^{a}* | TCS3S | TCS3R | (Unknown) | |
| *lytSR* | LytS | LytR | Autolysis and biofilm | (Brunskill and Bayles, 1996) (Rice *et al*., 2007) (Sharma-Kuinkel *et al*., 2009) |
| *graXRS* | GraS | GraR | CAMP resistance | (M Li *et al*., 2007) |
| *saePQRS*^{#} | SaeS | SaeR | Virulence factor production | (Giraudo *et al*., 1994) |
| *tcs7 (MW1207-MW1208)^{b}* | TCS7S | TCS7R | (Unknown) | |
| *arlRS* | ArlS | ArlR | Virulence factor production, autolysis, biofilm and agglutination | (Fournier and Hooper, 2000) (Fournier *et al*., 2001) (Toledo-Arana et *al*., 2005) |
| *srrAB* | SrrB | SrrA | Virulence and biofilm under anaerobic conditions and NO detoxification | (Yarwood *et al*., 2001) (Pragman *et al*., 2004) (Ulrich *et al*., 2007) (Kinkel *et al.,* 2013) |
| *phoPR* | PhoR | PhoP | (Unknown) | |
| *airSR (yhcSR)* | YhcS | YhcR | Anaerobic respiration and cell wall metabolism | (F Sun *et al*., 2012) (Haipeng Sun *et al*., 2013) |
| *vraSR* | VraS | VraR | Cell wall stress stimulon (CWSS) regulation | (Kuroda *et al*., 2003) |
| *agrBDCA*^{#} | AgrC | AgrA | Quorum-sensing, oxidative stress resistance and virulence | (Dunman *et al*., 2001) (Queck *et al*., 2008) |
| *kdpDE* | KdpD | KdpE | Quorum-sensing, virulence and c-di-AMP sensing | (Xue *et al*., 2011) (Zhao *et al.,* 2010) (Corrigan et *al*., 2013) |
| *hssRS* | HssS | HssR | Heme sensing and virulence | (Torres *et al*., 2007) |
| *nreABC*^{#} | NreB | NreC | Nitrogen regulation | (Schlag *et al*., 2008) |
| braRS | BraS | BraR | Antibiotic resistance and cell envelope damage sensing | (Kolar *et al*., 2011) |
| *kdpED-*like*** | | | ATP-dependent potassium transport | (Hanssen and Ericson Sollid, 2006) |

| | | | | |
|---|---|---|---|---|
| * Present in certain *S*. *aureus* strains that possess the SCC-*mec* type II element ^{#} This Table 1 describes the whole operon, including other genes present in the operon. In the second and third column can be found the names of the histidine kinase and the response regulator forming the TCS ^{a} TCS whose genes are annotated in the genome of the strain MW2 as MW0199-MW0198. A general name for this TCS has not been proposed yet ^{b} TCS whose genes are annotated in the genome of the strain MW2 as MW1207-MW1208. A general name for this TCS has not been proposed yet | | | | |

An additional TCS homologous to the chromosomal copy of *kdpDE* (*kdpED-*like) is carried in the SCCmec mobile element of some methicillin resistant strains, namely in the SCCmec type II element that is carried by a limited number of *S*. *aureus* isolates.

*S*. *aureus* is a regular commensal of the skin, skin glands, intestinal, genitourinary and upper respiratory tracts of animals and human population but its main ecological niche and reservoir is the human nose (Weidenmaier *et al.,* 2012). When S. *aureus* traverses the epithelial barrier and gains access to internal tissues, it can infect almost any organ and cause a broad spectrum of infections including abscesses, pneumonia, endocarditis, osteomyelitis, sepsis and infections associated with foreign-body implants (Lowy, 1998). It is well known its ability to adhere to the surfaces of catheters, prostheses, heart valves, clinical meshes and any kind of medical implants, and to grow on those surfaces giving rise to communities (biofilms) which are the cause of chronical infections associated to the implant that can be controlled only by removing the contaminated implant. Additionally to its versatility as a pathogenic agent, *S*. *aureus* infections are clinically important due the existence of multidrug resistant strains. Among such strains, methicillin-resistant *S*. *aureus* (MRSA) strains are particularly remarkable because they mean a serious public health concern worldwide, not only because MRSA infection has become one of the most common hospital-acquired infections, but also because new strains have emerged in the community that are capable of causing severe infections in otherwise healthy people. Under such circumstances, in spite of the broad repertoire of available anti-microbial compounds, the mortality rate associated to bacteraemias caused by *S*. *aureus* in developed countries remains to be 20-40%.

TCS have been thoroughly studied in *S*. *aureus* aiming to gain insight into the role of this system in host-pathogen interactions and some TCS represent the prototype of regulatory pathways in gram positive bacteria (Novick:1993vj). Also, due to the increasing prevalence of *S*. *aureus* multidrug resistant strains, TCS have attracted significant attention as new putative targets for antibiotic development (David and Daum, 2010). However, our understanding of the role of TCS in the *S*. *aureus* biology is limited to phenotypes associated with the absence of single TCS, without having considered possible compensatory effects or interferences with other members of *S*. *aureus* sensor system. Thus, for instance, international patent application WO 99/32657 describes hypersensitive mutant cells containing a mutant gene corresponding to histidine-kinase genes (HK) whose proteins belong to the group of the TCSs, as well as methods for screening antibacterial agents using such hypersensitive cells. Only examples of mutant where only one gene has been deleted are described.

Delaune et al. (Delaune *et al*., 2012) generated a *S*. *aureus* strain producing a constitutively active form of WaIR through a phosphomimetic amino acid replacement (WalRc, D55E). The WaIR mutant strain shows increased biofilm formation capacity and higher alpha-hemolytic activity, but a reduced virulence.

Boyle-Vavra *et al.* (Boyle-Vavra *et al*., 2013) mentioned the *vraT* gene (also known as *yvqF)* as target to make MRSA strains susceptible to methicillin again. VraTSR seems to comprise a three component regulator system that facilitates resistance to agents directed to the cell wall.

Patent Applications Nos. WO0071555, WO0068140, WO9936508 and EP0911406 refer to different polynucleotides which include coding sequences and to their corresponding polypeptides (denominated in the applications 509RR, 623RR, as well as certain histidine kinases) of the TCS family, isolated or preferably isolated from *S*. *aureus,* their use as targets for the development of antibiotics and the corresponding screening system. Each application refers only to a particular polynucleotide and polypeptide.

US patent US6406889B1 refers to a polynucleotide useful as probe for isolating cDNA and genomic clones encoding an histidine kinase denominated 509HK which is said to be related by amino acid sequence homology to polypeptide YhcY and which is considered useful for treating diseases caused by *S*. *aureus,* diagnosing the step and type of infection and the response of an organism to drugs.

Patent applications Nos. WO9723506 and WO9723505 also refer to a particular *S*. *aureus* polynucleotide and its corresponding protein, which is closely related to the protein ResD of *Bacillus subtilis.* Its inhibition prevents bacterial infection, avoiding that bacteria can produce factors necessary for pathogenesis.

As each of the above documents focuses on a particular TCS gene, without considering compensatory effects or interferences with other members of the *S*. *aureus* system, the biological process regulated by each individual TCS and their contribution to the sensing system of *S*. *aureus* remain poorly understood. Thus, it is unpredictable how the inhibition of the TCS signalling cascade would affect the bacterial biology.

It would be very desirable to have available a strain of *S*. *aureus* where the impact of acting on a particular TCS could be assessed without taking the risk of having the effect of the action minimized or hidden by the cross-talk and/or the compensatory action of other active TCS. Such strain would be very useful as a research tool both for improving the knowledge of TCS function and for the identification of antibiotics targeting a particular TCS. Such strain would be particularly useful if it were stable and could grow in laboratory conditions and if it were easily ameneable to genetic manipulations, so that it could be used, for instance, for expressing foreign proteins of interest, thus facilitating any additional industrial applicability that could be envisioned for the strain.

The present invention provides a solution to such problems.

### SUMMARY OF THE INVENTION

The present invention refers to a mutant strain of *S*. *aureus* whose genome is devoid of all TCS genes, except for those corresponding to the system that is essential for viability, VicRK (also known as WalKR), which can be in the mutant strain under the control of their natural transcriptional regulatory elements (*S*. *aureus* ΔXV) or having been put under the control of an inducible promoter that allows its controlled conditional expression (*S. aureus* ΔXVI). In the former case, the strain can also harbor a vector to express ectopically the WaIR response regulator (*S. aureus* ΔXVI*). The present invention also relates to each one the intermediate mutants used for obtaining the mutant strains mentioned before, as well as to any other mutant strain of *S*. *aureus* which is devoid of all but one TCS gene. The research and biotechnological uses of such strains are also encompassed in the scope of the present invention.

Thus, the present invention, in a first aspect, relates to a mutant strain of *Staphylococcus aureus* (*S. aureus*) characterized in that it is devoid of all TCS genes except for the one corresponding to the response regulator of the walKR system, *walR,* and, optionally, also except for the gene corresponding to the histidine kinase of the walKR system, *walK.* The genes *walR* and *walK* can be operatively linked to an inducible promoter and/or *walR* can be ectopically expressed, constitutively or inducibly.

In a second aspect, closely related to the first one and sharing with it several possible embodiments, the present invention relates to a mutant strain of *Staphylococcus aureus* (*S. aureus*), wherein any combination of at least two, three, four, five, six, seven, eight, nine, ten, eleven, twelve, thirteen, fourteen, fifteen or sixteen TCSs selected from the following group have been deleted or inactivated: yhcSR; the TCS comprised of the components expressed either i) by the genes *MW0199-MW0198* of *S*. *aureus* strain MW2 (tcs3), or ii) by the genes *SAOUHSC_00185-SAOUHSC_00184* of *S*. *aureus* strain 8325, or iii) by their corresponding orthologous genes of another *S*. *aureus* strain*; lytSR; graRS; saeRS;* the TCS comprised of the components expressed either i) by the genes *MW1207-MW1208* of strain MW2 (tcs7), or ii) by the genes *SAOUHSC_01313-SAOUHSC_01314* of *S*. *aureus* strain 8325, or iii) by their corresponding orthologous genes of another *S*. *aureus* strain; *hssRS; nreBC; braRS; kdpDE; vraSR; phoPR; arlRS; agr; srrAB* and *walKR.* The first fifteen systems are all the non-essential TCSs in *S*. *aureus* strains. For those strains that have the additional *kdpED-*like, which is also a non-essential TCS (see Table 2), the mutant can also lack the kdpED-like two component system and can be a mutant of a methicillin-resistant *S*. *aureus* strain. The strains MW2 and 8325 in particular can be also suitable parental strains.

Table 2 below shows the genes of strains MW2 and 8325 that are contained in the operons where the components (HK and RR) of each one of the TCSs are encoded. The first column of said table names each TCS including a number that indicates its relative position with regard to the other TCS in the genome of the mentioned strains MW2 and 8325.

**Table 2.- Genes of S. aureus MW2 and 8325 contained in the operon corresponding to each particular TCS**

| **TCS** | **Name** | ***S. aureus* MW2** | ***S. aureus* 8325** |
|---|---|---|---|
| *TCS1* | *vicRK*/*yycFG*/*wa*/*KR* | *MW0018-MW0019* | *SAOUHSC_00020-00021* |
| TCS2 | *KdpED like* | - | |
| TCS3 | *(tcs3)* | *MW0199-MW0198* | *SAOUHSC_00185-00184* |
| TCS4 | *lytSR* | *MW0236-MW0237* | *SAOUHSC_00230-00231* |
| TCS5 | *graRS* | *MW0621-MW0622* | *SAOUHSC_00665-00666* |
| TCS6 | *SaeRS* | *MW0667-MW0668* | *SAOUHSC_00714-00715* |
| TCS7 | *(tcs7)* | *MW1207-MW1208* | *SAOUHSC_01313-01314* |
| TCS8 | *ArlRS* | *MW1304-MW1305* | *SAOUHSC_01419-01420* |
| TCS9 | *SrrAB* | *MW1445-MW1446* | *SAOUHSC_01585-01586* |
| TCS10 | *PhoPR* | *MW1636-MW1637* | *SAOUHSC_01799-01800* |
| TCS11 | *YhcSR* | *MW1789-MW1790* | *SAOUHSC_01980-01981* |
| TCS12 | *VraRS* | *MW1824-MW1825* | *SAOUHSC_02098-02099* |
| TCS13 | *Agr* | *MW1960-MW1962* | *SAOUHSC_02263-02264* |
| TCS14 | *KdpDE* | *MW2002-MW2003* | *SAOUHSC_02315-02316* |
| TCS15 | *HssRS* | *MW2282-MW2283* | *SAOUHSC_02642-02643* |
| TCS16 | *NreBC* | *MW2313-MW2314* | *SAOUHSC_02675-02676* |
| TCS17 | *BraRS* | *MW2544-MW2545* | *SAOUHSC_02955-02956* |

A preferred embodiment of said aspect of the invention, compatible with all the previously mentioned embodiments, is that one wherein the mutant strain is one in which the following two-component signal systems are deleted: *yhcSR;* the TCS comprised of the components expressed either i) by the genes *MW0199-MW0198* of *S*. *aureus* strain MW2 (tcs3), or ii) by the genes *SAOUHSC_00185-SAOUHSC_00184* of *S*. *aureus* strain 8325, or iii) by their corresponding orthologous genes of a another *S*. aureus strain; *lytSR; graRS; saeRS;* the TCS comprised of the components expressed either i) by the the genes *MW1207-MW1208* of strain MW2 (tcs7), or ii) by the genes *SAOUHSC_01313-SAOUHSC_01314* of *S. aureus* strain 8325, or iii) by their corresponding orthologous genes of another *S.* aureus strain; *hssRS; nreBC; braRS; kdpDE; vraSR; phoPR; arlRS; agr,* and *srrAB.* This mutant is called in the present application, generically, ΔXV. Special particular embodiments of this one are: the mutant of the MW2 strain deposited in the Spanish Type Culture Collection with the accession number CECT 8756, or the mutant of the 8325 strain deposited in the Spanish Type Culture Collection with the accession number CECT 8755.

The above mentioned ΔXV mutant strains are mutant strains which are devoid of all TCS except for walKR, that is, they are mutant strains that are devoid of all non essential TCS. It can be said that this is a particularly preferred embodiment of the present invention: a *S. aureus* mutant strain that is devoid of all non-essential TCS, because all TCS except for walKR have been rendered inactive, by deletion or by another inactivating mutation including those that prevent the transcription from the corresponding operon, the translation of the mRNA or even mutations that give rise to inactive HK and/or RR proteins. Therefore, ΔXV mutants obtained from a parental strain with fifteen non-essential TCS (not having the kdpED-like TCS which is present in some SCC-*mec* elements) are embodiments of the first aspect of the invention and also of the second one, as they can be intermediate mutants for obtaining, for example, mutants in the walKR system.

Other preferred embodiments of the invention are those mutants wherein, additionally, the *walKR* TCSs can be selectively turned off. Therefore, also embodiments of the invention are mutant strains of *S. aureus* which can be special cases of any of the embodiments of mutants previously mentioned, wherein the chromosome genes corresponding to the *walKR* TCS are operatively linked to (that is, are under the transcriptional control of) a first inducible promoter and, optionally, WaIR is expressed ectopically under the control of a constitutive or a second inducible promoter. This mutant is called in the present application, generically, ΔXVI. Among such mutants, one particular specific embodiment is the mutant of the MW2 strain and the mutant 8325 strain, wherein all the fifteen two-component signal systems mentioned in the previous paragraph are deleted from the chromosome and the bacterial chromosome genes encoding proteins belonging to the *walKR* system are under the control of an inducible Pspac promoter, so that its expression is inducible by IPTG. Said mutants, called MW2 ΔXVI and 8325 ΔXVI, have been deposited in the Spanish Type Culture Collection with the accession numbers CECT 8915 (MW2 ΔXVI) and CECT 8916 (8325 ΔXVI). Optionally, a plasmid allowing the ectopic expression of WaIR can also be present in the ΔXVI mutant (ΔXVI* mutant strain), in which WaIR expression is under the control of a second inducible promoter or, preferably, under a constitutive promoter, so that, in the absence of the inductor of the promoter that controls the expression of the *walKR* system, the ΔXVI* strain only grows when WaIR is expressed from the plasmid.

Also the mutants generated from any one of the above mutants of the present invention which ectopically express one or both components (HK and/or RR) of one or more than one TCS are embodiments of the present invention.

Any of the above defined mutant strains of *Staphylococcus aureus* are considered mutant strains of the present invention.

A third aspect of the present invention is the use of any of the mutant strains of *S. aureus* of the present invention, specially the use as research tool or for biotechnological application. Different embodiments of this aspect of the invention are: the use of any of the mutant strains of the present invention for the expression of a heterologous protein, a chimeric protein and/or a fusion protein, the use for identifying compounds capable of inhibiting one or more two-component systems (which additionally might be implied the use for identifying candidates to antibiotics or antimicrobial compounds), the use for identifying promoters whose expression depends on environmental signals, the use for developing biosensor tracks for environmental signals, or the use of one of the mutants devoid of the fifteen non-essential TCS (or sixteen, if kdpED-like was initially present), optionally also with the *walKR* system deleted or selectively inactivated, in assays wherein the phenotype of the mutant strain is compared with the phenotype of the same mutant strain except for that it ectopically expresses a) one or both of the two components, histidine kinase and response regulator, of a TCS selected from the group of *yhcSR;* the TCS comprised of the components expressed either i) by the genes *MW0199-MW0198* of *S. aureus* strain MW2 (tcs3), or ii) by the genes *SAOUHSC_00185-SAOUHSC_00184* of *S. aureus* strain 8325 (tcs3), or iii) by their corresponding orthologous genes of a another *S. aureus* strain; *lytSR; graRS; saeRS;* the TCS comprised of the components expressed either i) by the genes *MW1207-MW1208* of strain MW2 (tcs7), or ii) by the genes *SAOUHSC_01313-SAOUHSC_01314* of *S. aureus* strain 8325 (tcs7), or iii) by their corresponding orthologous genes of another *S. aureus* strain; *hssRS; nreBC; braRS; kdpDE; vraSR; phoPR; arlRS; agr, srrAB* and *walKR,* and/or b) other polypeptide or groups of polypeptides, or any other gene product (such as an RNA, which can be an iRNA or a shRNA), which is an experimental approach for analyzing phenotypes associated to different TCS or different situations and their possibilities of complementation that is specially enabled by the present invention.

Yet another aspect of the present invention, closely related with the previous one, is a method for identifying a compound as a blocker or inactivator of the walKR system, using the ΔXV, ΔXVI or ΔXVI* strains, which comprises the steps of:
a) culturing the ΔXV, ΔXVI or ΔXVI* mutant strain of *S. aureus* under conditions wherein the WalKR should be expressed,
b) comparing the growth rate of the mutant strain in the presence of the compound with the growth rate in the absence of the compound,
c) identifying the compound as a blocker or inactivator of the walKR system when the growth rate is reduced in the presence of the compound,
d) optionally, checking whether the compound is a specific blocker of the expression of the walKR system by verifying that the reduction of the growth rate is observed when the strain or strains cultured in the presence of the compound is a *S.* aureus wild type strain and it is not observed when the strain or strains cultured in the presende of the compound is a *S. aureus* mutant strain wherein the walKR system is expressed under the control of a promoter that is different from the natural one and that gives rise to the expression of walKR under the culture conditions of steps a) and b),
e) optionally, verifying that the blocking or inactivation cannot be reverted by the expression of another TCS or by a combination of TCSs by checking that the reduction of the growth rate is not reverted when the strain or strains cultured in the presence of the compound is any one of the mutant strains which are devoid of at least one or any combination of two, three, four five, six, seven, eight, nine, ten, eleven, twelve, thirteen, fourteen or fifteen of the non-essential TCS (or even sixteen if kdpE-like is present in the parental strain), the parental strain corresponding to the mutant strain used in step a) and/or any other wild type strain,
f) optionally and additionally, identifying the compound as a candidate to antimicrobial compound when step d) has been carried out and the reduction of the growth rate is not reverted at least in the parental strain or any other wild type strain.

Yet another aspect of the present invention, closely related with the previous one, is a method for identifying physical or chemical signals sense by two-component systems in assays wherein the expression of a reporter gene under the control of a promoter regulated by a two-component system is compared with the phenotype of the same mutant strain except for that it ectopically expresses a TCS selected from the group of yhcSR; the TCS comprised of the components expressed either i) by the genes MW0199-MW0198 of *S. aureus* strain MW2 (tcs3), or ii) by the genes *SAOUHSC_00185-SAOUHSC_00184* of *S. aureus* strain 8325, or iii) by their corresponding orthologous genes of a another S. *aureus* strain; lytSR; graRS; saeRS; the TCS comprised of the components expressed either i) by the genes MW1207-MW1208 of strain MW2 (tcs7), or ii) by the genes SAOUHSC_01313-SAOUHSC_01314 of *S. aureus* strain 8325, or iii) by their corresponding orthologous genes of another *S. aureus* strain; hssRS; nreBC; braRS; kdpDE; vraSR; phoPR; arlRS; agr; srrAB, kdpED-like and walKR.

Another additional aspect of the present invention is the use of any mutant strain of the present invention, particularly the ΔXV, ΔXVI or ΔXVI* strains, as candidate to living attenuated strain for vaccination purposes.

### BRIEF DESCRIPTION OF THE DRAWINGS

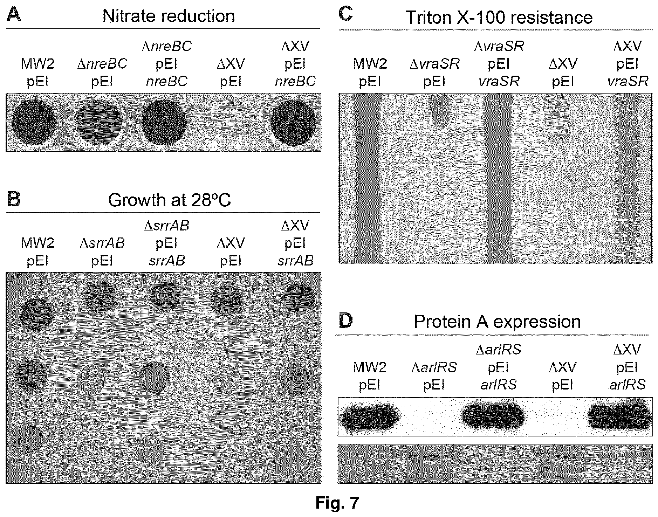

**Fig. 1****.** Organization of the 16 *S. aureus* TCSs. Domain organization and topology of the 16 *S. aureus* histidine kinases and response regulators. Domains are based on NCBI Conserved Domain search and transmembrane helix (TMH) domains are predicted by TMHMM Server v. 2.0. HKs are classified based on their DHp domain and RRs are classified based on their output domain. Black boxes correspond to the domains where the phosphorylable histidines (represented by "P" letters rounded by circles): the histidine phosphotransfer domains of Histidine Kinases and the receiver domains of the Response Regulators. The vertical band on the right of the histidine kinases names represents the cell membrane, and the dark boxes perperdicular to it correspond to transmembrane helix domains.

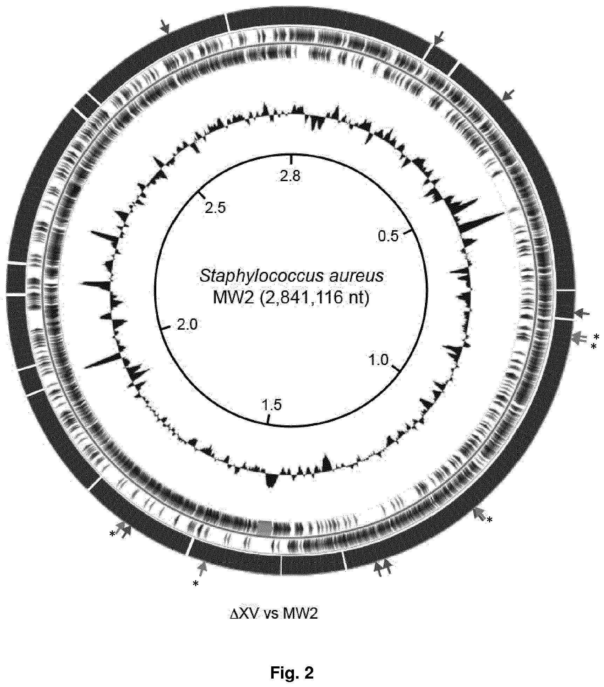

**Fig. 2****.** Schematic circular representation of the *S. aureus* MW2 ΔXV genome sequence, based on Illumina™ Next Generation Sequencing. The most internal circle is the positive chain of the circular genome. Peaks protruding from the intermediate circumference represent the GC content. The external circumference, drawn with a wider line, is intended to represent the results of the BlastN analysis of MW2 ΔXV vs MW2 wild type, so that the fifteen white lines interrupting the circumference represent the position of the fifteen TCS deletions, and arrows mark the positions of the SNPs produced spontaneously during the mutagenesis process in MW2 (darker arrows, red in the original) and 8325 strains (lighter arrows, light blue in the original, and marked with asterisks). The two collections of arrowheads surrounded by the most external circumference indicate the sense of transcription of the operons: clockwise in the case of the most external collection and anticlockwise in the case of the most internal collection of arrowheads.

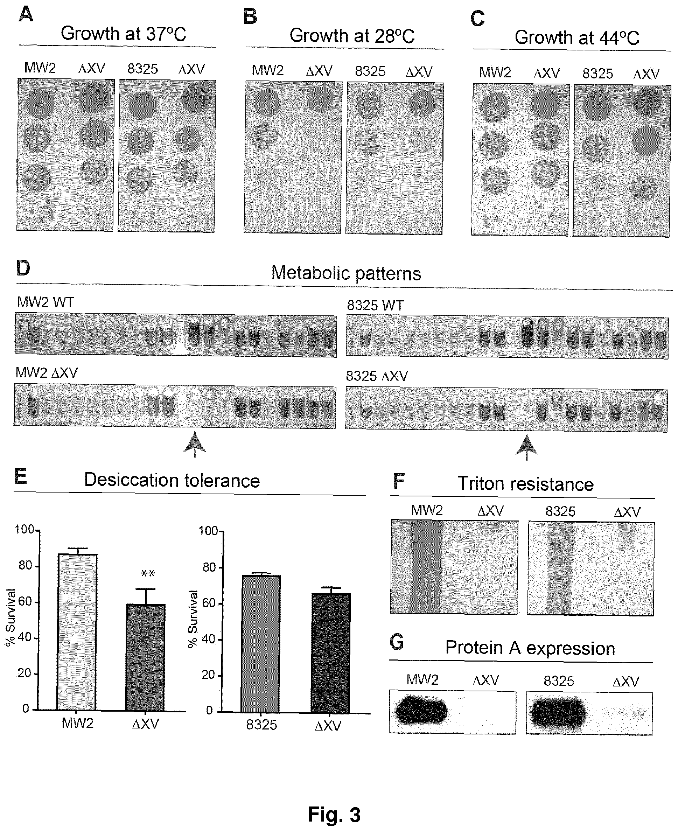

**Fig. 3****.** Phenotypic analyses of *S. aureus* MW2 and 8325 wild type strains and their corresponding ΔXV.
   - Panels A, B, C: Photographs of agar plates with surviving bacteria after overnight incubation at 37ºC (panel A), 28ºC (panel B), 44ºC (panel C). Dilutions of inoculation (from top to bottom): sample of overnight non-diluted culture (top), and dilutions 10⁻², 10⁻⁴, 10⁻⁶ (bottom plates).
   - Panel D: Metabolic pattern of wild type (WT) and ΔXV mutant according to API Staph galleries. Arrows indicate the position of the metabolic assays where differences between wild type and ΔXV mutants appear.
   - Panel E: Bar charts representing the desiccation tolerance after 21 days, indicated as the survival percentage after overnight culturing (initial numbers) and air-drying, storing at room temperature, rehydration and determination of remaining viable cells.
   - Panel F: Photographs of *S. aureus* strains MW2, MW2 ΔXV, 8325 and 8325 ΔXV, as indicated, growing on Triton X-100 concentration gradient plates.
   - Panel G: Analysis of Protein A expression by Western blotting.

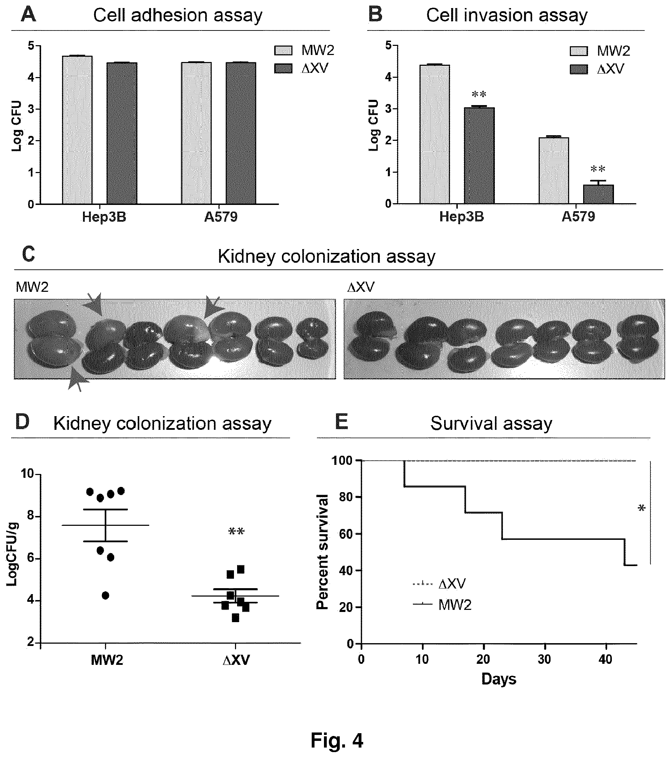

**Fig. 4****.** Phenotypic analyses of interaction with cells of the MW2 and MW2 ΔXV strains.
   - Panels A and B: (A) Adhesion and (B) invasion of *S. aureus* MW2 wild type and ΔXV mutant to human hepathocytes (Hep3B) and human lung epithelial cells (A549). Bar charts representing the logarithm of the colony forming units (CFU) counted after an adhesion assay to cells seeded in wells (panel A) and 1 hour infection, or after gentamicin assay (panel B).
   - Panels C and D. Seven mice per group were infected with 10⁷ cfu of bacteria by eye vain injection. Kidneys were removed aseptically and homogenized in PBS for enumeration of viable bacteria. Bacterial load, expressed as logarithm of CFUs, is represented in panel D for each mouse.
   - Panel E. Percentage of mice survival depending of the days after infection with 10⁷ cfu of bacteria by eye vain injection. Survival was monitored every day for 7 weeks.

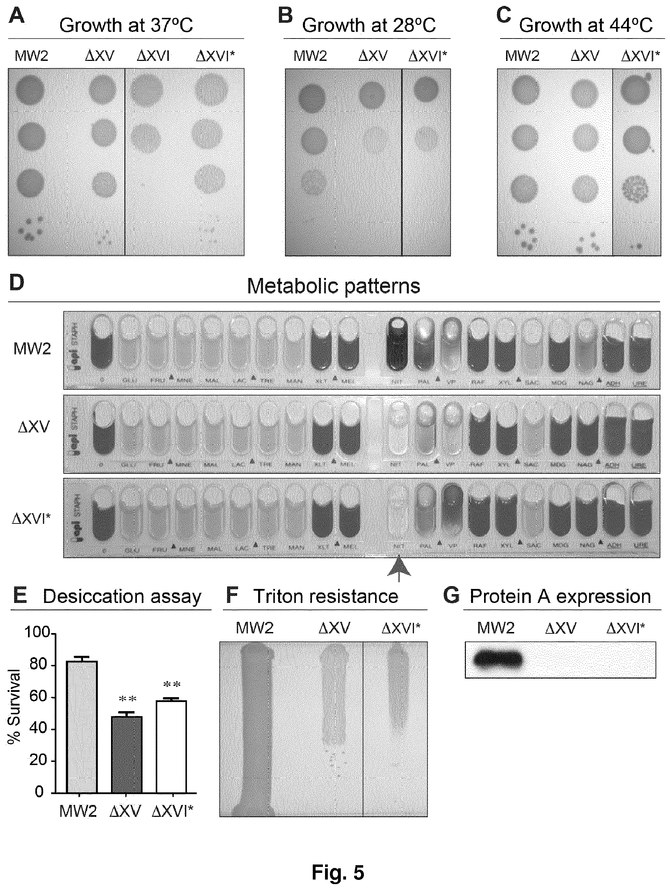

**Fig. 5****.** Phenotypic analyses of *S. aureus* MW2 wild type strain and the mutant strains ΔXV and ΔXVI*.
   - Panels A, B, C: Photographs of agar plates with surviving bacteria after overnight incubation at 37ºC (panel A), 28ºC (panel B), 44ºC (panel C). Dilutions of inoculation (from top to bottom): sample of overnight non diluted culture (top), and dilutions 10⁻², 10⁻⁴, 10⁻⁶ (bottom plates). In Panel A, comparisons of the growth of the ΔXVI strain complemented with an empty plasmid (ΔXVI, third column), complemented with the plasmid pEI walR (strain ΔXVI*: fourth column) .
   - Panel D: Metabolic pattern of wild type (WT) MW2 strain and ΔXV and ΔXVI* mutants according to API Staph galleries. The arrow indicates the position of the metabolic assay where differences between wild type and mutants appears.
   - Panel E: Bar cha representing the desiccation tolerance after 21 days, indicated as the survival percentage after overnight culturing (initial numbers) and air-drying, storing at room temperature, rehydration and determination of remaining viable cells.
   - Panel F: Photographs of *S. aureus* strains MW2 WT, MW2 ΔXV and MW2 ΔXVI*, as indicated over the photographs, growing on Triton X-100 concentration gradient plates.
   - Panel G: Analysis of Protein A expression by Western blotting in *S. aureus* strains MW2 WT, MW2 ΔXV and MW2 ΔXVI* .

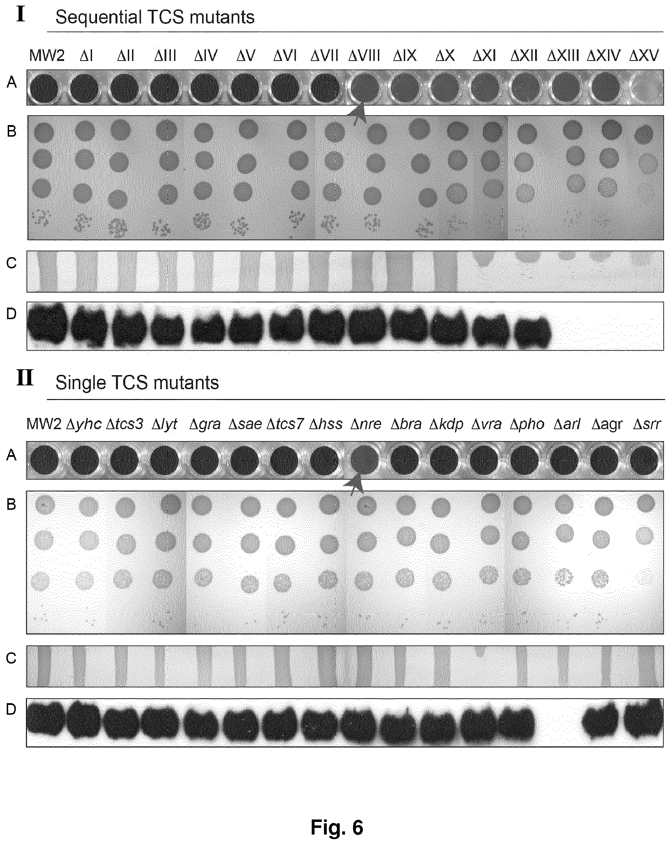

**Fig. 6****.** Phenotypic analyses of *S. aureus* MW2 sequential TCS mutants (I) (number of mutant indicated over each corresponding column) and single TCS mutants (II) of the MW2 strains.
   - Panel A. Nitrate reduction capacity under low oxygen concentration: colorimetrical determination of extracellular nitrite concentrations by the Griess Reagent System. The arrows indicate the position of the sequential mutant where the purple/magenta coloration becomes lighter, almost pink (part I) or the single TCS mutant showing a lighter coloration than the others (part II).
   - Panel B. Bacterial growth at 28ºC on agar plates of decreasing serial dilutions (not diluted, 10⁻², 10⁻⁴ and 10⁻⁶) of the MW2 wild type and mutant strains.
   - Panel C. Growth phenotype on Triton X-100 concentration gradient plates.
   - Panel D. Analysis of Protein A expression by Western blotting
**Fig. 7****.** Complementation of phenotypes in single and ΔXV mutant strains by ectopic expression of a single TCS from expression plasmids.
   - Panel A. Nitrate reduction capacity under low oxygen concentration: colorimetrical determination of extracellular nitrite concentrations by the Griess Reagent System. It can be seen that expression of *nreBC* in both the single Δ*nreBC* mutant (third plate from the left, labelled Δ*nreBC* pEI *nreBC*) and the ΔXV mutant (fifth plate from the left, labelled ΔXV pEI *nreBC*) is sufficient to recover the purple/magenta coloration that is lighter (single mutant with an empty plasmid, *"*Δ*nreBC* pEI") or absent (ΔXV mutant with an empty plasmid, "ΔXV pEI") in the corresponding mutant not expressing *nreBC.*
   - Panel B. Bacterial growth at 28ºC on agar plates of decreasing serial dilutions (not diluted, 10⁻², 10⁻⁴) of the MW2 wild type, transformed with an empty pEI plasmid, mutant strains *ΔsrrAB* and ΔXV transformed either with a pEI empty plasmid (second and fourth column) or with a pEI plasmid expressing the TCS *nreBC* (third and fifth column).
   - Panel C. Bacterial growth on Triton X-100 concentration gradient plates. Assayed strains: MW2 wild type transformed with an empty pEI plasmid, mutant strains Δ*vraSR* and ΔXV transformed either with a pEI empty plasmid (second and fourth column) or with a pEI plasmid expressing the TCS *vraSR* (third and fifth column).
   - Panel D. Analysis of Protein A expression by Western blotting. Assayed strains: MW2 wild type transformed with an empty pEI plasmid, mutant strains Δ*arlRS* and ΔXV transformed either with a pEI empty plasmid (second and fourth column) or with a pEI plasmid expressing the TCS *arlRS* (third and fifth column).

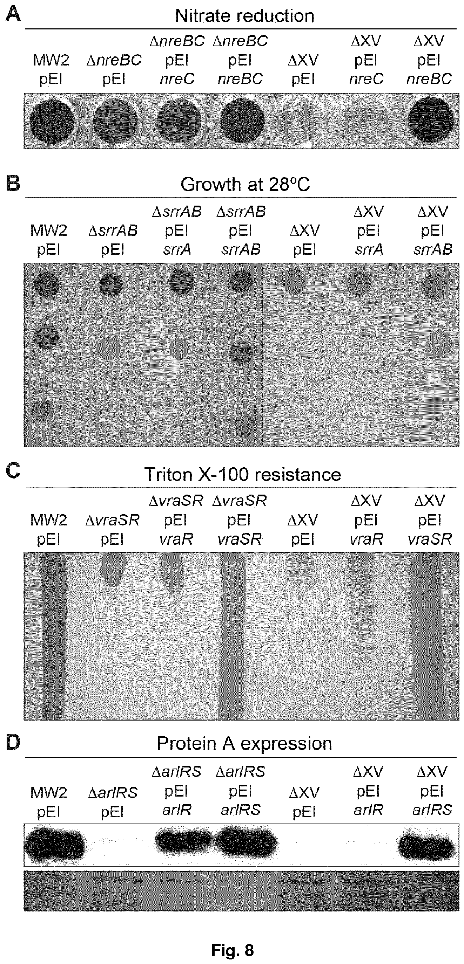

**Fig. 8****.** Complementation of the single and ΔXV mutant strains with the RR alone or in combination with the cognate HK.
   - Panel A. Nitrate reduction capacity under low oxygen concentration: colorimetrical determination of extracellular nitrite concentrations by the Griess Reagent System. Assayed strains: MW2 wild type transformed with an empty pEI plasmid, mutant strains Δ*nreBC* and ΔXV transformed either with a pEI empty plasmid (second and fifth column) or with a pEI plasmid expressing the RR nre*C* alone (third and sixth column) or also complemented with its HK *nreB* (fourth and seventh column). It can be seen that expression of *nreC* alone in both the single Δ*nreBC* mutant and the ΔXV mutant gives rise to the same color that the single mutant with the empty vector, the intense purple/magenta coloration being recover in both mutants expressing the RR and the HK of the TCS *nreBC.*
   - Panel B. Bacterial growth at 28ºC on agar plates of decreasing serial dilutions (not diluted, 10⁻², 10⁻⁴) of the MW2 wild type, transformed with an empty pEI plasmid, mutant strains *ΔsrrAB* and ΔXV transformed either with a pEI empty plasmid (second and fifth column), with a pEI plasmid expressing the RR *srrA* alone (third and sixth column) or also complemented with the HK *srrB* (fourth and seventh column).
   - Panel C. Growth phenotype on Triton X-100 concentration gradient plates. Assayed strains: MW2 wild type, transformed with an empty pEI plasmid, mutant strains *ΔvraSR* and ΔXV transformed either with a pEI empty plasmid (second and fifth column), with a pEI plasmid expressing the RR *vraS* alone (third and sixth column) or also complemented with the HK *vraR* (fourth and seventh column).
   - Panel D. Analysis of Protein A expression by Western blotting. Assayed strains: MW2 wild type, transformed with an empty pEI plasmid, mutant strains *ΔarlRS* and ΔXV transformed either with a pEI empty plasmid (second and fifth column), with a pEI plasmid expressing the RR *arlR* alone (third and sixth column) or also complemented with the HK *arlS* (fourth and seventh column)

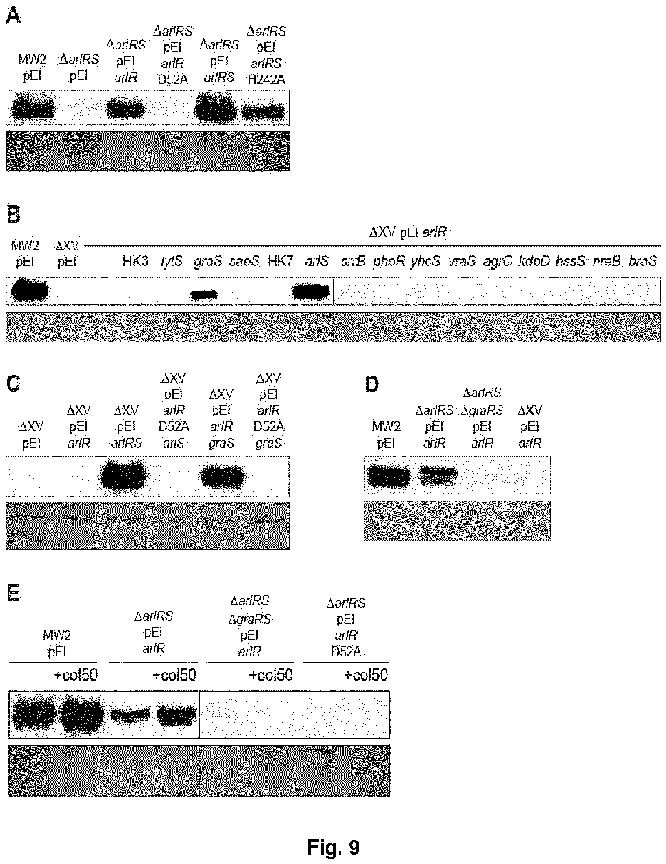

**Fig. 9****.** System-based analysis of cross-talk *in vivo.* Analysis of Protein A expression by Western blotting on the following strains:
   - Panel A: Single Δ*arlRS* mutant strain complemented with *arlR*, *arlR* D52A, *arlRS* and *arlRS* H242A
   - Panel B: Δ XV strain complemented with the set of plasmids containing *arl* RR combined with the 16 HKs. Note that GraS, apart from ArlS, is able to complement *arlR*-dependent protein A expression
   - Panel C: Δ XV strain complemented with *arlR* or non-phosphorylatable *arlR* D52A combined with ArIS or GraS. Note that phospho-transfer is necessary for *arlR*-dependent protein A activation by GraS
   - Panel D: Single Δ *arlRS,* double Δ *arlRSgraRS* and ΔXV mutant strains complemented with *arlR.* Note that cross-talk dissappears in the absence of *graRS*
   - Panel E: Single Δ *arlRS* and double Δ *arlRSgraRS* strains complemented with the *arlR* or *arlR* D52A in the presence or absence of 50 µg/ml of colistin. Note that colistin activates *arlR*-dependent protein A.

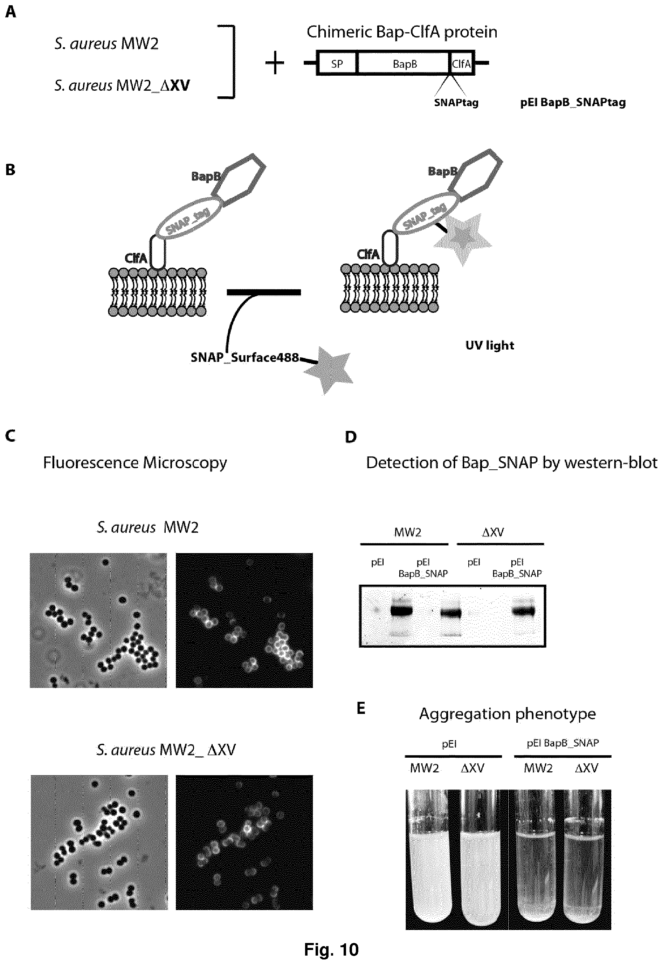

**Fig. 10****.** Expression of the Bap-ClfA chimeric protein in MW2 wild type and MW2 ΔXV strains.
   - Panel A: Schematic representation of the Bap-ClfA chimeric protein present in the plasmid pEI-BapB_SNAPtag, which chimeric protein containing the N-terminal region of *S. aureus* Bap protein ("BapB"), the C-terminal region of clumping factor A ("ClfA") and a tag named SNAP ("SNAPtag") located between them and that allows the fluorescent labelling of the chimeric protein.
   - Panel B: Schematic representation of the location of the chimeric protein on the bacterial surface and the fluorophore marker (SNAP_Surface488) which interacts with the SNAP peptide.
   - Panel C: Photographs obtained by fluorescence microscopy of an S. *aureus* MW2 parenteral strain (first row) or its isogenic mutant MW2 ΔXV (second row) expressing the Bap-ClfA chimeric protein on their surfaces. The expression of the protein is visualized by means of the SNAP_Surface488 fluorophore marker.
   - Panel D: Detection of the Bap-ClfA chimeric protein in SDS-PAGE gels in S. *aureus* strains MW2 and ΔXV complemented with an empty pEI plasmid (left lane in each pair of lanes corresponding to each strain) or with the plasmid pEI-BapB_SNAP containing the coding sequence of the Bap-ClfA chimeric protein.
   - Panel E: Photographs showing the aggregation phenotype associated to the absence (first two tubes starting from the left side) or presence (last two tubes of the series) of the expression of the Bap-ClfA chimeric protein, in the MW2 (first tube in each pair) or the ΔXV (second tube in each pair) strain.

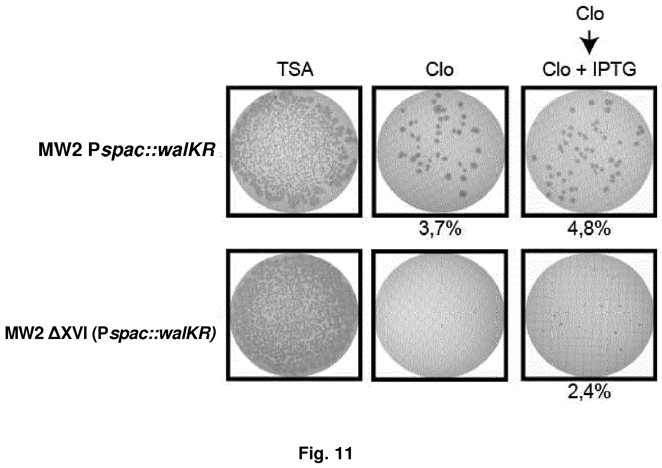

**Fig. 11****.** Photographs of Petri plates inoculated with an S. *aureus* MW2 parental strain (first row) or its isogenic mutant MW2 ΔXV (second row), in which strains the *walKR* operon is under *Pspac* control. Bacteria were first incubated for 24h in the presence of chloramphenicol (Col) and IPTG as indicated and then they were incubated in new agar plates for 12h in the presence or absence of chloramphenicol and IPTG as indicated.

### DETAILED DESCRIPTION OF THE INVENTION

The present invention relates, among others, to a mutant *Staphylococcus aureus* strain which is devoid of all TCSs not essential for viability, since all of them have been mutated so that they have been inactivated; the examples of the present application disclose particular embodiments of such mutant strains where the inactivation has been achieved by sequential deletion of the TCSs. The invention also relates to a mutant *S. aureus* strain completely devoid of TCSs, where the expression of the RR of the TCS *walKR* is maintained, because the *walKR* system is essential for growth of *S. aureus* bacteria. The intermediate mutants generated for obtaining said two previously mentioned mutant strains are also encompassed by the scope of the present invention. The use of the *S. aureus* mutant strains provided by the present application, particularly as research tools and/or as platforms for biotechnological applications such as the expression of heterologous proteins or the identification of candidates to antimicrobial agents from compounds capable to modulate or inactivate specific TCS, are also part of the present invention.

The present invention represents an approach completely different from the strategies followed until now for the study of the TCSs and their usefulness for biotechnological applications such as using them as targets for the identification and development of antimicrobial drugs, the expression of heterologous proteins, either constitutively or under the control of a specific environmental signal, and as a bacterial suicide or programme cell death control by the absence of IPTG or another induction system. Instead of aiming to infer the functions that each TCS perform from the phenotypes shown by strains where an individual TCS is mutated, without taking into account possible compensatory effects or interferences with the remaining members of *S. aureus* sensing system, the present inventors have decided to take advantage of the moderate complexity of the TCS signaling in *S. aureus* and have conceived and put into practice a reductionist approach: they have generated a strain that is completely devoid of TCS signaling system and used it to investigate the consequences that the removal of the complete TCS sensorial network has for the bacteria, as well as the biotechnological applicability of such mutant and of the intermediate mutants necessary for obtaining it.

The removal was done in two steps:
- First, all non-essential TCS (fifteen in the case of the specific examples shown in the present application) were sequentially deleted from the chromosome for inactivating them. The obtained mutant received the generic denomination ΔXV because the process was carried out using strains lacking the *kdpED-like* system, so that they contain fifteen non-essential TCSs. The present invention is also compatible with the use as starting strains of *S. aureus* strains also expressing the *kdpED-like* system but, in such strains, obtaining a mutant strain lacking all non-essential TCS implies the additional inactivation of the *kdpED-like* system, thus being sixteen the inactivated TCSs. Therefore, the mutant strains of *S. aureus* wherein the following TCSs have been deleted or inactivated: *yhcSR;* the TCS comprised of the components expressed either i) by the genes *MW0199-MW0198* of *S. aureus* strain MW2 (tcs3), or ii) by the genes *SAOUHSC_00185-SAOUHSC_00184* of *S. aureus* strain 8325 (tcs3), or iii) by their corresponding orthologous genes of a another *S. aureus* strain; lytSR; graRS; saeRS; the TCS comprised of the components expressed either i) by the genes *MW1207-MW1208* of strain MW2 (tcs7), or ii) by the genes *SAOUHSC_01313-SAOUHSC_01314* of S. *aureus* strain 8325 (tcs7), or iii) by their corresponding orthologous genes of another S. *aureus* strain; *hssRS; nreBC; braRS; kdpDE; vraSR; phoPR; arlRS; agr* and *srrAB,* and that additionally lack the *kdpED-*like two component signal system, either because it was not present in the wild-type strain used as starting point for obtaining the mutant strains or also because the *kdpED-like* was present in the wild-type strains but has been equally deleted or inactivated, are also encompassed within the scope of the present invention.
- Then, the expression of the remnant WalKR essential system was placed under the control of an inducible Pspac promoter, so that its expression is inducible by IPTG (1 mM). In the absence of IPTG, the strain lacks the sixteenth two-component systems (mutant strain ΔXVI).

The strain can grow in the absence of IPTG when WaIR is produced ectopically under the control of a constitutive promoter. Thus, the resulting strain lacks all the HKs and it contains a single RR. In the specific cases described in the present application, the resulting mutant can be named ΔXVI*.

The term "devoid of", as it is used in the present application, and applied to a particular TCS, means that the bacteria lack the genes, the component proteins (HK and RR) or the activity of said TCS. That is, a bacterium can be devoid of a particular TCS because the genes corresponding to the system are naturally not present in that strain, because said genes have been deleted, because said genes have been mutated so that they are not expressed (not transcribed or not translated) or even because they give rise to inactive proteins, because the corresponding proteins (HK and RR) are expressed but they are inactivated by means of (an) inhibitor(s) or because the bacterium is grown in conditions where the genes corresponding to said TCS are not expressed because they have been put under the control of (that is, they are operatively linked to) a promoter or any other control element that does not drive the expression of the genes of said TCS under such conditions. As all of such situations results in a loss of the activity of said TCS, in some points of the present application it has been considered that "being devoid of a TCS" can be used as having such TCS inactivated, so that "being devoid of a TCS" and "having a TCS inactivated" have been used as synonyms. Moreover, in the Examples of the present application, the process used for inactivating non-essential TCSs, as can be inferred from the explanation given above, has consisted of deleting them from the bacterial chromosome, so that, for most *S. aureus* mutated strains of the present invention, such as the strains named ΔI, ΔII, ΔIII, ΔIV, ΔV, ΔVI, ΔVII, ΔVIII, ΔIX, ΔX, ΔXI, ΔXII, ΔXIII, ΔXIV and ΔXV, deletion and inactivation can be considered synonyms.

Example 1 of the present application discloses the generation of two specific ΔXV mutants, each one obtained from a different strain: MW2 and 8325. Therefore, for reasons of clarity in those assays where both mutant strains are used, each ΔXV mutant is specifically denominated MW2 ΔXV or 8325 ΔXV in some locations of the present application, depending on the particular *S. aureus* strain used as starting point for its generation. As indicated above, the inactivation process used in Example 1 consisted of the sequential deletion of all 15 non essential TCS present in two particular *S. aureus* strains. As all *S. aureus* HK and RR pairs are located together, in the same operon, it is possible to define each step of the process as the deletion of a particular TCSs and defining each particular mutant strain obtained in each step with regard to the TCS or combination of TCSs deleted in such strains, as can be seen in the Examples section of the present application. An alternative way of defining the mutant strains obtained in each step is mentioning the specific genes deleted (or, in the general definition of the invention, inactivated), so that the mutant ΔXV, for instance, can be also defined as a mutant strain of *S. aureus* characterized in that all the folllowing genes of the two-component signal systems are deleted or inactivated: the gene *MW0199* of MW2 or the gene *SAOUHSC_00020* of strain 8325 or their orthologous gene that encodes the sensor component of the analogous TCS in the corresponding parental strain; the gene *MW0198* of MW2 or the gene *SAOUHSC_00021* of strain 8325 or their orthologous gene that encodes the response component of the analogous TCS in the used S. *aureus* parental strain, *lytS; lytR; graS; graR; saeS; saeR;* the gene *MW1207* of MW2 or the gene *SAOUHSC_01313* of strain 8325 or their orthologous gene that encodes the sensor component of the analogous TCS in the used *S. aureus* parental strain; the gene *MW1208* of MW2 or the gene *SAOUHSC_01314* of strain 8325 or the orthologous gene that encodes the response component of the analogous TCS in the used *S. aureus* parental strain; *arlS; arlR; srrB; srrA; phoR; phoP; yhcS; yhcR; vraS; vraR; agrC; agrA; kdpD; kdpE; hssS; hssR; nreB; nreC; braS; braR.* As can be seen in said Examples section, each step of the process gives rise to a particular intermediate mutant, which mutants are generically named ΔI, ΔII, ΔIII, ΔIV, ΔV, ΔVI, ΔVII, ΔVIII, ΔIX, ΔX, ΔXI, ΔXII, ΔXIII, ΔXIV and ΔXV (wherein the Roman number indicates the number of the TCSs inactivated, by deletion or by other means, in the mutant with regard to the *S. aureus* parental strain) in accordance with the number of TCSs deleted in each case. Where clarification is needed, the specific mutants obtained from the strain MW2 or from the strain 8325 are distinguished by including the name of the parental strain before the indication of the number of genes deleted. Table 2 of the present application provides information about the specific genes to be deleted or inactivated in strains MW2 and 8325 in order to inactivate each particular TCS; additional information about the genes corresponding to the TCSs herein denominated "tcs3" and "tcs7" is also given in Example 1. Regarding possible mutants obtained from other parental strains, it must be pointed out that the genome of *S. aureus* is well conserved among different strains and isolates, so that the homology among orthologous proteins (proteins with the same function) is high among different strains, existing an homology of 90%-95% or higher among the proteins that are components of TCSs and play the same function in different strain. Therefore, one skilled in the art will find obvious to identify the gene that must be deleted/inactivated in each *S. aureus* strain in order to obtain *S. aureus* mutants of the present invention starting with *S. aureus* parental strains different from those used in the Examples of the present application, even if the genes and/or the corresponding TCSs have a name not mentioned in the present application: it should be a gene whose protein product has the same function as the protein product of one of the above mentioned genes (a histidine kinase or a response element of a specific TCS) and an homology of at least 90% is expected with regard to the corresponding protein expressed by the strain MW2 or 8325 from the corresponding gene mentioned in Table 2 above, which will be the corresponding orthologus gene as the term is used in the present application.

The information provided in Example 1 shows that the TCS have not been deleted in the same order for the generation of the mutants derived from MW2 or from the 8325 parental strain, because the present invention is compatible with the deletion of the non-essential TCS in any order. Thus, each one of the intermediate mutants ΔII, ΔIII, ΔIV, ΔV, ΔVI, ΔVII, ΔVIII, ΔIX, ΔX, ΔXI, ΔXII, ΔXIII, ΔXIV, and ΔXV (wherein the Roman number indicates the number of non-essential TCSs inactivated, by deletion or by other means, in the mutant with regard to the *S. aureus* parental strain), are also comprised within the scope of the present invention and are an aspect of said invention. Embodiments of specific interest are those derived from the strain MW2 or the strain 8325, whose obtaining process is described in Example 1, mentioning the specific combination of TCSs deleted in each mutant. Also embodiments of particular interest are the ΔIV, ΔV, ΔVI, ΔVII, ΔVIII, ΔIX, ΔX, ΔXI, ΔXII, ΔXIII, ΔXIV and ΔXV mutants, particularly those derived from the MW2 or 8325 parental strains disclosed in the present application, among other reasons, because obtaining mutants with such number of deleted TCS requires a considerable amount of time and resources and the generation of such mutants had not been previously considered either for studies related to the functioning or possible interactions of the TCSs themselves or for other biotechnological applications.

Although the *S. aureus* mutant strains ΔI, ΔII, ΔIII, ΔIV, ΔV, ΔVI, ΔVII, ΔVIII, ΔIX, ΔX, ΔXI, ΔXII, ΔXIII, ΔXIV and ΔXV whose generation is described in the present application have been obtained by a process of sequential deletion of non essential TCSs starting from a parental strain (MW2 or 8325), the present invention is compatible with the use of any other inactivation techniques known for those skilled in the art for obtaining the inactivation for each one of the non essential TCS, such as the interruption of the genes corresponding to the sensor and the cytoplasmic response regulator, mutations in said genes giving rise to inactive proteins, mutations in the corresponding promoter making unable the synthesis of the corresponding mRNAs... Also compatible with the present invention, and included within the scope of the same, is the situation where the inactivation of one or a combination of TCSs is conditional, because the operon/s corresponding to one or more TCSs is expressed under the control of a regulatory element, such as a promoter, which is active only under particular conditions, such as it is disclosed in the present application for obtaining the sense devoid *S*. *aureus* mutant ΔXVI*, wherein the *walKR* operon was placed under the control of the inducible P*spac* promoter, the expression of *walKR* being inhibited in such mutant in the absence of alolactose or an analogue of alolactose capable of triggering the transcription of the lac operon, such as IPTG (isopropyl-β-D-1-thiogalactopyranoside). A multiplicity of promoters with similar characteristics, also compatible with the present invention, are well known, such as the tetracycline (Bateman *et al.,* 2001), cadmiun (Charpentier *et al.,* 2004) and xylose (Kim *et al.,* 1996) inducible promoters. Where the inactivation of several TCSs is made conditional in an individual mutant, all TCSs can be under the control of a control element responding to the same conditions (under the control of the same promoter, for instance) or each TCS can be under the control of a promoter that is active under conditions that are different from those activating other promoters controlling other TCSs, so that the number of TCS activated in a particular assay can be chosen selecting a particular combination of conditions, each one regarding a particular promoter.

The group of the present inventors carried out the deletion process described below in the section of Examples of the present application using as parental strains (the starting strains), two genetically unrelated strains: MW2 and 8325. MW2 is a community-acquire MRSA strain (deposit number in the American Type Culture Collection ATCC BAA-1707) and 8325 (deposit number in the National Collection of Type Cultures NCTC 8325) is a laboratory strain, that is still sensible to standard antibiotics such as methicillin. The use of genetically unrelated strains allows comparing the phenotypes caused by the sequential mutation of TCS in both ΔXV strains. This strategy is time-consuming but necessary when using reductionist approaches because complementation of the final strain to restore the phenotypes is technically not possible.

Both parental *S. aureus* strains, MW2 and 8325, lack the *kdpED-like* system, so that they are known to contain 16 TCSs and to have 15 non-essential TCSs. Also strains such as *S. aureus* Newman (PATRIC Genome ID:426430, GenBank Accession AP009351 version AP009351.1 GI:150373012), *S. aureus* JH1 (PATRIC Genome ID:359787.11, GenBank Accession CP000736 version CP000736.1 GI:149944932), *S. aureus* JH9 (PATRIC Genome ID:359787.11, GenBank Accession CP000703 version CP000703.1 GI:147739516), *S*. *aureus* Mu50 (PATRIC Genome ID:158878.14, GenBank Accession NC_002758 version NC_002758.2 GI:57634611), *S. aureus* N315 (PATRIC Genome ID:158879.11, GenBank Accession NC_002745 version NC_002745.2 GI:29165615) and *S. aureus* COL (PATRIC Genome ID:93062.19, GenBank Accession NC_002951 version NC_002951.2 GI:57650036), whose genome are completely sequenced on 21 August 2015 and are known to have 16 TCSs, are suitable parental strain. Also any other *S. aureus* strain cited in the section of the web site of PATRIC (Pathosystems Resource Integration Center) corresponding to *S. aureus* genome list (https://www.patricbrc.org/portal/portal/patric/GenomeList?cType=taxon&cId=1279 &displayMode=Complete&dataSource=PATRIC&pk=) or any other *S*. *aureus* strain, can be a suitable parental strain among others. In general, the use of parenteral *S*. *aureus* strains with 16 TCSs for the generation of the mutant strains of the present invention ΔI, ΔII, ΔIII, ΔIV, ΔV, ΔVI, ΔVII, ΔVIII, ΔIX, ΔX, ΔXI, ΔXII, ΔXIII, ΔXIV, ΔXV and ΔXVI is a suitable option for the purposes of the present invention. As commented above, the use of parenteral *S*. *aureus* strains with 17 TCSs is also compatible with the present invention, and the mutant strains generated from such parental strains wherein at least one or two, three, four five, six, seven, eight, nine, ten, eleven, twelve, thirteen, fourteen, fifteen or sixteen non-essential TCS are deleted or, in general, inactivated, rendering the strain devoid of such non-essential TCS, are also comprised within the scope of the present invention. Therefore, also comprised within the scope of the present invention is any mutant *S. aureus* strain wherein any combination of at least two, three, four five, six, seven, eight, nine, ten, eleven, twelve, thirteen, fourteen or fifteen two component signal systems selected from the group of *yhcSR;* the TCS comprised of the components expressed either i) by the genes *MW0199-MW0198* of S. *aureus* strain MW2 (tcs3), or ii) by the genes *SAOUHSC_00185-SAOUHSC_00184* of S. *aureus* strain 8325 (tcs3), or iii) by their corresponding orthologous genes of a another *S. aureus* strain; *lytSR; graRS; saeRS;* the TCS comprised of the components expressed either i) by the genes *MW1207-MW1208* of strain MW2 (tcs7), or ii) by the genes *SAOUHSC_01313-SAOUHSC_01314* of *S. aureus* strain 8325 (tcs7), or iii) by their corresponding orthologous genes of another S. *aureus* strain; *hssRS; nreBC; braRS; kdpDE; vraSR; phoPR; arlRS; agr; srrAB,* are deleted or inactivated, as well as any of said mutants which lacks the *kdpED-*like TCS, either because its parental strain naturally lacks it or because the *kdpED-*like system has been also deleted or inactivated, so that the mutant strain lacks the expression of such system.

The *S. aureus* mutants of the present invention having been generated from parental strains naturally having the *kdpED-like* system are an embodiment of the present invention that can be of particular interest for certain applications, particularly those related to the identification of potential antimicrobial compounds effective against *S. aureus,* because the *kdpED-like* system is carried by some methicillin resistant strains.

The ΔXV mutants whose generation is disclosed in the present application, as previously commented, have been generated using as parental *S. aureus* strains, MW2 and 8325, which lack the *kdpED-like* system, so that said ΔXV mutants are devoid of all non-essential TCSs. The analysis of the complete genome sequenced of both ΔXV mutants did not reveal the presence of common polymorphisms compared to their corresponding wild type parental strains, indicating that the deletion process had no selected for compensatory mutations and both mutants were appropriate for any complementation study as those disclosed in the present application.

Subsequently, the ΔXV mutant was used to generate a derivate also devoid of the WalKR system, using in this case the approach of placing the *walKR* operon under the control of an inducible promoter, Pspac, so that the *walKR* TCS is inactivated in such generated ΔXVI mutant when it is grown in media lacking an inductor of the Pspac promoter, such as IPTG. The mutant was complemented with a plasmid encoding the response regulator of the *walKR* system, the *walR* gene, under the control of a constitutive promoter, so that the generated ΔXVI* strain constitutively expresses the *walR* gene, but not the complete *walKR* system unless it is grown in a medium containing IPTG (1 mM) or other inductor of the Pspac promoter. Therefore, in the absence of an inductor of the expression of the *walKR* operon, the ΔXVI* mutant is devoid of all TCSs, because the WalKR system, as such TCS, is not active, due to the lack of the sensor component. Surprisingly, in spite of being a sense devoid *S. aureus* mutant, the ΔXVI* mutant is viable under normal laboratory growing conditions (see the Examples section for the particular conditions) and shows phenotypic characteristics similar to those of the ΔXV mutants: they exhibited growth rates indistinguishable from the wild type under aerobic conditions at 37ºC and 44ºC. Loss of sensing capacity causes growth defect at 28°C, decreased capacity for long-term survival during desiccation, higher susceptibility to detergents, deficiency in the capacity to reduce nitrate to nitrite, and renders the bacteria avirulent (see Examples 1 and 2). These results indicate that deprivation of the two-component sensorial system has less consequences for the bacterial biology than expected and demonstrate that, with the exception of *walKR* (and, particularly, the sensor or response regulator WalR), TCSs are not necessary for the maintenance of house-keeping functions under constant laboratory growing conditions. This can be considered particularly unexpected after having confirmed, as it is described in the first part of Example 1, that most TCSs are constitutively expressed in all the *S. aureus* strains tested, what indicates that bacteria need to have ready the TCSs to respond to the different stimuli that might encounter in different environmental conditions.

The mutants ΔXV and ΔXVI provided by the present invention (or, in a more generic denomination, the *S. aureus* mutant strains devoid of all non-essential TCS systems where, in the particular case of ΔXVI, *walKR* can be selectively turned off) are stable and manageable. The mutants ΔXV and ΔXVI provide an excellent platform to generate a collection of strains containing single TCS, additional to those remaining in the ΔXV or ΔXVI strains, by reintroducing a cassette with the corresponding coding sequences under the control of appropriate elements (for instance, an inducible or constitutive promoter), the cassette preferably being part of a suitable expression vector such as a plasmid or a recombinant phage. This collection, together with the mutant ΔXVI, is an excellent research tool for increasing the knowledge about *S. aureus* sensing system in general, enabling novel approaches for the identification of the biological processes regulated by each individual TCS, the possibilities of *in vivo* cross-talk among HKs and RRs each one belonging to different TCSs, the regulation of the expression of each TCSs, the compounds (inductors or inhibitors) affecting them, the possible influence of acting on different TCS in bacterial viability, the possible genes or systems that could complement the absence of the expression of a specific TCSs, the relation of each two-component system with host-pathogen interactions including *S. aureus* cell adhesion, invasion, tissue colonization, pathogenicity and antibiotic-resistance capabilities, the development of *S. aureus* avirulent strains or many other aspect of *S. aureus* biology. Increasing the knowledge about said matters will allow, directly or indirectly, developing many practical applications of direct industrial interest such as the use of TCS as targets for antibiotics identification and development, or even for identifying promoters whose expression depends on environmental signals or for developing biosensor tracks for environmental signals. Moreover, as they are still genetically manageable, both mutant strains represent excellent platforms for biotechnological applications by themselves, which applications can be independent of the analysis of the TCS network or could be designed to complement such analysis. Examples 3 to 6 of the present application show the applicability of the *S. aureus* mutant strains of the present application for such uses, since they show how the availability of the mutant strains of the present invention have enabled the clarification of the function of some particular TCS, their regulatory networks and the possibilities of cross talk among some particular TCS, as well as the applicability of the strains for some biotechnological uses.

For instance, Example 3 shows how the ectopic expression of single TCS in the ΔXV strain allows the identification of the regulatory networks that depend especifically on individual TCS. To date, the regulon affected by a TCS had been inferred from the phenotype and transcription profile displayed by the single mutant and its restoration after complementation. However, such approach could not distinguish between those members of the regulon that are direct targets of the TCS from those targets whose expression depends indirectly on a second TCS. In the present application it is disclosed how the present inventors used the ΔXV mutant complemented with the battery of TCS to analyze whether individual TCS were able to restore the phenotypes characteristic of the ΔXV strain. Interestingly, the results show that single TCSs are sufficient to restore the phenotypes of the ΔXV strain, supporting the notion that TCS regulatory pathways are insulated from one another and little interdependency exists among them. This system-level insulation might explain why phenotypes of ΔXV strains correspond to the sum of phenotypes controlled by each TCS, without noticeable pleiotropic effects on other cellular processes. And it also shows the interest of mutants obtained using as parental strains the ΔXV strains (or, more generally, the *S. aureus* strains devoid of all non-essential TCS) or the ΔXVI strains (or, in general, the *S. aureus* strains devoid of all TCS) but containing plasmids or other expression systems additional to the bacterial chromosome which allow the ectopic expression (constitutive or inducible) of one of the TCS that has been deleted or inactivated in the ΔXV strain. Such mutants are also an aspect of the present invention. And also a particularly preferred embodiment of the use as research tool or platform of the strains of the present invention is carrying out (an) assay(s) wherein one observes the phenotypic differences between a particular mutant strain of the present invention and another mutant that differs from the first mentioned mutant in that it ectopically expresses either: a) one or both of the two components, HK and RR, of a TCS selected from the group of *yhcSR;* the TCS comprised of the components expressed either i) by the genes *MW0199-MW0198* of S. *aureus* strain MW2 (tcs3), or ii) by the genes *SAOUHSC_00185-SAOUHSC_00184* of *S. aureus* strain 8325 (tcs3), or iii) by their corresponding orthologous genes of a another *S. aureus* strain; *lytSR; graRS; saeRS;* the TCS comprised of the components expressed either i) by the genes *MW1207-MW1208* of strain MW2 (tcs7), or ii) by the genes *SAOUHSC_01313-SAOUHSC_01314* of *S. aureus* strain 8325 (tcs7), or iii) by their corresponding orthologous genes of another S. *aureus* strain; *hssRS; nreBC; braRS; kdpDE; vraSR; phoPR; arlRS; agr; srrAB; kdpED-like* and *walKR,* or b) any other polypeptide (a natural protein, a mutated protein, a chimeric protein, a fusion protein or even a group of proteins, usually resulting from the same polycistronic messenger) or gene product (such as a iRNA). It is particularly preferred that the mutant strains of the present invention used in the assay is a mutant strain devoid of all non-essential TCS (such as one of the ΔXV strains whose generation is described in the Examples section of the present application) or a mutant strain devoid of all TCS (such as one of the ΔXVI strains whose generation is also described in the present application), because it allows to identify clearly any other protein or group of proteins that could complement the function of a particular TCS after its inactivation without the noise of the functioning of other TCS or the whole sensing system. Since there are a number of plasmids that are well known for those skilled in the art as plasmids that can be replicated in S. *aureus,* such as those used in the present application like pMAD, pSD-3 or pCN47, pCU1, pBT2, and also well known are promoters that can give rise to the expression of coding sequences operatively linked to them when they are part of expression cassetes present in *S. aureus* cells, either constitutively (all those promoters whose genes are expressed under any circumstaces, such as the promoter of the ribosomal genes, BlaZ, the promoter inducible by cadmium when the inductor is not present) or when an inductor is present (such as Pspac or also the tetracycline (Bateman *et al.,* 2001), cadmiun (Charpentier *et al.,* 2004) and xylose (Kim *et al.,* 1996) inducible promoters), the information provided in the present application is enough for enabling one skilled in the art to put into practice such aspect of the present invention.

Such assays can be useful for a variety of purposes, particularly when the second mutant strain ectopically expresses one or both of the two components, HK and RR, of a TCS and also any other polypeptide or groups of polypeptides. An interesting embodiment is that one wherein the other polypeptide or a polypeptide of the group of polypeptides is itself or comprises a fragment with the amino acid sequence of a reporter, selection marker or labelling protein, such as a fluorescent protein (like GFP or RFP), a protein that confers resistance to a particular antibiotic, another selection marker or any other protein capable, under particular conditions, of giving rise to a signal easy to detect selected among those well known by those skilled in the art. Particularly interesting might be those assays where the regulatory conditions of a promoter operatively linked to the reporter, marker or labelling protein (or to the fusion or chimeric protein containing it) are not completely known, because the assay can be used for identifying promoters whose expression depends on environmental signals: the appareance of the marker or labelling protein only when a particular environmental signal is present among the culture conditions will indicate that such promoter depends on that environmental signal. Moreover, the mutant strain where the assayed promoter is present can be a useful biosensor track for environmental signals. When the signal is specifically sensed by the TCS also ectopically expressed, a significant degree of specificity is also achieved with such mutant as biosensor track. Therefore, the mutant strains of the present application can be useful not only as research tools, but they can have direct applicability as in the above described situation.

In the Examples of the present application, the knowledge of the phenotypes activated by each TCS together with the ΔXV strain were used to perform a systematic analysis of cross-talk *in vivo* in two phases. First, the present inventors analyzed the capacity of a RR to activate the corresponding phenotype in the single mutant compared to the ΔXV strain. This simple strategy allows the rapid identification of those RRs that are activated in the presence of non-cognate HKs. The present inventors focused on those RRs for which a clear phenotype in the ΔXV strain was detected. In agreement with notion that cross-talk between TCS is rare, only ArlR, among the response regulators analyzed, was activated by non-cognate HK. Second, they searched for the HKs capable to activate ArIR by complementing the ΔXV strain with a set of plasmids containing the combination of *arlR* and the whole family of HKs. The results showed that mainly GraS and to a lower level SrrA were able to activate ArlR. Both systems, ArIS and GraS, have been involved in the regulation of bacterial autolysis, indicating that their regulons overlap. In this respect, and taking the advantage that ArlRS regulates also the production of some secreted proteins (Fournier *et al.,* 2001), they used protein A as a sensitive and easily detectable target to study the cross-talk between both TCSs. Cross-talk between GraS and ArIR occurs in the presence of ArlS, indicating that the phosphatase activity of ArIS cannot prevent the phosphorylation of ArIR by GraS. Furthermore, cross-talk between GraR and ArIR occurs in response to natural stimuli because activation of GraS with colistin increased the production of protein A in the wild type and arlRS mutant strains, whereas the levels remained similar when *graRS* mutant is grown in the presence of colistin. ArlRS and GraSR are not closely related to one another suggesting that the cross-regulation between GraS and ArIR has been evolutionary selected and represent and integral signalling event that enables *S. aureus* to adapt more efficiently to environmental perturbations.

In summary, these studies provide the first example of a free living bacteria in which all non-essential TCSs (ΔXV) and even the whole TCS signalling system (ΔXVI) has been removed. This strategy is especially useful to elucidate the connectivity between environmental signals, the sensor responsible for its perception and the set of regulated genes. Also, it may aid to carry out studies to investigate the molecular mechanisms that govern the specificity between sensors and RRs and to understand the interdependency between different TCS. As the ΔXV mutant (or, more generically, the mutant devoid of all non-essential TCS) is still genetically manageable, it provides an excellent platform for the systematic analysis of the TCS network. Also the strain devoid of all TCS, but expressing WaIR (such as the *S. aureus* ΔXVI* strain disclosed in the present application) can be useful for such object, alone or in assays or practical application wherein it is combined with any other of the *S. aureus* mutant strains disclosed in the present application. Therefore, it is also an aspect of the present invention the use of any of the *S. aureus* strains of the present invention as research tools, for metabolic assays in general, particularly those aimed to elucidate the functioning and/or connectivity between environmental signals, particularly those associated to TCSs, and also for the study of *S. aureus* pathogenicity and infection mechanisms. Both the mutant devoid of all non-essential TCS (such as the ΔXV strain of the Examples of the present application) or the mutant devoid of all TCS, (such as the ΔXVI strain of the Examples of the present application) are particularly preferred for such use.

From a biotechnological point of view, both the senseless *S. aureus* mutant or the mutant devoid of all non-essential TCS can be an ideal bacterial chassis for synthetic biology applications. *S. aureus* ΔXV has a genome that encodes the basic biological functions required for self-maintenance and growth, but is deleted of signal-sensing components that divert resources. Under natural circumstances, signalling functions enable microorganisms to interact with their environment, but they may not be required in the synthetic biology laboratory or in biotechnological settings. *S. aureus* ΔXV is robust, stable (low spontaneous variability) and easily amenable to genetic manipulations. Thus, *S. aureus* ΔXV can be engineered to create novel connections between stimuli and gene expression networks to develop different types of useful engineered cells. The assays described in Example 5 shows the applicability of the ΔXV strain of the present invention for expressing heterologous proteins, that can be chimeric proteins if it is wished, and that the ΔXV strain is an appropriate platform for directing them to the desired cell compartment, such as the cell membrane or the cell wall, if appropriate sequences are included in the construction designed for transforming the strain. The methods for obtaining such constructions and the appropriate elements to include in them in order to achieve expression in the desired conditions or to direct the expressed protein to a particular compartment are well known for those skilled in the art. Also well known are the methods for transforming bacteria, particularly Gram positive bacteria such as *S. aureus,* with any kind of appropriate expression vector (plasmids, phages...) and /or for inserting an expression cassette in the bacterial chromosome, if desired. Therefore, the stability of the mutant strains of the present invention and their amenability to genetic manipulations, together with the corroboration of the assay of Example 5, are enough for supporting their applicability for the expression of any heterologous and/or chimeric protein, for any desired purpose. Such purpose can be, for instance, their inclusion in any study about *S. aureus,* such as studies about its metabolic pathways, sensing, pathogenicity or any other desired feature, or any other practical purpose not directly related to the study of *S. aureus* biological processes.

Moreover, the phenotypic characterization of the ΔXV strains (see Example 1) show that they are capable to adhere to human lung epithelial or hepatocyte cell lines as efficiently as the wild type strain, although they invade both epithelial cell lines less efficiently. The antimicrobial susceptibility of the MW2 ΔXV and 8325 ΔXV strains is different when both strains are compared, but it is also different from that of their parental strains. The assays of Example 1 confirm that the TC sensorial system is important for the pathogenesis of *S. aureus* infection and that its susceptibility to different antibiotics is modified by TCS inactivation.

This confirms the applicability of the mutant strains of the present invention for identifying compounds capable to block TCSs, which could be a means for identifying candidates to antibiotic compounds. The method used for it could be based on the assay set forth in Example 6, which also shows that controlling the expression of certain TCS, particularly the expression of WalKR, would allow the creation of a biosafe *S. aureus* strain able to grow only in the presence of the corresponding inductor, which again means a valuable tool for assays related to *S. aureus* pathogenicity and means for controlling *S. aureus* infection.

The obtained results also aimed at *S. aureus* mutant strains of the present invention, particularly ΔXV and ΔXVI strains, as candidates to being used as living attenuated mutant strains for vaccination purposes, since they are avirulent but still have a capability of invading and colonizing tissues.

Following a procedure analogous to that of Example 6, both the ΔXV strain whose generation is disclosed in the assays of the present application (or, more generally, a *S. aureus* strain devoid of all TCS excepting the essential for viability, WalKR) or a ΔXVI strain wherein the *walKR* operon is under the control of an inducible promoter (such as the Pspac promoter) but growing under conditions wherein an inductor of the promoter is present, could be a good platform for identifying compounds capable of blocking / inactivating the WalKR system, which could be a way of identifying compounds with antimicrobial potential. In such method for identifying potential antimicrobial compounds, those compounds whose addition to the culture media would give rise to growth inhibition of the mentioned ΔXV strain could be potential antimicrobial drugs. The reversion of the effect by the expression of the WalKR components (ectopically from a plasmid, for instance) would confirm that the effect is due to having inactivated the WalKR system. The sequential transformation of the ΔXV strain with plasmids expressing each one of the other TCS or assays with the strains ΔI, ΔII, ΔIII, ΔIV, ΔV, ΔVI, ΔVII, ΔVIII, ΔIX, ΔX, ΔXI, ΔXII, ΔXIII, ΔXIV and the wild type parental strain would confirm the specificity of the effect, the possibility of inactivating simultaneously several systems and the potentiality of the compound as antimicrobial / antibiotic compound, in case the effect could not be reverted in the parental wild type strain or by the reintroduction of any of the systems.

In conclusion, the present invention provides a set of *S. aureus* mutant strains that allow:
- firstly, a novel approach for the study of *S. aureus* sensing system, very useful for increasing the knowledge about the functioning, regulation and related phenotypic effects of such system and their components, which has allowed increasing the knowledge about different aspect of such sensing system, as it is demonstrated by the examples of the present application and the findings and teachings provided in them, and which will facilitate or enable the design of assays very difficult or not possible before the disclosure of the present invention which will provide teachings that will further increase the knowledge of *S. aureus* biology, many of them probably with direct or indirect applicability in the control of *S. aureus* infection and the development of compounds for controlling such infection and/or for palliating its symptoms;
- secondly, direct biotechnological applicability, due to the fact that they are stable under normal growing laboratory conditions and still genetically manageable, being capable of replicating plasmids, expressing heterologous proteins or internalizing external compounds that can be known or putative inductors or inactivators of different cell functions, the latter being directly linked with their possible application for the identification of candidates to antimicrobial compounds, particularly those targeting the TCSs, and the development of more potent or more specific antibiotics from such compounds.

### EXAMPLES

The assays set forth and discussed in the following Examples have been carried out with the following materials and methodologies:

### - Oligonucleotides, plasmids, bacterial strains and culture conditions

Bacterial strains, plasmids and oligonucleotides used in the assays of the present application are listed below in Tables 1, 2 and 3, respectively.

**Table 3. Bacterial strains used in the present assays**

| ***S. aureus* strains** | **Relevant characteristics** | **MIC^{a}** | **Reference** |
|---|---|---|---|
| 15981 | Clinical isolate | 532 | (Valle *et al*., 2003) |
| RN10359 (8325) | RN450 lysogenic for 80α phage | 1327 | (Ubeda *et al*., 2007) |
| ISP479r | ISP479c with *rsbU* gene restored | 1680 | (Toledo-Arana *et al*., 2005) |
| MW2 | WT (NC_003923) | 3566 | (Baba *et a*/., 2002) |
| MW2 Δ3 | MW2 strain with *MW0199-MW0198* genes deleted | 4032 | This study |
| MW2 Δ4 | MW2 Δ*lytSR* | 2964 | This study |
| MW2 Δ5 | MW2 Δ*graRS* | 11 | This study |
| MW2 Δ6 | MW2 Δ*saeRS* | 2965 | This study |
| MW2 Δ7 | MW2 ΔMW1207-MW1208 | 4033 | This study |
| MW2 Δ8 | MW2 Δ*arlRS* | 4034 | This study |
| MW2 Δ9 | MW2 Δ*srrAB* | 2966 | This study |
| MW2 Δ10 | MW2 Δ*phoRP* | 4035 | This study |
| MW2 Δ11 | MW2 Δ*yhcSR* | 3670 | This study |
| MW2 Δ12 | MW2 Δ*vraRS* | 4036 | This study |
| MW2 Δ13 | MW2 Δ*agrBDCA* | 4037 | This study |
| MW2 Δ14 | MW2 Δ*kdpDE* | 4038 | This study |
| MW2 Δ15 | MW2 Δ*hssRS* | 2979 | This study |
| MW2 Δ16 | MW2 Δ*nreABC* | 2967 | This study |
| MW2 Δ17 | MW2 Δ*nsaRS* | 4039 | This study |
| MW2 ΔI | MW2 *Δyhc* | 3670 | This study |
| MW2 ΔII | MW2 ΔI *Δtcs3* | 3713 | This study |
| MW2 ΔIII | MW2 ΔII *Δlyt* | 3717 | This study |
| MW2 ΔIV | MW2 ΔIII *Δgra* | 66 | This study |
| MW2 ΔV | MW2 ΔIV *Δsae* | 371 | This study |
| MW2 ΔVI | MW2 ΔV *Δtcs7* | 768 | This study |
| MW2 ΔVII | MW2 ΔVI *Δhss* | 1336 | This study |
| MW2 ΔVIII | MW2 ΔVII *Δnre* | 1345 | This study |
| MW2 ΔIX | MW2 ΔVIII *Δbra* | 1379 | This study |
| MW2 ΔX | MW2 ΔIX *Δkdp* | 2955 | This study |
| MW2 ΔXI | MW2 ΔX *Δvra* | 2956 | This study |
| MW2 ΔXII | MW2 ΔXI *Δpho* | 2957 | This study |
| MW2 ΔXIII | MW2 ΔXII *Δarl* | 2958 | This study |
| MW2 ΔXIV | MW2 ΔXIII *Δagr* | 2960 | This study |
| MW2 ΔXV | MW2 ΔXIV *Δsrr* | 2961 | This study |
| 8325 | WT (NC_007795) | 2968 | |
| 8325 ΔI | 8325 Δ*yhc* | 3999 | This study |
| 8325 ΔII | 8325 ΔI *Δlyt* | 4000 | This study |
| 8325 ΔIII | 8325 Δll *Δgra* | 4001 | This study |
| 8325 ΔIV | 8325 ΔIII *Δsae* | 4002 | This study |
| 8325 ΔV | 8325 ΔIV *Δtcs7* | 4003 | This study |
| 8325 ΔVI | 8325 ΔV *Δtcs3* | 4004 | This study |
| 8325 ΔVII | 8325 ΔVI Δ*arl* | 4005 | This study |
| 8325 ΔVIII | 8325 ΔVII Δ*srr* | 4006 | This study |
| 8325 ΔIX | 8325 ΔVIII Δ*pho* | 4007 | This study |
| 8325 ΔX | 8325 ΔIX *Δhss* | 4008 | This study |
| 8325 ΔXI | 8325 ΔX Δ*nre* | 4009 | This study |
| 8325 ΔXII | 8325 ΔXI Δ*bra* | 4010 | This study |
| 8325 ΔXIII | 8325 ΔXII Δ*vra* | 4011 | This study |
| 8325 ΔXIV | 8325 ΔXII I Δ*kdp* | 4012 | This study |
| 8325 ΔXV | 8325 ΔXIV Δ*agr* | 2969 | This study |
| MW2 ΔXVI | MW2 ΔXV *PspaC-walKR* | 2975 | This study |
| MW2 ΔXVI* | MW2 ΔXV Pspac-*walKR* pEI walR | 4674 | This study |
| MW2 ΔXV pEI *arlR* | MW2 ΔXV strain harbouring the pEI *arlR* plasmid | 4891 | This study |
| MW2 ΔXV pEI *arlRS* | MW2 ΔXV strain harbouring the pEI *arlRS* plasmid | 4538 | This study |
| MW2 Δ8 pEI | MW2 Δ8 strain harbouring the pEI plasmid | 4704 | This study |
| MW2 Δ8 pEI *arlR* | MW2 Δ8 strain harbouring the pEI *arlR* plasmid | 4890 | This study |
| MW2 Δ8 pEI *arlRS* | MW2 Δ8 strain harbouring the pEI *arlRS* plasmid | 4539 | This study |
| MW2 ΔXV pEI | MW2 ΔXV strain harbouring the pEI plasmid | 4682 | This study |
| MW2 ΔXV pEI *vraR* | MW2 ΔXV strain harbouring the pEI *vraR* plasmid | 4708 | This study |
| MW2 ΔXV pEI *vraSR* | MW2 ΔXV strain harbouring the pEI *vraRS* plasmid | 4676 | This study |
| MW2 Δ12 pEI | MW2 Δ12 strain harbouring the pEI plasmid | 4684 | This study |
| MW2 Δ12 pEI *vraR* | MW2 Δ12 strain harbouring the pEI *vraR* plasmid | 4889 | This study |
| MW2 Δ12 pEI *vraSR* | MW2 Δ12 strain harbouring the pEI *vraRS* plasmid | 4675 | This study |
| MW2 ΔXV pEI *srrA* | MW2 ΔXV strain harbouring the pEI *srrA* plasmid | 4678 | This study |
| MW2 ΔXV pEI *srrAB* | MW2 ΔXV strain harbouring the pEI *srrAB* plasmid | 4680 | This study |
| MW2 Δ9 pEI | MW2 Δ9 strain harbouring the pEI plasmid | 4683 | This study |
| MW2 Δ9 pEI *srrA* | MW2 Δ9 strain harbouring the pEI *srrA* plasmid | 4679 | This study |
| MW2 Δ9 pEI *srrAB* | MW2 Δ9 strain harbouring the pEI *srrAB* plasmid | 4677 | This study |
| MW2 ΔXV pEI *nreBC* | MW2 ΔXV strain harbouring the pEI *nreBC* plasmid | 4536 | This study |
| MW2 Δ16 pEI *nreBC* | MW2 Δ16 strain harbouring the pEI *nreBC* plasmid | 4535 | This study |
| MW2 ΔXV pEI *nreC* | ΔXV strain harbouring the pEI *nreC* plasmid | 3888 | This study |
| MW2 Δ16 pEI | Δ16 strain harbouring the pEI plasmid | 3893 | This study |
| MW2 Δ16 pEI *nreC* | Δ16 strain harbouring the pEI *nreC* plasmid | 3889 | This study |
| MW2 ΔXV pEI arlR *hk3* | ΔXV strain harbouring the pEI *arlR hk3* plasmid | 5041 | This study |
| MW2 ΔXV pEI arlR *lytS* | ΔXV strain harbouring the pEI *arlR lytS* plasmid | 5042 | This study |
| MW2 ΔXV pEI arlR *graS* | ΔXV strain harbouring the pEI *arlR graS* plasmid | 5043 | This study |
| MW2 ΔXV pEI arlR *saeS* | ΔXV strain harbouring the pEI *arlR saeS* plasmid | 5044 | This study |
| MW2 ΔXV pEI arlR *hk7* | ΔXV strain harbouring the pEI *arlR hk7* plasmid | 5045 | This study |
| MW2 ΔXV pEI arlR *srrA* | ΔXV strain harbouring the pEI *arlR srrA* plasmid | 5046 | This study |
| MW2 ΔXV pEI arlR *phoS* | ΔXV strain harbouring the pEI *arlR phoS* plasmid | 5047 | This study |
| MW2 ΔXV pEI arlR *yhcS* | ΔXV strain harbouring the pEI *arlR yhcS* plasmid | 5048 | This study |
| MW2 ΔXV pEI arlR *vraS* | ΔXV strain harbouring the pEI *arlR vraS* plasmid | 5049 | This study |
| MW2 ΔXV pEI arlR agrC | ΔXV strain harbouring the pEI *arlR agrC* plasmid | 5050 | This study |
| MW2 ΔXV pEI arlR *kdpD* | ΔXV strain harbouring the pEI *arlR kdpD* plasmid | 5051 | This study |
| MW2 ΔXV pEI arlR hssS | ΔXV strain harbouring the pEI *arlR hssS* plasmid | 5052 | This study |
| MW2 ΔXV pEI arlR *nreC* | ΔXV strain harbouring the pEI *arlR nreC* plasmid | 5053 | This study |
| MW2 ΔXV pEI arlR *hk17* | ΔXV strain harbouring the pEI arlR *hk17* plasmid | 5054 | This study |
| MW2 Δ8 pEI arlR (D52A) | MW2 Δ8 strain harbouring the pEI *arlR* (D52A) plasmid | 5001 | This study |
| MW2 Δ8 pEI *arIRS* (H242A) | MW2 Δ8 strain harbouring the pEI *arIRS* (H242A) plasmid | 4924 | This study |
| MW2 ΔXV pEI *arlR* (D52A) | ΔXV strain harbouring the pEI *arlR* (D52A) plasmid | 5002 | This study |
| MW2 ΔXV pEI arIRS (H242A) | ΔXV strain harbouring the pEI *arIRS* (H242A) plasmid | 4925 | This study |
| MW2 ΔXV pEI arlR (D52A) *graS* | ΔXV strain harbouring the pEI *arlR* (D52A) *graS* plasmid | 5043 | This study |
| Newman | Widely used laboratory strain | | (Duthie and Lorenz, 1952) |

| | | | |
|---|---|---|---|
| ^{a}Microbial Biofilm Laboratory strain collection number of the inventors. Samples available upon request | | | |

**Table 4: Plasmids used in the assays**

| Plasmids | Relevant characteristic(s) | Source of reference |
|---|---|---|
| pMAD | *E. coli-S. aureus* shuttle vector with the thermosensitive origin or replication for Gram-positive bacteria. The vector contains the *bgaB* gene encoding a b-galactosidase under the control of a constitutive promoter as reporter of plasmid presence. Ap^{R}, Em^{R}. | (Arnaud *et al.,* 2004) |
| pMAD TCS3AD | pMAD plasmid containing the mutant allele for deletion of the sequence | This study |
| pMAD TCS4AD | pMAD plasmid containing the mutant allele for deletion of the *lytRS* genes | This study |
| pMAD TCS5AD | pMAD plasmidcontaining the mutant allele for deletion of the *graRS* genes | This study |
| pMAD TCS6AD | pMAD plasmid containing the mutant allele for deletion of the *saeRS* genes | (Toledo-Arana *et al.,* 2005) |
| pMAD TCS7AD | pMAD plasmid containing the mutant allele for deletion of the sequence | (Toledo-Arana *et al.,* 2005) |
| pMAD TCS8AD | pMAD plasmid containing the mutant allele for deletion of the *arIRS* genes | (Toledo-Arana et *al*., 2005) |
| pMAD TCS9AD | pMAD plasmidcontaining the mutant allele for deletion of the *srrAB* genes | (Toledo-Arana et *al*., 2005) |
| pMAD TCS10AD | pMAD plasmid containing the mutant allele for deletion of the *phoRP* genes | (Toledo-Arana et *al*., 2005) |
| pMAD TCS11AD | pMAD plasmidcontaining the mutant allele for deletion of the *yhcRS* genes | This study |
| pMAD TCS12AD | pMAD plasmid containing the mutant allele for deletion of the *vraRS* genes | (Toledo-Arana *et al.,* 2005) |
| pMAD TCS13AD | pMAD plasmid containing the mutant allele for deletion of the *agrBDCA* genes | (Valle *et al.,* 2003) |
| pMAD TCS14AD | pMAD plasmid containing the mutant allele for deletion of the *kdpDE* genes | This study |
| pMAD TCS15AD | pMAD plasmid containing the mutant allele for deletion of the *hssRS* genes | (Toledo-Arana et *al*., 2005) |
| pMAD TCS16AD | pMAD plasmid containing the mutant allele for deletion of the *nreBC* genes | This study |
| pMAD TCS17AD | pMAD plasmid containing the mutant allele for deletion of the *braRS* genes | This study |
| pSD3-3 | Derivative of pDH88. Pspac-walKR | (Dubrac *et al.,* 2007) |
| pCN51 | *E. coli*-*S. aureus* shuttle vector to express genes under the control of the P_{cad} cadmium-inducible promoter. Low copy number (20 to 25 copies/cell). Em^{R}. Renamed pEI | (Charpentier *et al.,* 2004) |
| pEI *walR* | pCN51 plasmid expressing *walR* | This study |
| pEI *arlRS* | pCN51 plasmid expressing *arlRS* | This study |
| pEI *arlR* | pCN51 plasmid expressing *arlR* | This study |
| pEI *srrAB* | pCN51 plasmid expressing *srrAB* | This study |
| pEI *srrB* | pCN51 plasmid expressing *srrB* | This study |
| pEI *vraSR* | pCN51 plasmid expressing *vraSR* | This study |
| pEI *vraR* | pCN51 plasmid expressing *vraR* | This study |
| pEI *nreBC* | pCN51 plasmid expressing *nreBC* | This study |
| pCN40 | *E. coli* - *S. aureus* shuttle vector to express genes under the control of the *P_{blaZ}* constitutive promoter. Low copy number (20 to 25 copies/cell). Em^{R} | (Charpentier *et al.,* 2004) |
| pCN47 | *E. coli* - *S. aureus* shuttle vector to express genes under its own promoter. Em^{R} | (Charpentier *et al.,* 2004) |
| pEI *nreC* | pEW plasmid constitutively expressing *nreC* | This study |
| pEI *arlR hk3* | pCN51 plasmid expressing *arlR hk3* | This study |
| pEI *arlR lytS* | pCN51 plasmid expressing *arlR lytS* | This study |
| pEI *arlR graS* | pCN51 plasmid expressing *arlR graS* | This study |
| pEI *arlR saeS* | pCN51 plasmid expressing *arlR saeS* | This study |
| pEI *arlR hk7* | pCN51 plasmid expressing *arlR hk7* | This study |
| pEI *arlR srrB* | pCN51 plasmid expressing *arlR srrB* | This study |
| pEI *arlR phoR* | pCN51 plasmid expressing *arlR phoR* | This study |
| pEI *arlR yhcS* | pCN51 plasmid expressing *arlR yhcS* | This study |
| pEI *arlR vraS* | pCN51 plasmid expressing *arlR vraS* | This study |
| pEI *arlR agrC* | pCN51 plasmid expressing *arlR agrC* | This study |
| pEI *arlR kdpD* | pCN51 plasmid expressing *arlR kdpD* | This study |
| pEI *arlR hssS* | pCN51 plasmid expressing *arlR hssS* | This study |
| pEI *arlR braS* | pCN51 plasmid expressing *arlR braS* | This study |
| pEI *arlR (D52A)* | pCN51 plasmid expressing *arlR (D52A)* | This study |
| pEI *arlR (H242A)* | pCN51 plasmid expressing *arlR (H242A)* | This study |
| pEI *arlR (D52A) arlS* | pCN51 plasmid expressing *arlR (D52A) arlS* | This study |
| pEI *arlR (D52A) graS* | graSpCN51 plasmid expressing *arlR (D52A) graS* | This study |
| pEI-BapB | pCN51 plasmid with Bap-B domain fused to the C-terminal R domain of *clumping factor* A gene (R-clfA) | This study |
| pEI-BapB-SNAP | pCN51 plasmid with Bap-B domain fused to a SNAP-tag followed by R-clfAl domain, expressed under the cadmium inducible promoter Pcad-cadC | This study |

**Table 5: Oligonucleotides used in the assays**

| **Oligo nucleotide** | **Sequence** | **SEQ ID NO:** |
|---|---|---|
| tcs3-A | *GGATCC*GTTATCAGAAACAATTGATCA | 1 |
| tcs3-B | *CCATGG*TGAATCATCTCCAAAAATTTAT | 2 |
| tcs3-C | *CCATGG*ATTAGCACATAACTAATGATTA | 3 |
| tcs3-D | *CTCGAG*TAAAGGCGATGCAGTTAATA | 4 |
| lyt-A | *GGATCC*TGACGTTGAACAAACGAATA | 5 |
| lyt-B | *AAGCTT*GATAGCACCTCAGTGAAT | 6 |
| lyt-C | *AAGCTT*CAGTAATCCTTTTTTTTATGC | 7 |
| lyt-D | *GAATTC*CAACTGTACCGATACCAAT | 8 |
| gra-A | *GGATCC*CAAATAGATATTGCTGTATTC | 9 |
| gra-B | *CCATGG*CCATATCACCCAATATCATT | 10 |
| gra-C | *CCATGG*ACATGCGTTTTGTTACTTAG | 11 |
| gra-D | *CTCGAG*TTAACGCCACCTAAAACAC | 12 |
| sae-A | ATTTGAATGGATAGGC | 13 |
| sae-B | ATTACGTCATAATCCG | 14 |
| sae-C | AAATCGGATTATGACGTAATAAGTGGGTCATCTATTTTTTCACC | 15 |
| sae-D | TAACACTACAAATCGC | 16 |
| tcs7-A | *GGATCC*AAAGGGGATCTAATTATGATA | 17 |
| tcs7-B | *AAGCTT*ATTTTATTCCGCCCTTTTAAA | 18 |
| tcs7-C | *AAGCTT*ATACAAACAAAAAAGTATTGAG | 19 |
| tcs7-D | *GAATTC*AAAAATACTTCTCTCTAGCAA | 20 |
| arl-A | GTTTCCTTTTTGGAGG | 21 |
| arl-B | TCATGACTGAGACGTC | 22 |
| arl-C | GCGTCATTTGTACACC | 23 |
| arl-D | GTGATGAGGTTTAAAC | 24 |
| srr-A | *GGATCC*GTGAATTTACTAATTTAATGA | 25 |
| srr-B | *CTCGAG*GCCTCTTGGCCATTACTTGCT | 26 |
| srr-C | *CTCGAG*ATCGAAGAGCATGGTGGTTC | 27 |
| srr-D | *GAATTC*ATCTTCGCAATGCACATAAA | 28 |
| pho-A | TGGTAGTAAGATACCC | 29 |
| pho-B | AAAGTGGTAACAGCGC | 30 |
| pho-C | ACACGCGCTGTTACCACTTTGGTATGCCTCCCTAAC | 31 |
| pho-D | CACATGGTACAGCTCC | 32 |
| yhc-A | TTAAAATGTGTGTAATTGCA | 33 |
| yhc-B | CAAATCGCTCCAATTCATTT | 34 |
| yhc-C | AATGAGCTTTTAAATATTTGTC | 35 |
| yhc-D | GTGTGTTACATCGCTTTTA | 36 |
| vra-A | GTATTACCAGGTGCAG | 37 |
| vra-B | TCAATAGTTCGTATTG | 38 |
| vra-C | AATTCAATACGAACTATTGACGATAAATCACCTCTA | 39 |
| vra-D | ACGTGGCCTTTTGGCG | 40 |
| agr-A | AGCACTGAGTCCAAGG | 41 |
| agr-B | TTTTACACCACTCTCC | 42 |
| agr-C | GTGAGGAGAGTGGTGTAAAAAAGATAATAAAGTCAGTTAACGGC | 43 |
| agr-D | CAGTTATTAGCAGGAT | 44 |
| kdp-A | *GGATCC*CCAATGATATTAGTTAATCCA | 45 |
| kdp-B | *CCATGG*AACCTTCACCTCGATAGC | 46 |
| kdp-C | *CCATGG*TTAAATAAAAAAGATCGCTGC | 47 |
| kdp-D | *GAATTC*GTTTTCAATAATTGATTCTCTG | 48 |
| hss-A | CATTAATAGCGACCTC | 49 |
| hss-B | CTCCCTTATCTTTTTC | 50 |
| hss-C | AAATGAAAAAGATAAGGGAGTCTCTACCTCCTGAAA | 51 |
| hss-D | AGGTGTAGTGTGCATC | 52 |
| nre-A | *GGATCC*CGCAACATTAGTAACCAATAT | 53 |
| nre-B | *CCATGG*GACTTACACCCTAATTCATC | 54 |
| nre-C | *CCATGG*AGTTTGAAATTAATATAATTCAGT | 55 |
| nre-D | *CTCGAG*TTATAAACAGCAAGACTT AGAA | 56 |
| bra-A | *GGATCC*GCTGCAGAATCAGTAATATT | 57 |
| bra-B | *AAGCTT*CTATAATCTTCTTCCTTCAAT | 58 |
| bra-C | *AAGCTT*AAACTTTCAATATTGTAAGCATA | 59 |
| bra-D | *GAATTC*GTGCCATAACAATCTTAACT | 60 |
| tcs3-E | ATCTACTCAAGTACTGCTTT | 61 |
| tcs3-F | TGTTTCAAACGTCTTTGTATA | 62 |
| lyt-E | GAAAAGAAAAATGTAGATTTGA | 63 |
| lyt-F | AATAACATCATTGGCAAATTG | 64 |
| gra-E | AAATGACTTGGATTCAGTGT | 65 |
| gra-F | AAAAAGATAGATGGCATAATG | 66 |
| sae-E | GTTGGTGATTTTAGACTTTTA | 67 |
| sae-F | ATAAGATTGTAGAGCACATAA | 68 |
| tcs7-E | AATTGCCGGAAGATGTTAAA | 69 |
| tcs7-F | TATCTTTGTAGGCTTTATCG | 70 |
| arl-E | AGTGATCTGAAACAATTCC | 71 |
| arl-F | AAGAATAGTAAATAAAACGC | 72 |
| srr-E | TTTGTACAAAGGTAGACTTG | 73 |
| srr-F | TGGTTACGGATTACTTTAAAG | 74 |
| pho-E | AATGAGACCATATGAAAGCC | 75 |
| pho-F | CAGGTGCAAACATTAATTATG | 76 |
| yhc-E | CATATAAAGGATCACCAATAA | 77 |
| yhc-F | CATAGTTATTCATTATACCAC | 78 |
| vra-E | TGACGAACAAGTGAAATGG | 79 |
| vra-F | CGTTCTATTATTGGGATGTG | 80 |
| agr-E | GGGGATGTTATTAATTATGAA | 81 |
| agr-F | TAGTCATTTATACGAAGGGA | 82 |
| kdp-E | TACTAATTAAACATGATAATGG | 83 |
| kdp-F | GAATTCGTTTTCAATAATTGATTCTCTG | 84 |
| hss-E | CAT ACATT GT GTCGTTTAAAA | 85 |
| hss-F | AACCAATGATTAAGCTAATAAA | 86 |
| nre-E | TTAAGTTCAGCGTCGGATAT | 87 |
| nre-F | AACTTTACATTATTACGATGAAA | 88 |
| bra-E | TACTTTCTGCTTGTTACTGT | 89 |
| bra-F | ACACAAGCGTATATTCAATC | 90 |
| Constitutive expression of TCS | | |
| SaeR-Rv (BamHI XmaI KpnI) | GGTACCAATACCCGGGCAATGGATCCATTATCGGCTCCTTTCAAAT | 91 |
| SaeR-Fw (XhoI SalI) | *GTCGA* CATTGCT *CGA* GCGAACAGAGGT GAAAAAAT AG | 92 |
| NreR-Rv (BamHI XmaI KpnI) | GGTACCAATACCCGGGCAATGGATCCAATTTCAAACTCTAAAACTCT A | 93 |
| NreR-Fw (XhoI SalI) | GTCGACATTGCTCGAGCATACATTGGGGGAATAAAA | 94 |
| WaIR-Rv (BamHI XmaI KpnI) | GGTACCAATACCCGGGCAATGGATCCCTCTACTCATGTTGTTGGA | 95 |
| WaIR-Fw (XhoI SalI) | *GTCGA* CATTGCT *CGA* GTT AAGAAAAGAGGTTTATGC | 96 |
| TCS3R-Rv(BamHI XmaI KpnI) | *GGTACC*AATA*CCCGGGC*AAT*GGATCC*AATCTATTTTGCTTGCTTAC | 97 |
| TCS3R-Fw (XhoI SalI) | GTCGACATTGCTCGAGTTCAAGGGGGAATGTAGAT | 98 |
| LytR-Rv (BamHI XmaI KpnI) | GGTACCAATACCCGGGCAATGGATCCACTGTTAAAGTAACCCTATC | 99 |
| LytR-Fw (XhoI SalI) | *GTCGA* CATTGCT *CGA* GGACAAGAGGAGGAAT AAAT A | 100 |
| GraR-Rv (BamHI XmaI KpnI) | *GGTACC*AATA*CCCGGG*CAAT*GGATCC*CAAATTATTCATGAGCCATA | 101 |
| GraR-Fw (XhoI SalI) | *GTCGACA*TTG*CTCGA*GAATGATATTGGGTGATATGG | 102 |
| TCS7R-Rv(BamHI XmaI KpnI) | GGTACCAATACCCGGGCAATGGATCCATTTAGATCCAGCCTTTTTC | 103 |
| TCS7R-Fw (XhoI SalI) | *GTCGAC*ATTG*CTCGAG*AACAGGAGGAATAGCATGA | 104 |
| ArIR-Rv (BamHI XmaI KpnI) | *GGTACC*AATA*CCCGGG*CAAT*GGATCC*TTTGTCATCGTATCACATAC | 105 |
| ArIR-Fw (XhoI SalI) | GTCGACATTGCTCGAGGTAATATGAGGTGTACAAAT | 106 |
| SrrA(R)-Rv(BamHI XmaI KpnI) | GGTACCAATACCCGGGCAATGGATCCACTATTTAGCCGGCTCATC | 107 |
| SrrA(R)-Fw (XhoI SalI) | *GTCGAC*ATTG*CTC*GAGTGTGTGGGAGGTATGACC | 108 |
| PhoP(R)-Rv (BamHIXmaI KpnI) | *GGTACC*AATA*CCCGGG*CAAT*GGATCC*TCATCATTGTTCTTTAGGTC | 109 |
| PhoP(R)-Fw (XhoI SalI) | *GTCGACA*TTG*CTCGAGA*TAAGTTAGGGAGGCATAC | 110 |
| YhcR-Rv (BamHI XmaI KpnI) | *GGTACC*AATA*CCCGGGC*AAT*GGATCC*TTCTAAATCAACTTATTTTCC | 111 |
| YhcR-Fw (XhoI SalI) | *GTCGACA*TT*GCTCGA*GATTTAAGGAGATAACCCATG | 112 |
| VraR-Rv (BamHI XmaI KpnI) | GGTACCAATACCCGGGCAATGGATCCGAACTATTGAATTAAATTATGT | 113 |
| VraR-Fw (XhoI SalI) | GTCGACATTGCTCGA GAAATAAGGAGGATTCGTATG | 114 |
| AgrA(R)-Rv(BamHI XmaI KpnI) | GGTACCAATACCCGGGCAATGGATCCTATCTTATTATATTTTTTTAAC G | 115 |
| AgrA(R)-Fw (XhoI SalI) | *GTCGACA*TTG*CTCGAGC*ATAAGGATGTGAATGTATG | 116 |
| KdpE(R)-Rv(BamHI XmaI KpnI) | *GGTACC*AATA*CCCGGG*CAAT*GGATCC*ATTATTTCTCTTTCCACTGC | 117 |
| KdpE(R)-Fw (XhoI SalI) | *GTCGAC*ATTG*CTCGA*GAATGAAGGAGACGTATAATG | 118 |
| HssR-Rv (BamHI XmaI KpnI) | *GGTACC*AATA*CCCGGG*CAAT*GGATCC*TTTAAACATGATTCTCCACC | 119 |
| HssR-Fw (XhoI SalI) | *GTCGACA*TTG*CTCGAG*GATAAGGGAGTTTATAGCTA | 120 |
| BraR-Rv(BamHIXmaI KpnI) | *GGTACC*AATA*CCCGGG*CAAT*GGATCC*CCTATACTTTATATCCGACA | 121 |
| BraR-Fw (XhoI SalI) | *GTCGACA*TT*GCTCGA*GTTGAAGGAAGAAGATTATAG | 122 |
| SaeS-Rv (XmaI AscI) | GGCGCGCCCGGGATCGGATTATGACGTAATGT | 123 |
| SaeS-Fw (BamHI) | *GGATCCATTTG* AAAGGA GCCGATAAT | 124 |
| NreS-Rv (XmaI AscI) | GGCGCGCCCGGGGTATGTTTCAAATTGGAATGT | 125 |
| NreS-Fw (BamHI) | GGATCCAGATGAATTAGGGTGTAAGT | 126 |
| WaIK(S)-Rv (XmaI AscI) | GGCGCGCCCGGGCCTTATTATTCATCCCAATC | 127 |
| WaIK(S)-Fw (BamHI) | GGATCCATGAGTAGAGGTCGAAACG | 128 |
| TCS3S-Rv (XmaI AscI) | GGCGCGCCCGGGTACCTTAAACATCTACATTC | 129 |
| TCS3S-Fw (BamHI) | *GGATC* CTTTTT GGAGATGATTCAATG | 130 |
| LytS-Rv (Xmal AscI) | GGCGCGCCCGGGTATTTATTCCTCCTCTTGTC | 131 |
| LytS-Fw (BamHI) | GGATCCAATTTACTGAGGTGCTATCG | 132 |
| GraS-Rv (XmaI-AscI) | GGCGCGCCCGGGCGCATGTTTAAAATGACAAA | 133 |
| GraS-Fw (BamHI) | GGATCCTAGGAAAAGGATATATGGCT | 134 |
| TCS7S-Rv (XmaI AscI) | *GGCGCGCCCG*GGAAAGATGTCATGCTATTCCT | 135 |
| TCS7S-Fw (BamHI) | GGATCCAAAGGGCGGAATAAAATATG | 136 |
| ArIS-Rv (XmaI AscI) | GGCGCGCCCGGGGATTAAAATATGATTTTAAACG | 137 |
| ArIS-Fw (BamHI) | GGATCCTGGCGTTGGGTATGTGATA | 138 |
| SrrB(S)-Rv (XmaI AscI) | GGCGCGCCCGGGCAATTTTATTCTGGTTTTGG | 139 |
| SrrB(S)-Fw (BamHI) | *GGATCC*ATTTGAGGTTAAATCTAATG | 140 |
| PhoR(S)-Rv (Xmal AscI) | GGCGCGCCCGGGTTTTATTCTTTATAATCTTTTAG | 141 |
| PhoR(S)-Fw (BamHI) | *GGATCC*ATTGGAAAGACCTAAAGAAC | 142 |
| YhcS-Rv (XmaI AscI) | *GGCGCGCCC*GGGGCTATTTTATAGGAATTGTG | 143 |
| YhcS-Fw (BamHI) | *GGATCC*AAATGAATTGGAGCGATTTG | 144 |
| VraS-Rv (Xmal AscI) | *GGCGC*G*CCCGGG*CTTTAATCGTCATACGAATC | 145 |
| VraS-Fw (BamHI) | *GGATCC*GAGACGTAGAGGTGATTTAT | 146 |
| AgrC(S)-Rv (XmaI AscI) | GGCGCGCCCGGGGGCTAGTTGTTAATAATTTC | 147 |
| AgrC(S)-Fw (BamHI) | *GGATCC*TATAAGAGAAAGTGTGATAG | 148 |
| KdpD(S)-Rv (XmaI AscI) | *GGCGCGCCCGGG*CATTATACGTCTCCTTCATT | 149 |
| KdpD(S)-Fw (BamHI) | *GGATCC*TATCGAGGTGAAGGTTATG | 150 |
| HssS-Rv (XmaI AscI) | *GGCGCGCCCGGG*AGATTAAAGTGAATTATTTGG | 151 |
| HssS-Fw (BamHI) | *GGATCCC*AAGGCTATAAGGTGGAGA | 152 |
| BraS-Rv (XmaI AscI) | *GGCGCGCCCGGG*TTTTTATTCATCTGGAAATTG | 153 |
| BraS-Fw (BamHI) | GGATCCAGTATAGGGTGAATGCAATG | 154 |

*S*. *aureus* strains were grown in trypticase soy broth supplemented with 0.25% glucose (TSB-gluc) (Pronadisa). *Escherichia coli* was grown in LB broth (Pronadisa). When required for selective growth, medium was supplemented with appropriated antibiotics at the following concentrations: erythromycin (Em), 1.5 µg/ml and 10 µg/ml; ampicillin (Amp), 100 µg/ml, chloramphenicol (Clo), 10 µg/ml and 20 µg/ml.

### - Electrocompetent Staphylococcal cells

*Staphyloccocal* electrocompetent cells were generated as previously described (Schenk and Laddaga, 1992). Briefly, bacteria were grown in 200 ml of B2 broth at 37°C under shaking conditions (200 rpm) until the culture reached an OD₆₀₀ₙₘ of 0.5. Culture was incubated for 15 min on ice. Culture was centrifuged and the pellet was washed three times with sterile water and once with 30 ml of ice-cold 10% glycerol. The pellet was resuspended into 15 ml of ice-cold 10% glycerol and incubated for 15 min at 20°C. Culture was centrifuged and bacterial pellet was resuspended into 200 µl of ice-cold 10% glycerol. Aliquots of 50 µl were performed and stored at -80°C. Plasmids were transformed into staphylococci by electroporation, using a previously described protocol (Cucarella *et al.,* 2001).

### - Preparation of Φ80 transducing lysates

For the donor lysate preparation, 50 ml of the S. aureus RN4220 overnight culture (containing pMAD plasmid to be transduced) were diluted in 5 ml of the phage broth (containing CaCl₂). 200 ml of phage dilutions 10⁵ 10⁶ 10⁷ 10⁸ (in phage broth containing CaCl2) were added to 300 ml of the donor cells and the mixture was incubated at room temperature for 30 min. 10 ml of molten phage top agar (containing CaCl₂) at 55°C were added to the tubes and immediately the liquid was poured over the surface of 2 phage base plates (containing CaCl₂). Plates were incubated overnight at 37°C. Next day the top layer containing the phage plaques was scraped off with a Pasteur pipette into a falcon of 50 ml. Falcons were centrifuged and the supernatant was filtered through 0.45 mm filter and stored at 4°C.

### - Phage Transduction

For the phage transduction, the recipient bacteria were grown in 20 ml of TSB overnight. Culture was centrifuged and resuspended in 1 ml of TSB. 500 ml of the culture were mixed with 1 ml of LB (containing CaCl₂) and 500 ml of the phage lysate. There was also a control sample that had no phages. Mixture was incubated at 37°C for 25 min on a water bath and then at 37°C for 15 min in a orbital shaker. Reaction was stopped on ice and 1 ml of cold 0.02M Na citrate was added. The tube was centrifuged and the pellet was resuspended in 1 ml of 0.02M Na citrate and incubated on ice for 2 h. The bacterial solution was spread onto five TSA plates (200 ml per plate) containing Erytromicin 1.5 µg/ml (Sigma Aldrich) and Na citrate.

### - Allelic exchange of chromosomal genes

To generate deletion mutants, the inventors implified by PCR two fragments of 500 bp that flanked the left (primers A and B, see Table 5, SEQ ID NO:1 to SEQ ID NO:60) and right sequences (primers C and D, see Table 5, SEQ ID NO:1 to SEQ ID NO:60) of the region targeted for deletion. The PCR products were purified and cloned separately in the pGEM-T Easy vector (Promega). Fragments were then fused by ligation into the shuttle vector pMAD (Arnaud *et al.,* 2004). The resulting plasmid was transformed into *S*. *aureus* 15981 or MW2 strains by electroporation. Homologous recombination experiments were performed as described (Valle *et al.,* 2003). Erythromycin sensitive white colonies, which no longer contained the pMAD plasmid, were tested by PCR using primers E and F (see Table 5, SEQ ID NO:61 to SEQ ID NO:90).

### - Genome sequencing of the MW2 and MW2 ΔXV strains by Illumina™

To exclude the possibility that spontaneous mutations might have emerged during the construction of the ΔXV strains to suppress essential functions of TCS, the ΔXV strains were re-sequenced. Comparison of ancestor and ΔXV mutant identified a total of 9 SNPs for MW2 and 5 SNPs for 8325. The absence of common mutations between both strains confirmed that none of the genetic changes had an influence on the behavior of the ΔXV strain. For Illumina™ Next Generation Sequencing, genomic DNA was prepared from an overnight culture of MW2, MW2 ΔXV, 8325 and 8325 ΔXV grown in TSB-gluc at 37°C, as described previously (Marmur, 1961) and sequenced on an Illumina Hi-Seq 2000 instrument (Genomics Platform of CIBIR, La Rioja, Spain). Reads (6M-9M 150-bp) were assembled de novo using de Bruijn graph alignment Tools (ABBYS, VELVET, SOAP2) checking different K sizes to ensure the best coverage. SOAP2 was the algorithm that gave the best N50 and therefore the inventors used it on all the samples. The reads were assembled into 466 contigs (>100 bp in size) corresponding to 2864441 bp in length, with a N50 value of 16063 and a GC content of 32.67 % for MW2, 236 contigs (>100 bp in size) corresponding to 2856644 bp in length, with a N50 value of 50702 and a GC content of 32.76 % for MW2 ΔXV, 283 contigs (>100 bp in size) corresponding to 2839577 bp in length, with a N50 value of 60685 and a GC content of 32.80 % for 8325 and 292 contigs (>100 bp in size) corresponding to 2807518 bp in length, with a N50 value of 79329 and a GC content of 32.80 % for 8325 ΔXV. Subsequently contigs were ordered with Mauve obtaining a coverage of 99% in MW2 and 98% in 8325 (Rissman *et al.,* 2009). Variant base calling of the mutant ΔXV relative to MW2 and 8325 was performed with SAMTOOLS (H Li *et al.,* 2009). Finally genomic reorganization, insertions and deletions were studied with the Breseq pipeline (Deatherage and Barrick, 2014).

### - Construction of the insertional mutation of walKR operon

To generate a strain in which the *walKR* operon was placed under the control of the inducible *Pspac* promoter, a 432-bp fragment generated by PCR with primers OSA34 (5'-TCTAGATATTAATGATTTAAGAAAAGAGG-3', SEQ ID NO:155) and OSA35bis (5'-AGATCTCCAGTGTCTTGTGCTGGTTGTGAG-3', SEQ ID NO:156), carrying the ribosome-binding site and 5' portion of *yycF,* was digested with *Xba*I and *Bgl*II and cloned into pDH88 to generate pSD3-3 (Dubrac *et al.,* 2007). The circular plasmid was then transformed into S. *aureus* strain ΔXV, and integration of the plasmid through a single crossover event generated strain S. aureus ΔXVI, in which the *yycF* operon was placed under *Pspac* control. Integration of the plasmid through a single crossover event generated strains MW2 Pspac-walKR and ΔXV Pspac-walKR in which the entire *walKR* operon is under the control of the Pspac iso-propyl-P-D-thiogalactopyranoside (IPTG)-inducible promoter.

*S*. *aureus* strains were grown in Trypticase soy broth (TSB) supplemented with chloramphenicol (10 µg/ml).

### - Transcriptome analysis

*--RNA extraction:* Bacteria were grown in TSB-gluc at 37ºC under shaking conditions (250 rpm) until the culture reached an OD₆₅₀ₙₘ of 0,8. Total RNA from bacterial pellets was extracted using TRIzol reagent method as previously described (Toledo-Arana *et al.,* 2009). Briefly, bacterial pellets were resuspended into 400 µl of solution A (Glucose 10%, Tris 12.5 mM pH7.6, EDTA 10 mM), and mixed to 60 µl of 0.5M EDTA. Resuspended cells were transferred into Lysing Matrix B tubes (MP Biomedicals) containing 500 µl of acid phenol, pH 4,5 (Ambion) and mixed. Bacteria were mechanically lysed using the Fastprep apparatus (BIO101) at speed 6.0 during 45 seconds at 4ºC. After lysis, tubes were centrifuged 10 min at 14,000 rpm at 4ºC. The aqueous phase was transferred to 2-ml tubes containing 1 ml TRIzol, mixed and incubated for 5 min at room temperature. 100 µl of chloroform was added, mixed gently and incubated for 3 min at room temperature. Tubes were centrifuged for 10 min at 14,000 rpm at 4°C. The aqueous phase was transferred into a 2-ml tube containing 200 µl of chloroform, mixed and incubated for 5 min at room temperature. Tubes were centrifuged for 5 min at 14,000 rpm at 4°C. RNA contained in the aqueous phase was precipitated by addition of 500 µl of isopropanol and incubated 15 min at room temperature. Tubes were centrifuged for 15 min at 14,000 rpm at 4°C. RNA pellets were washed with 75% ethanol. Dried RNA pellets were resuspended in DEPC-treated water. RNA concentrations were quantified and RNA qualities were determined using Agilent RNA Nano LabChips (Agilent Technologies). RNAs were stored at -80°C until needed.
*--RNA sequencing:* Total RNA was extracted after lysing bacteria in the Fastprep homogenizer (Bio101) using TRIzol reagent (Invitrogen). RNA libraries for Illumina™ sequencing were created using the TruSeq Stranded Total RNA library prep kit (Illumina). 10 µg of total RNA was depleted from 16S and 23S RNAs using the MicroExpress kit according to the instructions of the manufacturer (Ambion).
*--cDNA libraries for RNA sequencing, read mapping and statistics analysis.* Deep sequencing of RNAs from *S*. *aureus* 15981 strain was performed as previously described (Lasa *et al.,* 2011). Mapped reads were included in .wig files to be loaded at the *S*. *aureus* transcriptome browser (http://staph.unavarra.es/).

### - Construction of plasmids expressing Histidine Kinases (HK) and/or Response Regulators (RR) under the control of different promoters

To construct the plasmids expressing RRs the inventors amplified them by PCR using forward (Fw) and reverse (Rv) primers (see Table 5, SEQ ID NO:91 to SEQ ID NO:122) and the MW2 chromosomal DNA as template. Forward primers carried SalI-XhoI tail and reverse primers carried BamHI-XmaI-KpnI tail. The PCR products were amplified with Phusion® High-Fidelity DNA Polymerase (Fermentas-Thermo Scientific), purified and cloned separately in pCR®-Blunt II TOPO vector (Invitrogen). Fragments were then ligated using SalI and Kpnl enzymes in the vectors pEW and pEI.

Similarly, the HKs were amplified by PCR with the forward and reverse primers (see Table 5, SEQ ID NO:123 to SEQ ID NO:154) and the MW2 chromosomal DNA as template. Forward primers carried BamHI tail and reverse primers carried Xmal-Ascl tail. The PCR products were amplified with Phusion® High-Fidelity DNA Polymerase (Fermentas- Thermo Scientific), purified and cloned separately in pCR®-Blunt II TOPO vector (Invitrogen).

For the construction of the plasmids expressing a combination of HKs and RRs, TOPO HKn plasmids were digested with BamHI and Xmal enzymes and the BamHI/XmaI module, containing the HK, was transferred to the corresponding plasmids, pEW RRn or pEI RRn. Modules containing RRs and HKs can be moved together from one plasmid to another by digestion with SalI or Xhol and Xmal or KpnI.

### - Antibiotic Resistance

Antibiotic sensitivity tests were performed at the University Clinic of Navarra. Antibiotics routinely used in clinic for treatment of *S*. *aureus* infections were tested by VITEK 2 system.

### - Growth kinetics analysis

In order to compare growth kinetics, overnight cultures of the strains were serially diluted (10⁻¹, 10⁻², 10⁻³, 10⁻⁴, 10⁻⁵ and 10⁻⁶) in TSB-gluc and 5 µl of no diluted, 10⁻², 10⁻⁴ and 10⁻⁶ diluted cultures were spotted onto TSA plates. The plates were then incubated at 28ºC, 37ºC and 44°C overnight and representative pictures were taken with a digital camera.

### - General metabolism

API Staph galleries (Biomerieux) were used to analyze bacterial metabolic patterns, according to the manufacturer's instructions. Homogeneous bacterial suspensions with a turbidity equivalent to 0.5 McFarland were prepared using the API Staph Medium. The microtubes were then filled with the bacterial suspensions and the strip was incubated inside the incubation box at 37°C for 24 h. The changes in the bacterial metabolic patterns were analyzed comparing the corresponding mutants with the wild type *S*. *aureus* strain.

### - Quantification of nitrite production

Extracellular concentrations of nitrite were determined colorimetrically by the Griess Reagent System (Promega) according to the manufacturer's instructions. Conditions of decreased oxygen tension were created by growing the bacterial strains in 15-ml Falcon tubes that were completely filled with medium (Schlag *et al.,* 2008). Potassium nitrate (KNO₃) was added to the medium at 20 mM. A purple/magenta color is being indicative of the presence of nitrites in the media. Absorbance at OD₅₀₉ₙₘ was measured within 30 minutes in the Original Multiskan EX (Labsystems) microplate reader.

### - Desiccation Experiments

The desiccation experiment was adapted from a protocol as described (White and Surette, 2006). Briefly, 100 µl from overnight cultures grown in TSB-gluc medium at 37°C were tested immediately (initial numbers) or air dried and stored in 24-well tissue culture plates at room temperature for 21 days. After rehydration of bacteria in 500 µl TSB-gluc, the number of viable cells remaining in each sample was determined by serially diluting cell mixtures and plating in duplicate. The average and SD of three independent assays were recorded.

### - Triton resistance test

Gradient plates (0 to 0.5 %) were used to compare resistance phenotypes for Triton X-100 (USB) (Mccallum *et al.,* 2011). OmniTray single well plates (Nunc) were filled with triton containing agar and a slope was created when the media was still in liquid state. The triton gradient was obtained upon filling the plate with trypticase soy agar. Overnight cultures of the test strains were diluted in TSB-gluc to OD₆₅₀ₙₘ = 0.4 and the cell suspensions were swabbed across agar plates containing triton concentration gradients. Plates were incubated at 37°C for 24 hours. Triton sensitivity is observed as a growth deficiency, primarily pronounced on the concentrated area of the plate.

### - Cell adhesion and invasion assay

Human hepatic Hep3B and epithelial A549 cells were used for this study. Adherence and invasion experiments were performed as described previously (Valle *et al.,* 2003). Briefly, prior to use, wells were seeded with 0.3 x 10⁶ cells in 6-well tissue culture plates and 0.5 x 10⁵ cells in 24-well tissue culture plates. Once cells were confluent (1.2 x 10⁶ or 0.2 x 10⁵ cells per well) the culture medium was removed and cells were washed with Dulbecco's modified Eagle's medium (DMEM) (Gibco-BRL) plus 10% heat- inactivated fetal bovine serum. For adherence assays, overnight bacterial cultures were mixed vigorously and added to the monolayer cells in a multiplicity of infection of 10 in DMEM. Incubation was carried out 1 hour at 37°C in 5% CO₂. To remove non-adherent bacteria, cells were washed three times with sterile PBS. Eukaryotic cells were lysed with 0.1% Triton X-100. Before plating extracts were mixed vigorously by vortexing and sonication. The number of adherent bacteria (CFU) was determined by serial dilution, plating and CFU counts. For invasion assays, bacteria were added to the monolayer cells in a multiplicity of infection of 40 in DMEM. Incubation was carried out for 1 hour at 37°C in 5% CO₂. To kill extracellular bacteria, media was replaced with 2 ml of DMEM containing 50 µg/ml of gentamicin (SIGMA) for 2 hour. Cell monolayers were washed three times with sterile PBS and lysed with 0.1% Triton X-100. Before plating extracts were mixed vigorously by vortexing and sonication. The number of intracellular bacteria was determined by serial dilution and plating. Experiments were performed in triplicate and repeated four times for each cell line.

### - Mouse infection model

MW2 wild-type, ΔXV, ΔXIV (with *agr)* and ΔXIV (with *srrAB*) *S. aureus* strains were cultured overnight in TSB plates and a single colony was resuspended into 5 ml of PBS to an optical density at 650nm of 0.2 (1 x 10⁸ cfu/ml). For the infection model, CD1 mice (Charles River) were inoculated by eye vain injection with 100 µl (1 x 10⁷ cells) of these cultures of staphylococcal strains. Seven mice were used for each strain group. After one week mice were euthanized. Kidneys were removed, homogenized in PBS (9 ml per gram), and plated on tryptic soy agar for determination of the number of colony-forming units (CFU).

### - Western Blot analysis. Quantification of Protein A expression

Overnight cultures of the strains were diluted in TSB-gluc to an OD₆₅₀ₙₘ = 0.1 and 50 µl of the diluted cultures were mixed with 1950 µl of chemically defined medium HHWm (Hussain-Hastings-White modified medium) (Toledo-Arana *et al.,* 2005) to inoculate sterile 24-well polystyrene microtitre plates (Costar). Plates were incubated at 37°C for 24 hours, trying to mimic biofilm forming conditions. Eight ml of bacterial cultures were centrifuged and pellets were resuspended in 60 µl PBS. Then, 2 µl of Lysostaphin 1 mg/ml (Sigma) and 3 µl of DNase I 1mg/ml (Sigma) were added. After 2 h of incubation at 37°C cell lysates were centrifuged and supernatants were collected. Protein concentration was determined with the Bio-Rad protein assay (Bio-Rad). Samples were adjusted to 3-5 µg of total protein and one volume of Laemmli buffer was added. Total protein extracts were denatured by boiling at 100°C for 5 min. Proteins were separated on 10% SDS-polyacrylamide gels and stained with 0.25% Coomassie brilliant blue R250 (Sigma) as loading controls. For Western blotting, proteins were transferred onto Hybond-ECL nitrocellulose membranes (Amersham Biosciences) by semi-dry electroblotting. Membranes were blocked overnight with 5% skimmed milk in phosphate-buffered saline (PBS) with 0.1% Tween 20, and incubated with goat anti-mouse secondary antibodies labelled with phosphatase alkaline (Sigma) diluted 1:2500 for 2 h at room temperature. Protein A was detected with the SuperSignal West Pico Chemiluminescent Substrate (Thermo Scientific).

### - Construction of the Bap-Snap-CIfA quimeric protein and expression in S. aureus ΔXVI

The signal peptide (SP) and the Bap-B domain of the *bap* gene were amplified from *S*. *aureus* V858, using the pairs of primers Bapori-1mB (5'-GGATCCTTTATTTTGAGGTGAGTAAATATGGG-3', SEQ ID NO:157) containing a recognition sequence for BamHI / Bap-65c (5'-GGCTCTTTAATTGAATTAGATGAAGCACTATTTTGTACTTCCGC-3', SEQ ID NO:158), and Bap-66m (5'-GCGGAAGTACAAAATAGTGCTTCATCTAATTCAATTAAAGAGCC-3', SEQ ID NO:159) / Bap-63cK (5'-GGTACCGGTGCCTTCTGGTGAATTTGG-3', SEQ ID NO:160) containing a recognition sequence for Kpnl. A second overlapping PCR was performed with primers Bapori-1mB (SEQ ID NO:157) and Bap-63cK (SEQ ID NO:160) to obtain a single fragment. To allow anchoring of amplified B-Bap domain to the bacterial cell wall, the R region of clumping factor A gene containing an LPXTG motif was amplified from *S*. *aureus* Newman strain using primer K-ClfA (5'-GGTACCGAAATTGAACCAATTCCAGAGGAT-3', SEQ ID NO:161) with a recognition sequence for Kpnl, and primer CIfA-7cE (5'-GAATTCTTACACCCTATTTTTTCGCC-3', SEQ ID NO:162) with a recognition sequence for EcoRI. The BamHI/KpnI-restricted SP-B-Bap and KpnI/EcoRI-restricted R-clfA were ligated into the shuttle vector pCN51 that contains the Pcad-cadC promoter (Charpentier *et al.,* 2004), giving the pCN51-BapB plasmid. The SNAP-Tag was amplified from pSNAP-tag(T2)-2 plasmid (New England Biolabs) using the pair of primers Snap-Fw (5'- GGTACCATGGACAAAGATTGCGAAATG-3', SEQ ID NO:163) / Snap-Rv (5'- GGTACCTCCCAGACCCGGTTTACCCAG-3', SEQ ID NO:164). The Kpnl-restricted SNAP-tag was ligated into Kpnl-restricted pCN51-BapB plasmid. The final pCN51-BapB-SNAP plasmid contains Bap-B domain fused to a SNAP-tag followed by the C-terminal R domain of *clumping factor* A gene, expressed under the activity of a cadmium inducible promoter.

### - BapB-SNAP-ClfA protein preparation and Western blotting

Surface protein preparations were obtained from *S*. *aureus* cells under isosmotic conditions. Briefly, overnight cultures of the strains tested were diluted 1:100 in TSB-gluc, and 20 ml of this cell suspension were grown in 125 ml flasks until OD₆₀₀ₙₘ reached 0.8. Ten ml of bacterial cultures were centrifuged and pellets were resuspended in 100 µl of isosmotic digestion buffer (PBS containing 26% [wt/vol] raffinose). After addition of Lysostaphin 1 mg/ml (Sigma) and 3 µl of DNase l 1mg/ml (Sigma), the preparations were incubated with shaking at 37°C for 2 h. Protoplasts were sedimented by centrifugaction at 8,000 for 30 min with slow deceleration and supernatants were collected. Protein concentration was determined with the Bio-Rad protein assay (Bio-Rad). Samples were adjusted to 5-10 µg/µl of total protein and one volume of Laemmli buffer was added. Denaturation, separation in SDS-polyacrylamide gels, staining with Coomasie brilliant blue, protein transfer and membrane blocking were carried out as commented above in the section "Western Blot analysis". For detecting the BapB-SNAP-ClfA protein, membranes were incubated with an anti-Bap rabbit poly-clonal antibody described previously (Cucarella *et al.,* 2001) diluted 1:2,500 with TBS and immunoabsorbed with 5% skimmed milk. Alkaline phosphatase-conjugated goat anti-rabbit IgG (Sigma) diluted 1:10.000 in TBS-5% skimmed milk was used. The BapB-SNAP-ClfA protein was detected with the Amersham ECL Prime kit following the manufacturer's recommendations.

### - BapB-SNAP-ClfA imaging

For imaging, 10 µl of bacteria cell suspension grown in flasks until OD₆₀₀ₙₘ reached 0.8 were plated on coverslips. After fixation with 4% paraformamide, SNAP-surface Alexa-488 (1 mM) (New England Biolabs) was added and the cells were incubated for 10-20 min. The cells were washed three times to remove unreacted substrate. After this labeling, the cells were observed by fluorescent microscopy (Zeiss).

### - Inhibition of S. aureus ΔXV growth by repressing the expression of WalKR TCSs

An overnight culture of the mutant was inoculated with or without IPTG at an optical density at 600 nm (OD600) equal to 0.005 and incubated at 37°C. After six generations without IPTG, growth was arrested, indicating that the system is essential at physiological temperatures. The number of CFU was monitored in the same assay by plating the cultures on TSB medium with or without IPTG. Although no cell lysis was observed in the absence of IPTG, the number of bacteria able to form colonies was drastically decreased, leading to 95% cell death 3 h after cessation of WalKR expression. These results indicate that WalKR expression is necessary for *S. aureus* under replicating growth conditions.

### - Statistical analysis.

The statistical analysis was performed with the GraphPad Prism program. Data corresponding to adhesion, invasion and kidney colonization assay were compared using the Mann-Whitney U tests. However, data corresponding to dessication resistance were compared usin Unpaired Student's t-test. Finally data corresponding to the survival assay were compared using Log-rank (Mantel-Cox) test. All the tests were two-tailed and the significance level was 5%.

### - Example 1: Construction and characterization of the S. aureus ΔXV mutants

### 1.1. Deletion of the fifteen non-essential TCS in a single cell

The present inventors constructed *S. aureus* strains deficient in the fifteen non-essential TCS to uncover the range of phenotypes affected by TCS signaling in *S. aureus* biology. The deletions were done in two strain backgrounds, the S. *aureus* MW2 (Complete genome accessible with NCBI Access Number NC_003923, version NC_003923.1 GI:21281729) and NCTC 8325 (Complete genome accessible with NCBI Access Number NC_007795, version NC_007795.1 GI:88193823) strains, both of them lacking the *kdpED-like* system present in some MRSA strains, applying subsequently the process explained in the previous section "Allelic exchange of chromosomal genes". The sequence of generation of intermediate mutants, as can be deduced from Table 3, was:
*MWA ΔXV*
   MW2 ΔI: MW2 *Δyhc*
   MW2 ΔII (MW2 ΔI *Δtcs3):* MW2 *Δyhc Δtcs3*
   MW2 ΔIII (MW2 ΔII *Δlyt):* MW2 *Δyhc Δtcs3 Δlyt*
   MW2 ΔIV (MW2 ΔIII *Δgra):* MW2 *Δyhc Δtcs3 Δlyt Δgra*
   MW2 ΔV (MW2 ΔIV *Δsae*): MW2 *Δyhc Δtcs3 Δlyt Δgra Δsae*
   MW2 ΔVI (MW2 ΔV *Δtcs7):* MW2 *Δyhc Δtcs3 Δlyt Δgra Δsae Δtcs7*
   MW2 ΔVII (MW2 ΔVI *Δhss):* MW2 *Δyhc Δtcs3 Δlyt Δgra Δsae Δtcs7 Δhss*
   MW2 ΔVIII: (MW2 ΔVII *Δnre*)*:* MW2 *Δyhc Δtcs3 Δlyt Δgra Δsae Δtcs7 Δhss Δnre*
   MW2 ΔIX: (MW2 ΔVIII *Δbra*)*:* MW2 *Δyhc Δtcs3 Δlyt Δgra Δsae Δtcs7 Δhss Δnre Δbra*
   MW2 ΔX (MW2 ΔIX *Δkdp):* MW2 *Δyhc Δtcs3 Δlyt Δgra Δsae Δtcs7 Δhss Δnre Δbra Δkdp*
   MW2 ΔXI (MW2 ΔX *Δvra):* MW2 *Δyhc Δtcs3 Δlyt Δgra Δsae Δtcs7 Δhss Δnre Δbra Δkdp Δvra*
   MW2 ΔXII (MW2 ΔXI Δ*pho*)*:* MW2 *Δyhc Δtcs3 Δlyt Δgra Δsae Δtcs7 Δhss Δnre Δbra Δkdp Δvra Δpho*
   MW2 ΔXIII (MW2 ΔXII *Δarl*)*:* MW2 *Δyhc Δtcs3 Δlyt Δgra Δsae Δtcs7 Δhss Δnre Δbra Δkdp* Δ*vra* Δ*pho* Δ*arl*
   MW2 ΔXIV (MW2 ΔXIII *Δagr*): MW2 *Δyhc Δtcs3 Δlyt Δgra Δsae Δtcs7 Δhss Δnre Δbra Δkdp* Δ*vra* Δ*pho* Δ*arl Δagr*
   MW2 ΔXV (MW2 ΔXIV *Δsrr*): MW2 *Δyhc Δtcs3 Δlyt Δgra Δsae Δtcs7 Δhss Δnre Δbra Δkdp* Δ*vra* Δ*pho* Δ*arl Δagr Δsrr*
*8325 ΔXV*
   8325 ΔI: 8325 Δ*yhc*
   8325 ΔII (8325 ΔI *Δlyt*): 8325 Δ*yhc Δlyt*
   8325 ΔIII (8325 ΔII *Δgra*): 8325 *Δyhc Δlyt Δgra*
   8325 ΔIV (8325 ΔIII *Δsae*): 8325 Δ*yhc Δlyt Δgra Δsae*
   8325 ΔV (8325 ΔIV *Δtcs7*): 8325 Δ*yhc Δlyt Δgra Δsae Δtcs7*
   8325 ΔVI (8325 ΔV *Δtcs3*): 8325 Δ*yhc Δlyt Δgra Δsae Δtcs7 Δtcs3*
   8325 ΔVII (8325 ΔVI Δ*arl*): 8325 Δ*yhc Δlyt Δgra Δsae Δtcs7 Δtcs3* Δ*arl*
   8325 ΔVIII (8325 ΔVII Δ*srr*): 8325 Δ*yhc Δlyt Δgra Δsae Δtcs7 Δtcs3* Δ*arl* Δ*srr*
   8325 ΔIX (8325 ΔVIII Δ*pho*)*:* 8325 Δ*yhc Δlyt Δgra Δsae Δtcs7 Δtcs3* Δ*arl* Δ*srr Δpho*
   8325 ΔX (8325 ΔIX *Δhss*): 8325 Δ*yhc Δlyt Δgra Δsae Δtcs7 Δtcs3* Δ*arl* Δ*srr* Δ*pho Δhss*
   8325 ΔXI (8325 ΔX Δ*nre*): 8325 Δ*yhc Δlyt Δgra Δsae Δtcs7 Δtcs3* Δ*arl* Δ*srr* Δ*pho Δhss* Δ*nre*
   8325 ΔXII (8325 ΔXI Δ*bra*): 8325 Δ*yhc Δlyt Δgra Δsae Δtcs7 Δtcs3* Δ*arl* Δ*srr* Δ*pho Δhss* Δ*nre* Δ*bra*
   8325 ΔXIII (8325 ΔXII *Δvra*): 8325 Δ*yhc Δlyt Δgra Δsae Δtcs7 Δtcs3* Δ*arl* Δ*srr* Δ*pho Δhss* Δ*nre* Δ*bra Δvra*
   8325 ΔXIV (8325 ΔXIII Δ*kdp*): 8325 Δ*yhc Δlyt Δgra Δsae Δtcs7 Δtcs3* Δ*arl* Δ*srr Δpho Δhss* Δ*nre* Δ*bra Δvra* Δ*kdp*
   8325 ΔXV (8325 ΔXIV *Δagr*): 8325 Δ*yhc Δlyt Δgra Δsae Δtcs7 Δtcs3* Δ*arl* Δ*srr* Δ*pho Δhss* Δ*nre* Δ*bra Δvra* Δ*kdp Δagr*

As has been commented previously, the TCSs herein denominated tcs3 and tcs7 are those whose components are the HK and RR identified in the above mentioned *S*. *aureus* MW2 NC_003923) and 8325 (NC_007795) annotated genomes as:
- *Tcs3* HK:
   NC 003923.1 GI:21281729 (MW2)
      Gene: complement (233788..235344); /locus_tag="MW_RS01035"; /old_locus_tag="MW0199"
      CDS:_complement(233788..235344); /locus_tag="MW_RS01035"; /old_locus_tag="MW0199"; /inference="EXISTENCE: similar to AA; sequence:RefSeq:WP_000127992.1"; /note="Derived by automated computational analysis using gene prediction method: Protein Homology."; codon_start=1; /transl_table=11; /product="histidine kinase"; /protein_id="WP_000127997.1"; /db_xref="GI:446050142"
   NC 007795.1 GI:21281729 (8325)
      Gene: complement (202799..204355); /locus_tag="SAOUHSC_00185"; /db_xref="GeneID:3919499"
      CDS: complement(202799..204355); /locus_tag="SAOUHSC_00185"; /note="conserved hypothetical protein"; /codon_start=1; /transl_table=11; /product="hypothetical protein"; /protein_id="YP_498782.1"; /db_xref="GI:88193995"; /db_xref="GeneID:3919499"
- *Tcs3* RR:
   NC 003923.1 GI:21281729 (MW2)
      Gene: complement (233037..233795); /locus_tag="MW_RS01030"; /old_locus_tag="MW0198"
      CDS complement (233037..233795); /locus_tag="MW_RS01030"; /old_locus_tag="MW0198"; /inference="EXISTENCE: similar to AA; sequence:RefSeq:WP_000477527.1"; /note="Derived by automated computational analysis using; gene prediction method: Protein Homology."; /codon_start=1; /transl_table=11; /product="AraC family transcriptional regulator"; /protein_id="WP_000477521.1"; /db_xref="GI:446399666"
   NC 007795.1 GI:21281729 (8325);
      Gene: complement(202048..202806); /locus_tag="SAOUHSC_00184"; /db_xref="GeneID:3919498";
      CDS: complement (202048..202806); /locus_tag="SAOUHSC_00184"; /note="Response regulator receiver domain protein"; /codon_start=1; /transl_table=11; /product="response regulator receiver domain-containing; protein"; /protein_id="YP_498781.1"; /db_xref="GI:88193994"; /db_xref="GeneID:3919498";
- *Tcs7* HK:
   NC 003923.1 GI:21281729 (MW2)
      Gene: 1321404..1322495; /locus_tag="MW_RS06485"; /old_locus_tag="MW1208"
      CDS: 1321404..1322495; /locus_tag="MW_RS06485"; /old_locus_tag="MW1208" ;/inference="EXISTENCE: similar to AA; sequence:RefSeq:WP_001556634.1"; /note="Derived by automated computational analysis using;gene prediction method: Protein Homology."; /codon_start=1;/transl_table=11; /product="histidine kinase"; /protein_id="WP_000670300.1"; /db_xref="GI:446592954"
   NC 007795.1 GI:21281729 (8325)
      Gene: 1257226..1258317; /locus_tag="SAOUHSC_01313"; /db_xref="GeneID:3920139"
      CDS 1257226..1258317; /locus_tag="SAOUHSC_01313"; /note="Histidine kinase-, DNA gyrase B-, and HSP90-like; ATPase domain protein"; /codon_start=1; /transl_table=11; /product="histidine kinase"; /protein_id="YP_499843.1"; /db_xref="GI:88195043"; /db_xref="GeneID:3920139"
- *Tcs7* RR:
   NC 003923.1 GI:21281729 (MW2)
      Gene: 1320669..1321400; /locus_tag="MW_RS06480"; /old_locus_tag="MW1207"
      CDS 1320669..1321400; /locus_tag="MW_RS06480"; /old_locus_tag="MW1207"; /inference="EXISTENCE: similar to AA; sequence:RefSeq:WP_000603962.1"; /note="Derived by automated computational analysis using; gene prediction method: Protein Homology."; /codon_start=1; /transl_table=11; /product="multidrug ABC transporter permease"; /protein_id="WP_000603969.1"; /db_xref="GI:446526623";
   NC 007795.1 GI:21281729 (8325)
      Gene: 1258314..1258916; /locus_tag="SAOUHSC_01314"; /db_xref="GeneID:3920140"
      CDS: 1258314..1258916; /locus_tag="SAOUHSC_01314"; /note="conserved hypothetical protein"; /codon_start=1; /transl_table=11; /product="hypothetical protein"; /protein_id="YP_499844.1"; /db_xref="GI:88195044"; /db_xref="GeneID:3920140"

Due to the particular combination of oligonucleotide primers used in this particular case for obtaining the above mentioned mutant strains (see Table 4), in some particular operons, not only the genes corresponding to the HK and RR component have been deleted, but also certain genes also present in the operon. This is the case of the genes: graX in the case of the operon corresponding to the TCS graRS, saeP and saeQ in the case of the operon corresponding to the TCS saeRS, agrB and agrD in the case of the operon agr, and nreA in the case of the operon of the TCS nreBC. For such reason, in the sequence of mutant generation until obtaining MW2 ΔXV and 8325 ΔXV strains, the names of the operons are indicated without indicating thereafter the initials of the HK and RR components.

The genomes of the resulting strains, *S*. *aureus* MW2 ΔXV and *S*. *aureus* 8325 ΔXV, were sequenced as described in the corresponding above section, to exclude the possibility that spontaneous mutations might have emerged during the successive deletion process. The absence of common mutations between both strains confirmed that the absence of the fifteen TCS does not select for a particular genetic change that influenced the behavior of the ΔXV strains (Table 6 and 7).

**Table 6. Spontaneous changes produced during the mutagenesis on the MW2 genome**

| **Position** | **Mutation** | **Annotation** | **Gene** | **Description** |
|---|---|---|---|---|
| 239,826 | G→A | A220T (GCA→ACA) | *pflA* → | formate acetyltransferase activating enzyme |
| 379,604 | G→T | G249V (GGA→GTA) | *MW0330* → | hypothetical protein |
| 746,147 | G→T | E123* (GAA→TAA) | *nagA* → | N-acetylglucosamine-6-phosphate deacetylase |
| 1,021,368 | G→T | D263Y (GAT→TAT) | *menB* → | naphthoate synthase |
| 1,105,909 | Ω43545 bp | Phage insertion | *rpmF*→/←*isdB* | 50S ribosomal protein L3/hypothetical protein |
| 1,267,878 | G→T | R475L (CGT→CTT) | *pnpA* → | polynucleotide phosphorylase/polyadenylase |
| 1,283,489 | G→C | G502A (GGT→GCT) | *MW1169* → | phosphodiesterase |
| 1,691,188 | G→T | L10I (CTA→ATA) | *MW1565 ←* | hypothetical protein |
| 2,639,004 | GT→TG | Y441S (TAC→TCA) | *rocA* ← | 1 -pyrroline-5-carboxylate dehydrogenase |
| 2,639,012 | T→A | T439S (ACA→TCA) | *rocA* ← | 1-pyrroline-5-carboxylate dehydrogenase |

| | | | | |
|---|---|---|---|---|
| * Position in the genome of the MW2 strain (NC_003923.1) | | | | |

**Table 7: Spontaneous changes produced during the mutagenesis on the 8325 genome**

| **Position*** | **Mutation** | **Annotation** | **Gene** | **Description** |
|---|---|---|---|---|
| 781,629 | C→T | Y258Y (TAC→TAT) | *eno* → | phosphopyruvate |
| 787,913 | C→T | intergenic (+907/+415) | *smpB*→/← *SA00806* | SsrA-binding protein/ hypothetical protein |
| 1,101,720 | A→G | intergenic (+149/-237) | *SA01150*→/→*SA01152* | cell division protein FtsZ/hypothetical protein |
| 1,560,503 | C→T | A190T (GCT→ACT) | *SA01646←* | glucokinase |
| 1,703,050 | T→C | intergenic (-589/-23) | *SA01802←*/→*SA01803* | hypothetical protein/ hypothetical protein |

| | | | | |
|---|---|---|---|---|
| * Position in the genome of the 8325 strain (NC_007795.1) | | | | |

### 1.2. Phenotypic characterization of S. aureus ΔXV strains

It was then determined how the removal of the complete sensorial system affects the bacterial physiology, performing several comparative tests as described above.

Firstly, a comparative growth kinetics analysis was performed as described above in the corresponding methodological section. As can be seen in Fig. 3, S. *aureus* ΔXV strains exhibited growth rates indistinguishable from the wild type under aerobic conditions at 37°C and 44°C, while it showed a growth defect at 28°C.

With regard to dessication tolerance, *S*. *aureus* ΔXV showed a decreased capacity for long-term survival during desiccation in the absence of nutrients (see Fig. 3E).

In standard biochemical and fermentation tests carried out with API Staph galleries and quantification of nitrite production, both ΔXV mutant strains only exhibited a deficiency in the capacity to reduce nitrate to nitrite compared to wild type strain (see Fig. 3D).

The assays of susceptibility of ΔXV mutants to cell lysis induced by a nonionic detergent, Triton X-100, which reflects deficiencies in the regulation of autolytic activity (de Jonge *et al.,* 1991), revealed that ΔXV mutants exhibited a much higher autolysis rate compared to the wild-type strain in Triton X-100 gradient plates (see Fig. 3F).

Next, because *S*. *aureus* protein A is a representative cell wall-associated exoprotein and a major determinant of virulence in *S*. *aureus,* the present inventors also compared the protein levels of protein A between the ΔXV mutant and the wild type. The results confirmed that the expression of protein A in the ΔXV mutant was lower than that in the wild type (see Fig. 3G).

Finally, antimicrobial susceptibility test against the panel of antibiotics routinely used in clinics for treatment of *S*. *aureus* infections revealed that MW2 ΔXV mutant is more susceptible to oxacillin, levofloxacin, linezolid, nitrofurantoin and cefoxitin while the sensitivity for the rest of antibiotics tested remained similar. The 8325 ΔXV mutant show higher susceptibility to bencilpenicillin, ampicillin, vancomycin and fosfomycin while the sensitivity for the rest of antibiotics tested remained similar. The results are summarized in Table 8 below.

**Table 8. Antimicrobial susceptibility test against the panel of antibiotics routinely used in clinics for treatment of S. aureus infection**

| **ANTIBIOTICS** | **MW2** | **MW2 ΔXV** | **8325** | **8325 ΔXV** |
|---|---|---|---|---|
| β-lactam | + | + | - | - |
| Cefoxitin detection | + | + | - | - |
| Bencilpenicillin | R | R | R (0.25) | S (0.12) |
| Ampicillin | R | R | R | S |
| Cloxacillin | R | R | S | S |
| Oxacillin | R (≥4)^{a} | R (2)^{a} | S | S |
| Cefalotin | R | R | S | S |
| Cefuroxime | R | R | S | S |
| Gentamicin | S | S | S | S |
| Tobramycin | S | S | S | S |
| Ciprofloxacin | S | S | S | S |
| Levofloxacin | S (≤0.25)^{a} | S (≤0.12)^{a} | S | S |
| Inducible resistance to Clindamycin | - | - | - | - |
| Azithromycin | S | S | S | S |
| Clarithromycin | S | S | S | S |
| Erithromycin | S | S | S | S |
| Clindamycin | S | S | S | S |
| Quinuspristin/Dalfopristin | S | S | S | S |
| Linezolid | S (2)^{a} | S (1)^{a} | S | S |
| Teicoplanin | S | S | S | S |
| Vancomycin | S | S | S (1)^{a} | S (≤0.5)^{a} |
| Tigecycline | S | S | S | S |
| Fosfomycin | S | S | S (16)^{a} | S (≤8)^{a} |
| Nitrofurantoin | S (32)^{a} | S (≤16)^{a} | S | S |
| Fusidic acid | S | S | S | S |
| Mupirocin | S | S | S | S |
| Rifampicin | S | S | S | S |
| Trimethoprim/Sulfamethoxazole | S | S | S | S |
| Cefoxitin | R | S | S | S |
| Chloramphenicol | S | S | S | S |
| MRSA | + | + | - | - |

| | | | | |
|---|---|---|---|---|
| ^{a}MIC, Minimal inhibitory concentration; R: resistant; S: susceptible | | | | |

### 1.3. S. aureus ΔXV mutant is attenuated in vivo

An essential step in the establishment of *S*. *aureus* infection is the adhesion of bacteria to surface components of epithelial cells. Therefore, the present inventors analyzed the consequences of the absence of the TCS signaling on the adherence capacity of *S*. *aureus* to two different cell lines, a human lung epithelial (A549) and a human hepatocyte (Hep-3B) cell lines. Remarkably, as can be seen in Fig. 4A, ΔXV mutant strains adhere to both cell lines as efficiently as the wild type strain (P > 0.05). In contrast, ΔXV mutant strain invaded both epithelial cell lines less efficiently than the wild type strain (Fig. 4B).

Because *S*. *aureus* is a leading cause of bacteraemia and sepsis, it was then investigated the contribution of TCS to invasive staphylococcal disease using a mouse renal abscess model. The results revealed that wild type *S*. *aureus* MW2 formed abscesses in kidneys with a mean bacterial load of 7.59 x 10⁷ CFU per gram of tissue. In contrast, ΔXV mutant was unable to form abscesses and displayed a >1000-fold (log 4.24) reduction in bacterial load as compared with the wild type counterpart (P < 0.01; Fig. 4C and 4D).

The mice survival upon intravenous injection of 10⁷ CFU of wild type or ΔXV strains was also analysed. All mice infected with the mutant strain survive after 50 days, while 50% of the animals inoculated with MW2 wild type strain died by day 21 post-infection (see Fig. 4E).

Taken together, these data indicated that TCS network is important for the pathogenesis of *S*. *aureus* infections, whether measured as the ability of *S*. *aureus* to form abscesses and persist in host tissues or lethal bacteremia.

### - Example 2: Sense devoid S. aureus mutant

It is well documented that WalKR is essential for *S*. *aureus* growth. To generate a mutant unable to sense environmental signals, the present inventors generated a derivative of *S*. *aureus* ΔXV strain (named *S*. *aureus* ΔXVI) in which the *walKR* operon was placed under the control of an inducible promoter, Pspac iso-propyl-β-D-thiogalactopyranoside (IPTG)-inducible promoter (Dubrac and Msadek, 2004).

In the absence of IPTG (that is, in the situation where the strain lacks the expression of the *walKR* operon from the chromosome), the *S*. *aureus* ΔXVI strain only grows when it is complemented with a plasmid encoding the *walR* gene under the control of a constitutive promoter (pEI walR), giving rise to the ΔXVI* strain.

In the absence of IPTG, ΔXVI* strain exhibited growth rates indistinguishable from the ΔXV strain a 37ºC y 28ºC (see Figs. 5A, B, C), showed similar capacity for long-term survival during desiccation in the absence of nutrients (Fig. 5E), susceptibility to triton X-100 (Fig. 5F), deficiency in the capacity to reduce nitrate to nitrite (Fig. 5D) and reduction in protein A expression than the ΔXV strain (Fig. 5G).

Taken together, these results indicate that the constitutive expression of WalR in the absence of WalK sensor was sufficient to suppress lethality and that a *S*. *aureus* strain devoid of the TCSs is viable under laboratory conditions.

### - Example 3. Single TCS-dependent phenotypes

### 3.1. Preparation and phenotypic characterization of single TCS mutants

All previous phenotypes related with TCS have been deduced from the comparison between the wild type and the corresponding isogenic mutant. This strategy allows the identification of phenotypes for which the presence of the corresponding TCS is necessary. However, identification of a TCS necessary for a phenotype does not necessarily imply that the TCS is sufficient to account for this particular phenotype in the absence of other TCS.

To identify the correlation between the phenotypes displayed by the ΔXV strain with individual TCS, the present inventors prepared a collection of mutants of the MW2 strain, each one lacking one TCS, named MW2 Δ4 to MW2 Δ17 (see Table 3). The TCS deleted in each strain can be found in Table 3. Mutant MW2 AI (MW2 Δ*yhc*)*,* whose construction was the first step for obtaining MW2 ΔXV as described in Example 1 of the present application, was also a part of such collection of single mutants.

In order to identify those phenotypes that depend exclusively on a single TCS, the present inventors compared the phenotypes previously characterized in the ΔXV strain with the collection of single mutants, the collection of sequential mutants and the collection of ΔXV derivatives each complemented with a single TCS. The inventors hypothesized that if a single TCS controls a phenotype, then, the phenotype should be characteristic of the single mutant in this TCS, the sequential mutant after deletion of uniquely this TCS and the phenotype should be restored with the ectopic expression of this TCS in the ΔXV strain.

The results revealed that growth defect under replicating conditions depends on the WalKR system, the defect on the growth at 28°C depends on the SrrAB, the capacity to reduce nitrite to nitrate depends on NreBC, the expression of protein A depends on ArlRS, and susceptibility to triton X-100 depends on VraRS.

### 3.2. Mutants with ectopic expression of a TCS previously deleted in the ΔXV strain

In order to confirm the adscription of a particular loss of function to a particular TCS in accordance with the results of the previous section, complementation of the ΔXV mutant with the expression of such TCS was sought.

For the complementation purposes, the present inventors engineered a procedure in which each HK and RR is produced as an individual module that can be very easily combined with other modules into the same plasmid, as it is explained in the methodological section of "Construction of plasmids expressing HKs and/or RRs under the control of different promoters". This strategy gave the inventors the possibility to express each RR either alone, together with the cognate HK pair or in combination with the non-cognate HKs. Depending on the selecting plasmid the expression of HK-RR pairs could be under the control of the cadmium-inducible promoter (*P_{cad}*) (pEI and pCI plasmids) or the constitutive promoter P*_{blaZ}* (pEW and pCW plasmids).

In all of the instances assayed in section 3.1., ectopic expression of a functional copy of the corresponding TCS was sufficient to fully rescue each phenotype in the ΔXV strain (Fig. 7), as well as in the corresponding single mutant. Taken together, these results indicate that phenotypes of ΔXV strain depend on individual TCS, and ectopic restoration of the corresponding TCS is sufficient to restore phenotypes associated to the ΔXV strain.

### - Example 4. System-based analysis of cross talk in vivo

### 4.1. Testing of general cross-talk

Next, the inventors exploited the ΔXV strain as well as the collections of single mutants to carry on a systematic analysis of the connectivity between HKs and RRs *in vivo.* Their reasoning was that if ectopic expression of a native RR is capable to activate the phenotype in the corresponding single mutant but it is unable to activate a specific phenotype in the ΔXV strain, it implies the existence of cross talk from one or various non-cognate sensor kinases present in the single mutant but absent in the ΔXV strain.

Based on the previously observed correlation between phenotypes and single TCS, MW2 mutants in *srrAB, nreBC, arlRS, vraRS* and the ΔXV mutant were complemented with the corresponding SrrB, NreC, ArlR and VraR response regulators. The results are shown in Fig. 8.

Interestingly, ectopic expression of the native *arlR* gene was able to restore protein A expression in the single *S*. *aureus arlRS* mutant whereas complementation of ΔXV mutant with the same plasmid did not. Contrary to this, complementation with SrrB, NreC and VraR response regulators could not activate their corresponding phenotype in the single mutant, indicating that cross-talked between these RR and non-cognate HK does not occur between non-cognate HK and these RR at least under laboratory growth conditions.

### 4.2. Cross-phosphorylation

The observation that ArlR restored protein A production in the absence of ArlS led the inventors to hypothesize that cross-talk from another HK to ArlR occurs *in vivo.* They first confirmed that phosphorylation of ArlR by ArlS was necessary to induce protein A expression (Fig. 9A). For that, the aspartic residue at position 52 in ArlR was replaced with a nonphosphorylatable alanine residue. Complementation of *arlRS* mutant with ArlRD52A allele could not activate protein A expression, suggesting that phospho-transfer to ArlR was necessary to induce protein A expression.

The phosphatase activity of the cognate HK is one of the mechanisms to prevent the non-specific phosphorylation of the cognate RR and it also provides a mechanism to reset the signal transduction to the baseline state. Therefore, cross-talk from non-cognate HK in the absence of the phosphatase catalytic activity of the cognate histidine kinase has been previously reported (Stock et al., 2000; Amemura et al., 1990). To test whether cross-talk of ArlR by a non-cognate HK could occur in the presence of the phosphatase activity of ArlS, ArlR was expressed together with the ArlSH242A allele, which contains an histidine-to-alanine substitution at position 242, and therefore it retains the phosphatase activity but it cannot phosphorylate ArlR. The resulting strain showed a level of expression of protein A similar to that of the strain complemented with ArlR alone, indicating that the phosphatase activity of ArlS cannot supress cross-talk from other histidine kinases to ArlR (Fig. 9A).

The present inventors next developed a system-level approach to identify cross-phosphorylation between HKs and ArlR in vivo. A set of fifteen plasmids each containing the *arlR* gene in combination with one HK was used to complement ΔXV strain. This strategy allows to test the ability of non-cognate HKs to activate ArlR-dependent protein A in the absence of other sources of phosphotransfer to ArlR (Fig. 9B). In addition to ArlS itself, GraS is able to restore protein A expression in ΔXV strain. This restoration was dependent on the phosphorylation of ArlR because substitution of ArlR wild type by the nonphosphorylatable ArlRD52A allele suppresses the cross-talk. It is also notable that cross-talk with ArlR occurs in the single *arlRS* mutant, implying that the GraS cross-phosphorylates ArlR even in the presence of its cognate GraR RR. Together, these results indicate that cross-talk between otherwise distinct TCSs (ArlR and GraS) through phosphotransfer occurs *in vivo,* though it seems to be limited to few specific TCS.

Since GraS is activated in the presence of colistin in the media, the present inventors hypothesized that cross-talk from GraS to ArlR might be induced in the presence of colistin (Fig. 9E). As expected, when GraS was stimulated with 50 µg of colistin, activation of the expression of protein A was observed in the wild type and single *arlRS* mutant but not in *arlRS graSR* double mutant. Furthermore, activation of protein A expression was absent when the ArlR phosphate accepting aspartate was mutated.

Together these data indicate that natural activation of GraS with cationic peptides can cross-phosphorylate ArlR to activate protein A expression *in vivo.*

### - Example 5. Use of the ΔXV strain for expressing heterologous proteins

Once verified that the *S*. *aureus* ΔXV strains are robust and stable and easily ameneable to genetic manipulations, their applicability for synthetic biology applications and, particularly, for the expression of heterologous proteins was also confirmed.

To that aim, a vector for the expression of a quimeric protein Bap-Snap-ClfA was constructed from plasmid pCN51 as explained above. The final pEI-BapB-SNAP plasmid contains the Bap-B domain (N-terminal region of the Bap protein of *S*. *aureus*) fused to a SNAP-tag followed by the C-terminal R domain of the *clumping factor* A gene, expressed under the activity of a cadmium inducible promoter (see Fig. 10A).

*Clumpling factor A* is a surface-protein, which mediates binding of *S*. *aureus* to human fibrinogen. BapB, as previously explained, is a cell wall surface protein that seems to be involved in biofilm formation. The SNAP peptide tag allows the fluorescent labelling of the quimeric protein by means of a fluorophore which recognizes and associates with the SNAP peptide.

Both the MW2 wild type and MW2 ΔXV strain were transformed with the pEI-BapB-SNAP plasmid and the resulting mutant was cultured under conditions where the protein could be expressed. The assays of protein preparation and Western blotting showed that the quimeric protein was expressed in both strains (see Fig. 10D).

The expression of the protein on the bacterial surface (see schematic representation on Fig. 10B) was verified by labelling with SNAP-surface Alexa-488 and fluorescent microscopy, being possible to detect fluorescent signal around the bacteria in both strains containing the plasmid, in the one derived from MW2 WT and in the MW2 ΔXV strain transformed with the plasmid (see Fig. 10C).

Finally, the aggregation experiments showed that the expression of the Bap-Snap-ClfA protein completely changes the aggregation phenotype of both the MW2 WT and the ΔXV strains when such protein is expressed in the strains (see Fig. 10E), demonstrating the funcionality of the expressed protein.

The assays show that the ΔXV strain is not only capable of producing heterologous proteins but also of including them in the membrane, when the heterologous protein contains appropriate sequence fragments for it. The expressed proteins are expected to be functional according to the assays.

### - Example 6. Use of the mutant strains for identifying candidates to S. aureus antimicrobial drugs through the identification of compounds capable of blocking individual TCSs

In order to verify that inhibition assays with the addition or remotion of certain compounds can be used to identify compounds capable to act on individual TCSs and to block *S*. *aureus* growth, thus being candidate compounds for being used as antimicrobial drugs, comparative assays of the growth of the parental MW2 and its isogenic mutant ΔXV were carried, after having placed, in both strains, the expression of the walKR operon was placed under the control of an inducible promoter, Pspac iso-propyl-β-D-thiogalactopyranoside (IPTG)-inducible promoter, thus generating the strains MW2 P*spac::walKR* and ΔXV P*spac::walKR* (see Example 2).

An overnight culture of each mutant (MW2 P*spac::walKR* and ΔXV P*spac::walKR*) was inoculated with or without IPTG at an optical density at 600 nm equal to 0.005 and incubated at 37ºC. After six generations without IPTG, growth was arrested, indicating that the system is essential at physiological temperatures. The number of CFU was monitored in the same assay by plating the cultures on TSB medium with or without IPTG (see Fig. 11). Although no cell lysis was observed in the absence of IPTG, the number of bacteria able to form colonies was drastically reduced leading to 95% cell death 3 hours after cessation of growth, suggesting that in this conditions the absence of *walKR* expression is lethal for *S*. *aureus.* It can be seen that many bacteria lose viability after having been incubated in the absence of IPTG and they are not able to grow again even after a subsequent incubation in the presence of IPTG.

The conditions of growth without IPTG can be assimilated to those conditions where a strain is growing in presence of an inhibitor, which condition of inhibitor could be assayed following a procedure similar to the one used in the present assay. The use of the ΔXV mutant would like to identify inhibitors capable to block the *walKR* system, which is indispensable for *S*. *aureus* life, among those compounds capable to block the growth of a ΔXV strain with an intact *walKR* system. The use of other individual mutants or the reintroduction of each individual TCS in the ΔXV mutants would allow the identification of compounds capable to block other individual TCS among those compounds provoking the cessation of growth when they are present, which growth can be observed when the compound is absent of the culture media of the same mutant strain.

This assay also demonstrate that using inducible regulatory elements (such as riboswitches, thermosensors, or transcriptional regulators) to control the expression of WalKR would allow the creation of a biosafe *S*. *aureus* strain able to grow only in the presence of the corresponding inductor.

### DEPOSIT OF MICROORGANISMS

The mutant strains MW2 ΔXV, 8325 ΔXV, MW2 ΔXVI and 8325 ΔXVI whose generation is disclosed in the present application were deposited in the Colección Española de Cultivos Tipos (Spanish Type Culture Collection), Parc Cientific Universitat de València, Catedrático Agustín Escardino, 9, 46980 Paterna, Valencia, Spain, under the Budapest Treaty. The deposit date and assigned number are as follows:

| **Mutant strain** | **Abbreviated name** | **Deposit number** | **Date of deposit** |
|---|---|---|---|
| *S*. *aureus* MW2 ΔXV | MW2 ΔXV | CECT 8756 | 30^{th} October 2014 |
| *S*. *aureus* NCTC 8325 ΔXV | 8325 ΔXV | CECT 8755 | 30^{th} October 2014 |
| *S*. *aureus* MW2 DeltaXVI | MW2 ΔXVI | CECT 8915 | 3^{rd} July 2015 |
| *S*. *aureus* NCTC 8325 DeltaXVI | 8325 ΔXVI | CECT 8916 | 3^{rd} July 2015 |

The four strains were deposited indicating as contact person one of the inventors, Dr. Lasa, as employee of one of the applicants, acting for and on behalf of the two applicants, Universidad Pública de Navarra and Consejo Superior de Investigaciones Científicas.

### References

Alm, E., Huang, K., and Arkin, A. (2006) The evolution of two-component systems in bacteria reveals different strategies for niche adaptation. PLoS Comput Biol 2: e143-e143.
Arnaud, M., Chastanet, A., and Débarbouille, M. (2004) New Vector for Efficient Allelic Replacement in Naturally Nontransformable, Low-GC-Content, Gram-Positive Bacteria. Appl Environ Microbiol 70: 6887-6891.
Baba, T., Takeuchi, F., Kuroda, M., Yuzawa, H., Aoki, K.I., Oguchi, A., et al. (2002) Genome and virulence determinants of high virulence community-acquired MRSA. The Lancet 359: 1819-1827.
Bateman, B., Donegan, N., Jarry, T., Palma, M., and Cheung, A. (2001) Evaluation of a tetracycline-inducible promoter in Staphylococcus aureus in vitro and in vivo and its application in demonstrating the role of sigB in microcolony formation. Infect Immun 69: 7851-7857.
Boyle-Vavra, S., Yin, S., Jo, D.S., Montgomery, C.P., and Daum, R.S. (2013) VraT/YvqF is required for methicillin resistance and activation of the VraSR regulon in Staphylococcus aureus. Antimicrobial Agents and Chemotherapy 57: 83-95.
Brunskill, E.W., and Bayles, K.W. (1996) Identification and molecular characterization of a putative regulatory locus that affects autolysis in Staphylococcus aureus. J Bacteriol 178: 611-618.
Capra, E.J., Perchuk, B.S., Skerker, J.M., and Laub, M.T. (2012) Adaptive Mutations that Prevent Crosstalk Enable the Expansion of Paralogous Signaling Protein Families. Cell 150: 222-232.
Charpentier, E., Anton, A.I., Barry, P., Alfonso, B., Fang, Y., and Novick, R.P. (2004) Novel Cassette-Based Shuttle Vector System for Gram-Positive Bacteria. Appl Environ Microbiol 70: 6076-6085.
Corrigan, R.M., Campeotto, I., Jeganathan, T., Roelofs, K.G., Lee, V.T., and Gründling, A. (2013) Systematic identification of conserved bacterial c-di-AMP receptor proteins. Proc Natl Acad Sci U S A 110: 9084-9089.
Cucarella, C., Solano, C., Valle, J., Amorena, B., Lasa, I., and Penadés, J.R. (2001) Bap, a Staphylococcus aureus surface protein involved in biofilm formation. J Bacteriol 183: 2888-2896.
David, M.Z., and Daum, R.S. (2010) Community-associated methicillin-resistant Staphylococcus aureus: Epidemiology and clinical consequences of an emerging epidemic. Clin Microbiol Rev 23: 616-687.
de Jonge, B.L., de Lencastre, H., and Tomasz, A. (1991) Suppression of autolysis and cell wall turnover in heterogeneous Tn551 mutants of a methicillin-resistant Staphylococcus aureus strain. J Bacteriol 173: 1105-1110.
Deatherage, D.E., and Barrick, J.E. (2014) Identification of mutations in laboratory-evolved microbes from next-generation sequencing data using breseq. Methods Mol Biol 1151: 165-188.
Delaune, A., Dubrac, S., Blanchet, C., Poupel, O., Mäder, U., Hiron, A., et al. (2012) The WalKR system controls major staphylococcal virulence genes and is involved in triggering the host inflammatory response. Infect Immun 80: 3438-3453.
Dubrac, S., and Msadek, T. (2004) Identification of genes controlled by the essential YycG/YycF two-component system of Staphylococcus aureus. J Bacteriol 186: 1175-1181.
Dubrac, S., Boneca, I.G., Poupel, O., and Msadek, T. (2007) New insights into the WalK/WalR (YycG/YycF) essential signal transduction pathway reveal a major role in controlling cell wall metabolism and biofilm formation in Staphylococcus aureus. J Bacteriol 189: 8257-8269.
Dunman, P.M., Murphy, E., Haney, S., Palacios, D., Tucker-Kellogg, G., Wu, S., et al. (2001) Transcription profiling-based identification of Staphylococcus aureus genes regulated by the agr and/or sarA loci. J Bacteriol 183: 7341-7353.
Duthie, E.S., and Lorenz, L.L. (1952) Staphylococcal coagulase; mode of action and antigenicity. J Gen Microbiol 6: 95-107.
Fournier, B., and Hooper, D.C. (2000) A new two-component regulatory system involved in adhesion, autolysis, and extracellular proteolytic activity of Staphylococcus aureus. J Bacteriol 182: 3955-3964.
Fournier, B., Klier, A., and Rapoport, G. (2001) The two-component system ArlS-ArlR is a regulator of virulence gene expression in Staphylococcus aureus. Mol Microbiol 41: 247-261.
Fridman, M., Williams, G.D., Muzamal, U., Hunter, H., Siu, K.W.M., and Golemi-Kotra, D. (2013) Two unique phosphorylation-driven signaling pathways crosstalk in Staphylococcus aureus to modulate the cell-wall charge: Stk1/Stp1 meets GraSR. Biochemistry 52: 7975-7986.
Galperin, M.Y. (2010) Diversity of structure and function of response regulator output domains. Curr Opin in Microbiol 13: 150-159.
Giraudo, A.T., Raspanti, C.G., Calzolari, A., and Nagel, R. (1994) Characterization of a Tn551-mutant of Staphylococcus aureus defective in the production of several exoproteins. Can J Microbiol 40: 677-681.
Guckes, K.R., Kostakioti, M., Breland, E.J., Gu, A.P., Shaffer, C.L., Martinez, C.R., et al. (2013) Strong cross-system interactions drive the activation of the QseB response regulator in the absence of its cognate sensor. Proc Natl Acad Sci U S A 110: 16592-16597.
Hanssen, A.-M., and Ericson Sollid, J.U. (2006) SCC mecin staphylococci: genes on the move. FEMS Immunol Med Microbiol 46: 8-20.
Holland, L.M., O'Donnell, S.T., Ryjenkov, D.A., Gomelsky, L., Slater, S.R., Fey, P.D., et al. (2008) A Staphylococcal GGDEF Domain Protein Regulates Biofilm Formation Independently of Cyclic Dimeric GMP. J Bacteriol 190: 5178-5189.
Kim, L., Mogk, A., and Schumann, W. (1996) A xylose-inducible Bacillus subtilis integration vector and its application. Gene 181: 71-76.
Kinkel, T.L., Roux, C.M., Dunman, P.M., and Fang, F.C. (2013) The Staphylococcus aureus SrrAB Two-Component System Promotes Resistance to Nitrosative Stress and Hypoxia. mBio 4: e00696-13-e00696-13.
Kofoid, E.C., and Parkinson, J.S. (1988) Transmitter and Receiver Modules in Bacterial Signaling Proteins. Proc Natl Acad Sci USA 85: 4981-4985.
Kolar, S.L., Nagarajan, V., Oszmiana, A., Rivera, F.E., Miller, H.K., Davenport, J.E., et al. (2011) NsaRS is a cell-envelope-stress-sensing two-component system of Staphylococcus aureus. Ann Rev Microbiol 157: 2206-2219.
Kuroda, M., Kuroda, H., Oshima, T., Takeuchi, F., Mori, H., and Hiramatsu, K. (2003) Two-component system VraSR positively modulates the regulation of cell-wall biosynthesis pathway in Staphylococcus aureus. Mol Microbiol 49: 807-821.
Lasa, I., Toledo-Arana, A., Dobin, A., Villanueva, M., de Los Mozos, I.R., Vergara-Irigaray, M., et al. (2011) Genome-wide antisense transcription drives mRNA processing in bacteria. Proc Natl Acad Sci U S A 108: 20172-20177.
Li, H., Handsaker, B., Wysoker, A., Fennell, T., Ruan, J., Homer, N., et al. (2009) The Sequence Alignment/Map format and SAMtools. Bioinformatics 25: 2078-2079.
Li, M., Lai, Y., Villaruz, A.E., Cha, D.J., Sturdevant, D.E., and Otto, M. (2007) Gram-positive three-component antimicrobial peptide-sensing system. Proc Natl Acad Sci U S A 104: 9469-9474.
Lowy, F. (1998) Staphylococcus aureus infections. N Engl J Med 339: 520-532.
Maestro, B., Novaková, L., Hesek, D., Lee, M., Leyva, E., Mobashery, S., et al. (2011) Recognition of peptidoglycan and Î2-lactam antibiotics by the extracellular domain of the Ser/Thr protein kinase StkP from Streptococcus pneumoniae. FEBS Letters 585: 357-363.
Marmur, J. (1961) A procedure for isolation of deoxyribonucleic acid from microorganisms. J Mol Biol 3: 208-218.
Martin, P.K., Li, T., Sun, D.X., Biek, D.P., and Schmid, M.B. (1999) Role in cell permeability of an essential two-component system in Staphylococcus aureus. J Bacteriol 181: 3666-3673.
Mccallum, N., Meier, P.S., Heusser, R., and Berger-Bachi, B. (2011) Mutational analyses of open reading frames within the vraSR operon and their roles in the cell wall stress response of Staphylococcus aureus. Antimicrob Agents Chemother 55: 1391-1402.
Podgornaia, A.I., and Laub, M.T. (2013) Determinants of specificity in two-component signal transduction. Curr Opin in Microbiol 16: 156-162.
Pragman, A., Yarwood, J., Tripp, T., and Schlievert, P. (2004) Characterization of virulence factor regulation by SrrAB, a two-component system in Staphylococcus aureus. J Bacteriol 186: 2430-2438.
Queck, S.Y., Jameson-Lee, M., Villaruz, A.E., Bach, T.H.L., Khan, B.A., Sturdevant, D.E., et al. (2008) RNAIII-independent target gene control by the agrquorum-sensing system: insight into the evolution of virulence regulation in Staphylococcus aureus. Mol Cell 32: 150-158.
Rice, K.C., Mann, E.E., Endres, J.L., Weiss, E.C., Cassat, J.E., Smeltzer, M.S., and Bayles, K.W. (2007) The cidA murein hydrolase regulator contributes to DNA release and biofilm development in Staphylococcus aureus. Proc Natl Acad Sci U S A 104: 8113-8118.
Rissman, A.I., Mau, B., Biehl, B.S., Darling, A.E., Glasner, J.D., and Perna, N.T. (2009) Reordering contigs of draft genomes using the Mauve Aligner. Bioinformatics 25: 2071-2073.
Ruiz de Los Mozos, I., de Los Mozos, I.R., Vergara-Irigaray, M., Segura, V., Villanueva, M., Bitarte, N., et al. (2013) Base pairing interaction between 5"- and 3-"UTRs controls icaR mRNA translation in Staphylococcus aureus. PLoS Genetics 9: e1004001-e1004001.
Schenk, S., and Laddaga, R. (1992) Improved method for electroporation of Staphylococcus aureus. FEMS Microbiol Lett 73: 133-8.
Schlag, S., Fuchs, S., Nerz, C., Gaupp, R., Engelmann, S., Liebeke, M., et al. (2008) Characterization of the oxygen-responsive NreABC regulon of Staphylococcus aureus. J Bacteriol 190: 7847-7858.
Sharma-Kuinkel, B.K., Mann, E.E., Ahn, J.S., Kuechenmeister, L.J., Dunman, P.M., and Bayles, K.W. (2009) The Staphylococcus aureus LytSR Two-Component Regulatory System Affects Biofilm Formation. J Bacteriol 191: 4767-4775.
Siryaporn, A., and Goulian, M. (2008) Cross-talk suppression between the CpxA-CpxR and EnvZ-OmpR two-component systems in E. coli. Mol Microbiol 70: 494-506.
Skerker, J.M., Prasol, M.S., Perchuk, B.S., Biondi, E.G., and Laub, M.T. (2005) Two-Component Signal Transduction Pathways Regulating Growth and Cell Cycle Progression in a Bacterium: A System-Level Analysis. PLoS Biol 3: e334.
Stock, A.M., Robinson, V.L., and Goudreau, P.N. (2000) Two-component signal transduction. Annu Rev Biochem 69: 183-215.
Sun, F., Ji, Q., Jones, M.B., Deng, X., Liang, H., Frank, B., et al. (2012) AirSR, a [2Fe-2S] cluster-containing two-component system, mediates global oxygen sensing and redox signaling in staphylococcus aureus. J Am Chem Soc 134: 305-314.
Sun, H., Yang, Y., Xue, T., and Sun, B. (2013) Modulation of cell wall synthesis and susceptibility to vancomycin by the two-component system AirSR in Staphylococcus aureus NCTC8325. BMC Microbiol 13: 286.
Toledo-Arana, A., Dussurget, O., Nikitas, G., Sesto, N., Guet-Revillet, H., Balestrino, D., et al. (2009) The Listeria transcriptional landscape from saprophytism to virulence. Nature 459: 950-956.
Toledo-Arana, A., Merino, N., Vergara-Irigaray, M., Débarbouillé, M., Penadés, J.R., and Lasa, I. (2005) Staphylococcus aureus develops an alternative, ica-independent biofilm in the absence of the arlRS two-component system. J Bacteriol 187: 5318-5329.
Torres, V., Stauff, D., Pishchany, G., Bezbradica, J., Gordy, L., Iturregui, J., et al. (2007) A Staphylococcus aureus Regulatory System that Responds to Host Heme and Modulates Virulence. Cell Host & Microbe 1: 109-119.
Ubeda, C., Maiques, E., Tormo, M.A., Campoy, S., Lasa, I., Barbe, J., et al. (2007) SaPI operon I is required for SaPI packaging and is controlled by LexA. Mol Microbiol 65: 41-50.
Ulrich, M., Bastian, M., Cramton, S.E., Ziegler, K., Pragman, A.A., Bragonzi, A., et al. (2007) The staphylococcal respiratory response regulator SrrAB induces ica gene transcription and polysaccharide intercellular adhesin expression, protecting Staphylococcus aureus from neutrophil killing under anaerobic growth conditions. Mol Microbiol 65: 1276-1287.
Valle, J., Toledo-Arana, A., Berasain, C., Ghigo, J.-M., Amorena, B., Penadés, J.R., and Lasa, I. (2003) SarA and not sigmaB is essential for biofilm development by Staphylococcus aureus. Mol Microbiol 48: 1075-1087.
Weidenmaier, C., Goerke, C., and Wolz, C. (2012) Staphylococcus aureus determinants for nasal colonization. Trends in Microbiology 20: 243-250.
White, A.P., and Surette, M.G. (2006) Comparative Genetics of the rdar Morphotype in Salmonella. J Bacteriol 188: 8395-8406.
Xue, T., You, Y., Hong, D., Sun, H., and Sun, B. (2011) The Staphylococcus aureus KdpDE two-component system couples extracellular K + sensing and agr signaling to infection programming. Infect Immun 79: 2154-2167.
Yarwood, J., McCormick, J., and Schlievert, P. (2001) Identification of a novel two-component regulatory system that acts in global regulation of virulence factors of Staphylococcus aureus. J Bacteriol 183: 1113-1123.
Zhao, L., Xue, T., Shang, F., Sun, H., and Sun, B. (2010) Staphylococcus aureus AI-2 quorum sensing associates with the KdpDE two-component system to regulate capsular polysaccharide synthesis and virulence. Infect Immun 78: 3506-3515.

## Claims

1. A mutant strain of *Staphylococcus aureus (S. aureus),* **characterized in that** it is devoid of all TCS genes except for the one corresponding to the response regulator (RR) of the *walKR* system, *walR,* and, optionally, also except for the gene corresponding to the histidine kinase (HK) of the *walKR* system, *walK.*

2. Mutant strain of *S*. *aureus* according to claim 1, wherein the genes corresponding to the response regulator and the histidine kinase of the *walKR* system, *walR* and *walK,* are operatively linked to an inducible promoter.

3. Mutant strain of *S*. *aureus* according to claim 1 or 2, wherein *walR* is ectopically expressed, in a constitutive or in an inducible manner.

4. A mutant strain of *S*. *aureus,* wherein any combination of at least two, three, four, five, six, seven, eight, nine, ten, eleven, twelve, thirteen, fourteen or fifteen or sixteen two component signal systems (TCSs) selected from the following group, have been deleted or inactivated:
*yhcSR;*
the TCS comprised of the components HK and RR expressed either
i) by the genes *MW0199-MW0198* of *S*. *aureus* strain MW2 (tcs3), or
ii) by the genes *SAOUHSC_00185-SAOUHSC_00184* of *S*. *aureus* strain 8325 (tcs3), or
*iii)* by their corresponding orthologous genes of another *S*. *aureus* strain;
*lytSR,*
*graRS;*
*saeRS;*
the TCS comprised of the components HK and RR expressed either
i) by the genes *MW1207-MW1208* of *S*. *aureus* strain MW2 (tcs7), or
ii) by the genes *SAOUHSC_01313-SAOUHSC_01314* of *S*. *aureus* strain 8325 (tcs7), or
*iii)* by their corresponding orthologous genes of another *S*. *aureus* strain;
*hssRS;*
*nreBC;*
*braRS;*
*kdpDE;*
*vraSR;*
*phoPR;*
*arlRS;*
*agr;*
*srrAB,* and
*walKR.*

5. Mutant strain of *S*. *aureus* according to claim 4, which also lacks the *kdpED*-like TCS of some SCC-mec element.

6. Mutant strain of *S*. *aureus* according to any one of claims 1 to 5, which is a mutant of a methicillin-resistant S. *aureus* strain.

7. Mutant strain of *S*. *aureus* according to any one of claims 1 to 4, which is a mutant of the MW2 strain or a mutant of the 8325 strain.

8. Mutant strain of *S*. *aureus* according to any one of claims 1 to 7, wherein all the following two-component signal systems are deleted:
*yhcSR;*
the TCS comprised of the components HK and RR expressed either
i) by the genes MW0199-MW0198 of *S*. *aureus* strain MW2 (tcs3), or
ii) by the genes *SAOUHSC_00185-SAOUHSC_00184* of *S*. *aureus* strain 8325 (tcs3), or
*iii)* by their corresponding orthologous genes of another *S*. *aureus* strain;
*lytSR,*
*graRS;*
*saeRS;*
the TCS comprised of the components HK and RR expressed either
i) by the genes *MW1207-MW1208* of strain MW2 (tcs7), or
ii) by the genes *SAOUHSC_01313-SAOUHSC_01314* of *S*. *aureus* strain 8325 (tcs7), or
*iii)* by their corresponding orthologous genes of another *S*. *aureus* strain;
*hssRS;*
*nreBC;*
*braRS;*
*kdpDE;*
*vraSR;*
*phoPR;*
*arlRS;*
*agr;* and
*srrAB.*

9. Mutant strain according to any one of claims 1 to 8, which is devoid of all TCS except for walKR.

10. Mutant strain of *S*. *aureus* according to claim 7, which is the mutant of the MW2 strain deposited in the Spanish Type Culture Collection with the accession number CECT 8756, or the mutant of the 8325 strain deposited in the Spanish Type Culture Collection with the accession number CECT 8755.

11. Mutant strain of *S*. *aureus* according to any one of claims 1 to 9, wherein the chromosome genes corresponding to the *walKR* TCS are operatively linked to a first inducible promoter and, optionally, WalR is expressed ectopically under the control of a constitutive promoter or a second inducible promoter.

12. Mutant strain of *S*. *aureus* according to claim 11, which is a mutant of the MW2 strain or a mutant of the 8325 strain and wherein the bacterial chromosome genes encoding proteins belonging to the *walKR* system are operatively linked to the inducible Pspac promoter, and all the following two-component signal systems are deleted from the chromosome:
*yhcSR;*
the TCS comprised of the components HK and RR expressed either
i) by the genes *MW0199-MW0198* of *S. aureus* strain MW2 (tcs3), or
ii) by the genes *SAOUHSC_00185-SAOUHSC_00184* of *S. aureus* strain 8325 (tcs3), or
*iii)* by their corresponding orthologous genes of another *S. aureus* strain;
*lytSR,*
*graRS;*
*saeRS;*
the TCS comprised of the components HK and RR expressed either
i) by the genes *MW1207-MW1208* of strain MW2 (tcs7), or
ii) by the genes *SAOUHSC_01313-SAOUHSC_01314* of *S. aureus* strain 8325 (tcs7), or
*iii)* by their corresponding orthologous genes of another *S. aureus* strain;
*hssRS;*
*nreBC;*
*braRS;*
*kdpDE;*
*vraSR;*
*phoPR;*
*arlRS;*
*agr;* and
*srrAB.*

13. Mutant strain of *S. aureus* according to claim 12, which is the mutant of the MW2 strain deposited in the Spanish Type Culture Collection with the accession number CECT 8915, or the mutant of the 8325 strain deposited in the Spanish Type Culture Collection with the accession number CECT 8916.

14. Mutant strain of *S. aureus* according to claim 11 or 12, wherein a plasmid allowing the ectopic expression of WalR is present, in which plasmid WalR expression is under the control of a second inducible promoter or, preferably, under the control of a constitutive promoter.

15. Mutant strain of *S. aureus* according to any one of the claims 4 to 7, which is selected from the group of strains of:
a) the following mutants of the strain MW2:
MW2 ΔII (MW2 ΔI *Δtcs3*): MW2 *Δyhc Δtcs3*
MW2 ΔIII (MW2 ΔII *Δlyt*): MW2 *Δyhc Δtcs3 Δlyt*
MW2 ΔIV (MW2 ΔIII *Δgra*): MW2 *Δyhc Δtcs3 Δlyt Δgra*
MW2 ΔV (MW2 ΔIV Δ*sae*): MW2 *Δyhc Δtcs3 Δlyt Δgra Δsae*
MW2 ΔVI (MW2 ΔV *Δtcs7*): MW2 *Δyhc Δtcs3 Δlyt Δgra Δsae Δtcs7*
MW2 ΔVII (MW2 ΔVI *Δhss*): MW2 *Δyhc Δtcs3 Δlyt Δgra Δsae Δtcs7 Δhss*
MW2 ΔVIII: (MW2 ΔVII Δ*nre*): MW2 *Δyhc Δtcs3 Δlyt Δgra Δsae Δtcs7 Δhss Δnre*
MW2 ΔIX: (MW2 ΔVIII Δ*bra*): MW2 *Δyhc Δtcs3 Δlyt Δgra Δsae Δtcs7 Δhss Δnre Δbra*
MW2 ΔX (MW2 ΔIX *Δkdp*): MW2 *Δyhc Δtcs3 Δlyt Δgra Δsae Δtcs7 Δhss Δnre Δbra Δkdp*
MW2 ΔXI (MW2 ΔX *Δvra*): MW2 *Δyhc Δtcs3 Δlyt Δgra Δsae Δtcs7 Δhss Δnre Δbra Δkdp Δvra*
MW2 ΔXII (MW2 ΔXI *Δpho*): MW2 *Δyhc Δtcs3 Δlyt Δgra Δsae Δtcs7 Δhss Δnre Δbra Δkdp Δvra Δpho*
MW2 ΔXIII (MW2 ΔXII *Δarl*): MW2 *Δyhc Δtcs3 Δlyt Δgra Δsae Δtcs7 Δhss Δnre Δbra Δkdp Δvra Δpho Δarl*
MW2 ΔXIV (MW2 ΔXIII Δagr): MW2 *Δyhc Δtcs3 Δlyt Δgra Δsae Δtcs7 Δhss Δnre Δbra Δkdp Δvra Δpho Δarl Δagr*
MW2 ΔXV (MW2 ΔXIV *Δsrr*): MW2 *Δyhc Δtcs3 Δlyt Δgra Δsae Δtcs7 Δhss Δnre Δbra Δkdp Δvra Δpho Δarl Δagr Δsrr*
or
b) the following mutants of the strain 8325
8325 ΔII (8325 ΔI *Δlyt*): 8325 *Δyhc Δlyt*
8325 ΔIII (8325 ΔII *Δgra*): 8325 *Δyhc Δlyt Δgra*
8325 ΔIV (8325 ΔIII *Δsae*): 8325 *Δyhc Δlyt Δgra Δsae*
8325 ΔV (8325 ΔIV *Δtcs7*): 8325 *Δyhc Δlyt Δgra Δsae Δtcs7*
8325 ΔVI (8325 ΔV *Δtcs3*): 8325 *Δyhc Δlyt Δgra Δsae Δtcs7 Δtcs3*
8325 ΔVII (8325 ΔVI *Δarl*): 8325 *Δyhc Δlyt Δgra Δsae Δtcs7 Δtcs3 Δarl*
8325 ΔVIII (8325 ΔVII *Δsrr*): 8325 *Δyhc Δlyt Δgra Δsae Δtcs7Δtcs3 Δarl Δsrr*
8325 ΔIX (8325 ΔVIII *Δpho*): 8325 *Δyhc Δlyt Δgra Δsae Δtcs7 Δtcs3 Δarl Δsrr Δpho*
8325 ΔX (8325 ΔIX *Δhss*): 8325 *Δyhc Δlyt Δgra Δsae Δtcs7 Δtcs3 Δarl Δsrr Δpho Δhss*
8325 ΔXI (8325 ΔX *Δnre*): 8325 *Δyhc Δlyt Δgra Δsae Δtcs7 Δtcs3 Δarl Δsrr Δpho Δhss Δnre*
8325 ΔXII (8325 ΔXI *Δbra*): 8325 *Δyhc Δlyt Δgra Δsae Δtcs7 Δtcs3 Δarl Δsrr Δpho Δhss Δnre Δbra*
8325 ΔXIII (8325 ΔXII *Δvra*): 8325 *Δyhc Δlyt Δgra Δsae Δtcs7 Δtcs3 Δarl Δsrr Δpho Δhss Δnre Δbra Δvra*
8325 ΔXIV (8325 ΔXIII *Δkdp*): 8325 *Δyhc Δlyt Δgra Δsae Δtcs7 Δtcs3 Δarl Δsrr Δpho Δhss Δnre Δbra Δvra Δkdp*
8325 ΔXV (8325 ΔXIV *Δagr):* 8325 *Δyhc Δlyt Δgra Δsae Δtcs7Δtcs3 Δarl Δsrr Δpho Δhss Δnre Δbra Δvra Δkdp Δagr.*

16. A mutant strain of *S. aureus* according to any one of claims 1 to 15, which ectopically expresses one or both of the two components, histidine kinase (HK) and response regulator (RR), of a TCS selected from the group of:
*yhcSR;*
the TCS comprised of the components HK and RR expressed either
i) by the genes *MW0199-MW0198* of *S. aureus* strain MW2 (tcs3), or
ii) by the genes *SAOUHSC_00185-SAOUHSC_00184* of *S. aureus* strain 8325 (tcs3), or
*iii)* by their corresponding orthologous genes of another *S. aureus* strain;
*lytSR,*
*graRS;*
*saeRS;*
the TCS comprised of the components HK and RR expressed either
i) by the genes *MW1207-MW1208* of strain MW2 (tcs7), or
ii) by the genes *SAOUHSC_01313-SAOUHSC_01314* of *S. aureus* strain 8325 (tcs7), or
*iii)* by their corresponding orthologous genes of another *S. aureus* strain;
*hssRS;*
*nreBC;*
*braRS;*
*kdpDE;*
*vraSR;*
*phoPR;*
*arlRS;*
*agr;*
*srrAB,* and
*walKR.*

17. Use of a mutant strain of ***S.** aureus* according to any one of claims 1 to 16 for the expression of a heterologous protein, a chimeric protein and/or a fusion protein.

18. Use of a mutant strain of *S. aureus* according to any one of claims 1 to 16 as candidate to living attenuated strain for vaccination purposes.

19. Use of a mutant strain of *S. aureus* according to any one of claims 1 to 16 for identifying compounds capable of inhibiting one or more TCSs.

20. The use according to claim 19, for additionally identifying candidates to antibiotics.

21. Use of a mutant strain of *S. aureus* according to any one of claims 1 to 16 for identifying promoters whose expression depends on environmental signals.

22. Use of a mutant strain of *S. aureus* according to any one of claims 1 to 16 for developing biosensor tracks for environmental signals.

23. Use of a mutant strain of *S. aureus* according to any one of claims 1 to 16 as platform or research tool.

24. The use according to claim 23, for studying *S. aureus* sensing system, the identification of the biological processes regulated by each individual TCSs, the possibilities of *in vivo* cross-talk among HKs and RRs each one belonging to different TCSs, the regulation of the expression of each TCSs, the inductor or inhibitor compounds affecting them, the possible influence of acting on different TCS in bacterial viability, the possible genes or systems that could complement the absence of the expression of a specific TCSs, the relation of each two-component with host-pathogen interactions including *S. aureus* cell adhesion, invasion, tissue colonization, pathogenicity and antibiotic-resistance capabilities and/or the development of *S. aureus* avirulent strains.

25. The use according to any one of claims 17 to 25, wherein the mutant strain of *S. aureus* is a strain of any one of claims 1 to 3 or 8 to 14.

26. The use according to any one of claims 21 to 25, wherein the phenotype of the used mutant strain is compared with the phenotype of a second mutant strain that differs from the first mutant strain in that it ectopically expresses:
a. one or both of the two components, histidine kinase and response regulator, of a TCS selected from the group of:
*yhcSR;*
the TCS comprised of the components HK and RR expressed either
i) by the genes *MW0199-MW0198* of *S. aureus* strain MW2 (tcs3), or
ii) by the genes SAOUHSC 00185-SAOUHSC 00184 of S. *aureus* strain 8325, or
*iii)* by their corresponding orthologous genes of another S. *aureus* strain;
*lytSR,*
*graRS;*
*saeRS;*
the TCS comprised of the components HK and RR expressed either
i) by the genes *MW1207-MW1208* of strain MW2 (tcs7), or
ii) by the genes *SAOUHSC_01313-SAOUHSC_01314* of *S. aureus* strain 8325, or
*iii)* by their corresponding orthologous genes of another S. *aureus* strain;
*hssRS;*
*nreBC;*
*braRS;*
*kdpDE;*
*vraSR;*
*phoPR;*
*arIRS;*
*agr;*
*srrAB;*
*kdpED-like,* and
*walKR;*
and/or
b. any other polypeptide or groups of polypeptides, or any other gene product.

27. The use according to claim 26, wherein at least one of the polypeptides of option b comprises a fragment with the amino acid sequence of a reporter, selection marker or labelling protein.

28. A method for identifying a compound as a blocker or inactivator of the walKR system which comprises the steps of:
a) culturing a mutant strain of *S. aureus* of any one of claims 1 to 3 or 8 to 14 under conditions wherein the WalKR system should be expressed,
b) comparing the growth rate of the mutant strain in the presence of the compound with the growth rate in the absence of the compound,
c) identifying the compound as a blocker or inactivator of the WalKR system when the growth rate is reduced in the presence of the compound,
d) optionally, checking whether the compound is a specific blocker of the expression of the *walKR* system by verifying that the reduction of the growth rate is observed when the strain or strains cultured in the presence of the compound is a *S. aureus* wild type strain and it is not observed when the strain of strains cultured in the presence of the compound is a *S. aureus* strains wherein the *walKR* system is expressed under the control of a promoter that is different from the natural one and that gives rise to the expression of walKR under the culture conditions of step a) and b),
e) optionally, verifying that the blocking or inactivation cannot be reverted by the expression of another TCSs or by a combination of TCSs by checking that the reduction of the growth rate is not reverted when the strain or strains cultured in the presence of the compound is any one of the mutant strains of claims 1 to 4, the mutant strain of claim 9, the parental strain corresponding to the mutant strain used in step a), and/or any other wild type strain,
f) optionally and additionally, identifying the compound as a candidate to antimicrobial compound when step d) has been carried out and the reduction of the growth rate is not reverted at least in the parental strain or any other wild type strain.

29. A method for identifying physical or chemical signals sense by two-component systems in assays wherein the expression of a reporter gene under the control of a promoter regulated by a two-component system is compared with the phenotype of the same mutant strain except for that it ectopically expresses a TCS selected from the group of:
*yhcSR;*
the TCS comprised of the components HK and RR expressed either
i) by the genes *MW0199-MW0198* of *S. aureus* strain MW2 (tcs3), or
ii) by the genes *SAOUHSC_00185-SAOUHSC_00184* of *S. aureus* strain 8325 (tcs3), or
*iii)* by their corresponding orthologous genes of another *S. aureus* strain;
*lytSR,*
*graRS;*
*saeRS;*
the TCS comprised of the components HK and RR expressed either
i) by the genes *MW1207-MW1208* of strain MW2 (tcs7), or
ii) by the genes *SAOUHSC_01313-SAOUHSC_01314* of *S. aureus* strain 8325 (tcs7), or
*iii)* by their corresponding orthologous genes of another S. *aureus* strain;
*hssRS;*
*nreBC;*
*braRS;*
*kdpDE;*
*vraSR;*
*phoPR;*
*arIRS;*
*agr;*
*srrAB;*
*kdpED-like,* and
*walKR.*


SEQUENCE LISTING

<110>  Universidad Publica de Navarra
       Consejo Superior de Investigaciones Científicas

<120>  NOVEL STRAINS OF STAPHYLOCOCCUS AUREUS AND USES THEREOF

<130>  PCT-803

<160>  164

<170>  PatentIn version 3.5

<210>  1
<211>  27
<212>  DNA
<213>  Artificial sequence

<220>
<223>  Primer for deletion of the TCS tcs3

<400>  1
ggatccgtta tcagaaacaa ttgatca                                          27


<210>  2
<211>  28
<212>  DNA
<213>  Artificial sequence

<220>
<223>  Primer for deletion of the TCS tcs3

<400>  2
ccatggtgaa tcatctccaa aaatttat                                         28


<210>  3
<211>  28
<212>  DNA
<213>  Artificial sequence

<220>
<223>  Primer for deletion of the TCS tcs3

<400>  3
ccatggatta gcacataact aatgatta                                         28


<210>  4
<211>  26
<212>  DNA
<213>  Artificial sequence

<220>
<223>  Primer for deletion of the TCS tcs3

<400>  4
ctcgagtaaa ggcgatgcag ttaata                                           26


<210>  5
<211>  26

<212>  DNA
<213>  Artificial sequence

<220>
<223>  Primer for deletion of the TCS lyt

<400>  5
ggatcctgac gttgaacaaa cgaata                                          26


<210>  6
<211>  24
<212>  DNA
<213>  Artificial sequence

<220>
<223>  Primer for deletion of the TCS lyt

<400>  6
aagcttgata gcacctcagt gaat                                            24


<210>  7
<211>  27
<212>  DNA
<213>  Artificial sequence

<220>
<223>  Primer for deletion of the TCS lyt

<400>  7
aagcttcagt aatccttttt tttatgc                                         27


<210>  8
<211>  25
<212>  DNA
<213>  Artificial sequence

<220>
<223>  Primer for deletion of the TCS lyt

<400>  8
gaattccaac tgtaccgata ccaat                                           25


<210>  9
<211>  27
<212>  DNA
<213>  Artificial sequence

<220>
<223>  Primer for deletion of the TCS gra

<400>  9
ggatcccaaa tagatattgc tgtattc                                         27


<210>  10
<211>  26
<212>  DNA
<213>  Artificial sequence

<220>
<223>  Primer for deletion of the TCS gra

<400>  10
ccatggccat atcacccaat atcatt                                          26


<210>  11
<211>  26
<212>  DNA
<213>  Artificial sequence

<220>
<223>  Primer for deletion of the TCS gra

<400>  11
ccatggacat gcgttttgtt acttag                                          26


<210>  12
<211>  25
<212>  DNA
<213>  Artificial sequence

<220>
<223>  Primer for deletion of the TCS gra

<400>  12
ctcgagttaa cgccacctaa aacac                                           25


<210>  13
<211>  16
<212>  DNA
<213>  Artificial sequence

<220>
<223>  Primer for deletion of the TCS sae

<400>  13
atttgaatgg ataggc                                                     16


<210>  14
<211>  16
<212>  DNA
<213>  Artificial sequence

<220>
<223>  Primer for deletion of the TCS sae

<400>  14
attacgtcat aatccg                                                     16


<210>  15
<211>  44
<212>  DNA
<213>  Artificial sequence

<220>
<223>  Primer for deletion of the TCS sae

<400>  15
aaatcggatt atgacgtaat aagtgggtca tctatttttt cacc                        44


<210>  16
<211>  16
<212>  DNA
<213>  Artificial sequence

<220>
<223>  Primer for deletion of the TCS sae

<400>  16
taacactaca aatcgc                                                       16


<210>  17
<211>  27
<212>  DNA
<213>  Artificial sequence

<220>
<223>  Primer for deletion of the TCS tcs7

<400>  17
   ggatccaaag gggatctaat tatgata                                            27


<210>  18
<211>  27
<212>  DNA
<213>  Artificial sequence

<220>
<223>  Primer for deletion of the TCS tcs7

<400>  18
aagcttattt tattccgccc ttttaaa                                           27


<210>  19
<211>  28
<212>  DNA
<213>  Artificial sequence

<220>
<223>  Primer for deletion of the TCS tcs7

<400>  19
aagcttatac aaacaaaaaa gtattgag                                          28


<210>  20
<211>  27
<212>  DNA
<213>  Artificial sequence

<220>
<223>  Primer for deletion of the TCS tcs7

<400>  20
gaattcaaaa atacttctct ctagcaa                                           27

<210>  21
<211>  16
<212>  DNA
<213>  Artificial sequence

<220>
<223>  Primer for deletion of the TCS arl

<400>  21
gtttcctttt tggagg                                                        16


<210>  22
<211>  16
<212>  DNA
<213>  Artificial sequence

<220>
<223>  Primer for deletion of the TCS arl

<400>  22
tcatgactga gacgtc                                                        16


<210>  23
<211>  16
<212>  DNA
<213>  Artificial sequence

<220>
<223>  Primer for deletion of the TCS arl

<400>  23
gcgtcatttg tacacc                                                        16


<210>  24
<211>  16
<212>  DNA
<213>  Artificial sequence

<220>
<223>  Primer for deletion of the TCS arl

<400>  24
gtgatgaggt ttaaac                                                        16


<210>  25
<211>  27
<212>  DNA
<213>  Artificial sequence

<220>
<223>  Primer for deletion of the TCS srr

<400>  25
ggatccgtga atttactaat ttaatga                                            27


<210>  26
<211>  27

<212>  DNA
<213>  Artificial sequence

<220>
<223>  Primer for deletion of the TCS srr

<400>  26
ctcgaggcct cttggccatt acttgct                                          27


<210>  27
<211>  26
<212>  DNA
<213>  Artificial sequence

<220>
<223>  Primer for deletion of the TCS srr

<400>  27
ctcgagatcg aagagcatgg tggttc                                           26


<210>  28
<211>  26
<212>  DNA
<213>  Artificial sequence

<220>
<223>  Primer for deletion of the TCS srr

<400>  28
gaattcatct tcgcaatgca cataaa                                           26


<210>  29
<211>  16
<212>  DNA
<213>  Artificial sequence

<220>
<223>  Primer for deletion of the TCS pho

<400>  29
tggtagtaag ataccc                                                      16


<210>  30
<211>  16
<212>  DNA
<213>  Artificial sequence

<220>
<223>  Primer for deletion of the TCS pho

<400>  30
aaagtggtaa cagcgc                                                      16


<210>  31
<211>  36
<212>  DNA
<213>  Artificial sequence

<220>
<223>  Primer for deletion of the TCS pho

<400>  31
acacgcgctg ttaccacttt ggtatgcctc cctaac                                36


<210>  32
<211>  16
<212>  DNA
<213>  Artificial sequence

<220>
<223>  Primer for deletion of the TCS pho

<400>  32
cacatggtac agctcc                                                      16


<210>  33
<211>  20
<212>  DNA
<213>  Artificial sequence

<220>
<223>  Primer for deletion of the TCS yhc

<400>  33
ttaaaatgtg tgtaattgca                                                  20


<210>  34
<211>  20
<212>  DNA
<213>  Artificial sequence

<220>
<223>  Primer for deletion of the TCS yhc

<400>  34
caaatcgctc caattcattt                                                  20


<210>  35
<211>  22
<212>  DNA
<213>  Artificial sequence

<220>
<223>  Primer for deletion of the TCS yhc

<400>  35
aatgagcttt taaatatttg tc                                               22


<210>  36
<211>  19
<212>  DNA
<213>  Artificial sequence

<220>
<223>  Primer for deletion of the TCS yhc

<400>  36
gtgtgttaca tcgctttta                                                    19


<210>  37
<211>  16
<212>  DNA
<213>  Artificial sequence

<220>
<223>  Primer for deletion of the TCS vra

<400>  37
gtattaccag gtgcag                                                       16


<210>  38
<211>  16
<212>  DNA
<213>  Artificial sequence

<220>
<223>  Primer for deletion of the TCS vra

<400>  38
tcaatagttc gtattg                                                       16


<210>  39
<211>  36
<212>  DNA
<213>  Artificial sequence

<220>
<223>  Primer for deletion of the TCS vra

<400>  39
aattcaatac gaactattga cgataaatca cctcta                                 36


<210>  40
<211>  16
<212>  DNA
<213>  Artificial sequence

<220>
<223>  Primer for deletion of the TCS vra

<400>  40
acgtggcctt ttggcg                                                       16


<210>  41
<211>  16
<212>  DNA
<213>  Artificial sequence

<220>
<223>  Primer for deletion of the TCS agr

<400>  41
agcactgagt ccaagg                                                       16

<210>  42
<211>  16
<212>  DNA
<213>  Artificial sequence

<220>
<223>  Primer for deletion of the TCS agr

<400>  42
ttttacacca ctctcc                                                       16


<210>  43
<211>  44
<212>  DNA
<213>  Artificial sequence

<220>
<223>  Primer for deletion of the TCS agr

<400>  43
gtgaggagag tggtgtaaaa aagataataa agtcagttaa cggc                        44


<210>  44
<211>  16
<212>  DNA
<213>  Artificial sequence

<220>
<223>  Primer for deletion of the TCS agr

<400>  44
cagttattag caggat                                                       16


<210>  45
<211>  27
<212>  DNA
<213>  Artificial sequence

<220>
<223>  Primer for deletion of the TCS kdp

<400>  45
ggatccccaa tgatattagt taatcca                                           27


<210>  46
<211>  24
<212>  DNA
<213>  Artificial sequence

<220>
<223>  Primer for deletion of the TCS kdp

<400>  46
ccatggaacc ttcacctcga tagc                                              24


<210>  47
<211>  27

<212>  DNA
<213>  Artificial sequence

<220>
<223>  Primer for deletion of the TCS kdp

<400>  47
ccatggttaa ataaaaaaga tcgctgc                                          27


<210>  48
<211>  28
<212>  DNA
<213>  Artificial sequence

<220>
<223>  Primer for deletion of the TCS kdp

<400>  48
gaattcgttt tcaataattg attctctg                                         28


<210>  49
<211>  16
<212>  DNA
<213>  Artificial sequence

<220>
<223>  Primer for deletion of the TCS hss

<400>  49
cattaatagc gacctc                                                      16


<210>  50
<211>  16
<212>  DNA
<213>  Artificial sequence

<220>
<223>  Primer for deletion of the TCS hss

<400>  50
ctcccttatc tttttc                                                      16


<210>  51
<211>  36
<212>  DNA
<213>  Artificial sequence

<220>
<223>  Primer for deletion of the TCS hss

<400>  51
aaatgaaaaa gataagggag tctctacctc ctgaaa                                36


<210>  52
<211>  16
<212>  DNA
<213>  Artificial sequence

<220>
<223>  Primer for deletion of the TCS hss

<400>  52
aggtgtagtg tgcatc                                                       16


<210>  53
<211>  27
<212>  DNA
<213>  Artificial sequence

<220>
<223>  Primer for deletion of the TCS nre

<400>  53
ggatcccgca acattagtaa ccaatat                                           27


<210>  54
<211>  26
<212>  DNA
<213>  Artificial sequence

<220>
<223>  Primer for deletion of the TCS nre

<400>  54
ccatgggact tacaccctaa ttcatc                                            26


<210>  55
<211>  30
<212>  DNA
<213>  Artificial sequence

<220>
<223>  Primer for deletion of the TCS nre

<400>  55
ccatggagtt tgaaattaat ataattcagt                                        30


<210>  56
<211>  28
<212>  DNA
<213>  Artificial sequence

<220>
<223>  Primer for deletion of the TCS nre

<400>  56
ctcgagttat aaacagcaag acttagaa                                          28


<210>  57
<211>  26
<212>  DNA
<213>  Artificial sequence

<220>
<223>  Primer for deletion of the TCS bra

<400>  57
ggatccgctg cagaatcagt aatatt                                            26


<210>  58
<211>  27
<212>  DNA
<213>  Artificial sequence

<220>
<223>  Primer for deletion of the TCS bra

<400>  58
aagcttctat aatcttcttc cttcaat                                           27


<210>  59
<211>  29
<212>  DNA
<213>  Artificial sequence

<220>
<223>  Primer for deletion of the TCS bra

<400>  59
aagcttaaac tttcaatatt gtaagcata                                         29


<210>  60
<211>  26
<212>  DNA
<213>  Artificial sequence

<220>
<223>  Primer for deletion of the TCS bra

<400>  60
gaattcgtgc cataacaatc ttaact                                            26


<210>  61
<211>  20
<212>  DNA
<213>  Artificial sequence

<220>
<223>  Primer for testing deletion of the TCS tcs3

<400>  61
atctactcaa gtactgcttt                                                   20


<210>  62
<211>  21
<212>  DNA
<213>  Artificial sequence

<220>
<223>  Primer for testing deletion of the TCS tcs3

<400>  62
tgtttcaaac gtctttgtat a                                                 21

<210>  63
<211>  22
<212>  DNA
<213>  Artificial sequence

<220>
<223>  Primer for testing deletion of the TCS lyt

<400>  63
gaaaagaaaa atgtagattt ga                                                22


<210>  64
<211>  21
<212>  DNA
<213>  Artificial sequence

<220>
<223>  Primer for testing deletion of the TCS lyt

<400>  64
aataacatca ttggcaaatt g                                                 21


<210>  65
<211>  20
<212>  DNA
<213>  Artificial sequence

<220>
<223>  Primer for testing deletion of the TCS gra

<400>  65
aaatgacttg gattcagtgt                                                   20


<210>  66
<211>  21
<212>  DNA
<213>  Artificial sequence

<220>
<223>  Primer for testing deletion of the TCS gra

<400>  66
aaaaagatag atggcataat g                                                 21


<210>  67
<211>  21
<212>  DNA
<213>  Artificial sequence

<220>
<223>  Primer for testing deletion of the TCS sae

<400>  67
gttggtgatt ttagactttt a                                                 21


<210>  68
<211>  21

<212>  DNA
<213>  Artificial sequence

<220>
<223>  Primer for testing deletion of the TCS sae

<400>  68
ataagattgt agagcacata a                                                21


<210>  69
<211>  20
<212>  DNA
<213>  Artificial sequence

<220>
<223>  Primer for testing deletion of the TCS tcs7

<400>  69
aattgccgga agatgttaaa                                                  20


<210>  70
<211>  20
<212>  DNA
<213>  Artificial sequence

<220>
<223>  Primer for testing deletion of the TCS tcs7

<400>  70
tatctttgta ggctttatcg                                                  20


<210>  71
<211>  19
<212>  DNA
<213>  Artificial sequence

<220>
<223>  Primer for testing deletion of the TCS arl

<400>  71
agtgatctga aacaattcc                                                   19


<210>  72
<211>  20
<212>  DNA
<213>  Artificial sequence

<220>
<223>  Primer for testing deletion of the TCS arl

<400>  72
aagaatagta aataaaacgc                                                  20


<210>  73
<211>  20
<212>  DNA
<213>  Artificial sequence

<220>
<223>  Primer for testing deletion of the TCS srr

<400>  73
tttgtacaaa ggtagacttg                                                    20


<210>  74
<211>  21
<212>  DNA
<213>  Artificial sequence

<220>
<223>  Primer for testing deletion of the TCS srr

<400>  74
tggttacgga ttactttaaa g                                                  21


<210>  75
<211>  20
<212>  DNA
<213>  Artificial sequence

<220>
<223>  Primer for testing deletion of the TCS pho

<400>  75
aatgagacca tatgaaagcc                                                    20


<210>  76
<211>  21
<212>  DNA
<213>  Artificial sequence

<220>
<223>  Primer for testing deletion of the TCS pho

<400>  76
caggtgcaaa cattaattat g                                                  21


<210>  77
<211>  21
<212>  DNA
<213>  Artificial sequence

<220>
<223>  Primer for testing deletion of the TCS yhc

<400>  77
catataaagg atcaccaata a                                                  21


<210>  78
<211>  21
<212>  DNA
<213>  Artificial sequence

<220>
<223>  Primer for testing deletion of the TCS yhc

<400>  78
catagttatt cattatacca c                                                21

<210>  79
<211>  19
<212>  DNA
<213>  Artificial sequence

<220>
<223>  Primer for testing deletion of the TCS vra

<400>  79
tgacgaacaa gtgaaatgg                                                   19

<210>  80
<211>  20
<212>  DNA
<213>  Artificial sequence

<220>
<223>  Primer for testing deletion of the TCS vra

<400>  80
cgttctatta ttgggatgtg                                                  20

<210>  81
<211>  21
<212>  DNA
<213>  Artificial sequence

<220>
<223>  Primer for testing deletion of the TCS agr

<400>  81
ggggatgtta ttaattatga a                                                21

<210>  82
<211>  20
<212>  DNA
<213>  Artificial sequence

<220>
<223>  Primer for testing deletion of the TCS agr

<400>  82
tagtcattta tacgaaggga                                                  20

<210>  83
<211>  22
<212>  DNA
<213>  Artificial sequence

<220>
<223>  Primer for testing deletion of the TCS kdp

<400>  83
tactaattaa acatgataat gg                                               22

<210>  84
<211>  28
<212>  DNA
<213>  Artificial sequence

<220>
<223>  Primer for testing deletion of the TCS kdp

<400>  84
gaattcgttt tcaataattg attctctg                                          28


<210>  85
<211>  21
<212>  DNA
<213>  Artificial sequence

<220>
<223>  Primer for testing deletion of the TCS hss

<400>  85
catacattgt gtcgtttaaa a                                                 21


<210>  86
<211>  22
<212>  DNA
<213>  Artificial sequence

<220>
<223>  Primer for testing deletion of the TCS hss

<400>  86
aaccaatgat taagctaata aa                                                22


<210>  87
<211>  20
<212>  DNA
<213>  Artificial sequence

<220>
<223>  Primer for testing deletion of the TCS nre

<400>  87
ttaagttcag cgtcggatat                                                   20


<210>  88
<211>  23
<212>  DNA
<213>  Artificial sequence

<220>
<223>  Primer for testing deletion of the TCS nre

<400>  88
aactttacat tattacgatg aaa                                               23


<210>  89
<211>  20

<212>  DNA
<213>  Artificial sequence

<220>
<223>  Primer for testing deletion of the TCS bra

<400>  89
tactttctgc ttgttactgt                                                    20


<210>  90
<211>  20
<212>  DNA
<213>  Artificial sequence

<220>
<223>  Primer for testing deletion of the TCS bra

<400>  90
acacaagcgt atattcaatc                                                    20


<210>  91
<211>  46
<212>  DNA
<213>  Artificial sequence

<220>
<223>  primer for constructing plasmids expressing SaeR (reverse, with
       tail BamHI XmaI KpnI)

<400>  91
ggtaccaata cccgggcaat ggatccatta tcggctcctt tcaaat                       46


<210>  92
<211>  37
<212>  DNA
<213>  Artificial sequence

<220>
<223>  primer for constructing plasmids expressing SaeR (forward, with
       tail XhoI SalI)

<400>  92
gtcgacattg ctcgagcgaa cagaggtgaa aaaatag                                 37


<210>  93
<211>  48
<212>  DNA
<213>  Artificial sequence

<220>
<223>  primer for constructing plasmids expressing NreR (reverse, with
       tail BamHI XmaI KpnI)

<400>  93
ggtaccaata cccgggcaat ggatccaatt tcaaactcta aaactcta                     48


<210>  94
<211>  36

<212>  DNA
<213>  Artificial sequence

<220>
<223>  primer for constructing plasmids expressing NreR (forward, with
       tail XhoI SalI)

<400>  94
gtcgacattg ctcgagcata cattggggga ataaaa                                36


<210>  95
<211>  45
<212>  DNA
<213>  Artificial sequence

<220>
<223>  primer for constructing plasmids expressing WalR (reverse, with
       tail BamHI XmaI KpnI)

<400>  95
ggtaccaata cccgggcaat ggatccctct actcatgttg ttgga                      45


<210>  96
<211>  36
<212>  DNA
<213>  Artificial sequence

<220>
<223>  primer for constructing plasmids expressing WalR (forward, with
       tail XhoI SalI)

<400>  96
gtcgacattg ctcgagttaa gaaaagaggt ttatgc                                36


<210>  97
<211>  46
<212>  DNA
<213>  Artificial sequence

<220>
<223>  primer for constructing plasmids expressing TCS3R (reverse, with
       tail BamHI XmaI KpnI)

<400>  97
ggtaccaata cccgggcaat ggatccaatc tattttgctt gcttac                     46


<210>  98
<211>  35
<212>  DNA
<213>  Artificial sequence

<220>
<223>  primer for constructing plasmids expressing TCS3R (forward, with
       tail XhoI SalI)

<400>  98
gtcgacattg ctcgagttca agggggaatg tagat                                  35

<210>  99
<211>  46
<212>  DNA
<213>  Artificial sequence

<220>
<223>  primer for constructing plasmids expressing LytR (reverse, with
       tail BamHI XmaI KpnI)

<400>  99
ggtaccaata cccgggcaat ggatccactg ttaaagtaac cctatc                    46


<210>  100
<211>  36
<212>  DNA
<213>  Artificial sequence

<220>
<223>  primer for constructing plasmids expressing LytR (forward, with
       tail XhoI SalI)

<400>  100
gtcgacattg ctcgaggaca agaggaggaa taaata                              36


<210>  101
<211>  46
<212>  DNA
<213>  Artificial sequence

<220>
<223>  primer for constructing plasmids expressing GraR (reverse, with
       tail BamHI XmaI KpnI)

<400>  101
ggtaccaata cccgggcaat ggatcccaaa ttattcatga gccata                    46


<210>  102
<211>  36
<212>  DNA
<213>  Artificial sequence

<220>
<223>  primer for constructing plasmids expressing GraR (forward, with
       tail XhoI SalI)

<400>  102
gtcgacattg ctcgagaatg atattgggtg atatgg                              36


<210>  103
<211>  46
<212>  DNA
<213>  Artificial sequence

<220>
<223>  primer for constructing plasmids expressing TCS7R (reverse, with
       tail BamHI XmaI KpnI)

<400>  103
ggtaccaata cccgggcaat ggatccattt agatccagcc tttttc                    46

<210>  104
<211>  35
<212>  DNA
<213>  Artificial sequence

<220>
<223>  primer for constructing plasmids expressing TCS7R (forward, with
       tail XhoI SalI)

<400>  104
gtcgacattg ctcgagaaca ggaggaatag catga                                35


<210>  105
<211>  46
<212>  DNA
<213>  Artificial sequence

<220>
<223>  primer for constructing plasmids expressing ArlR (reverse, with
       tail BamHI XmaI KpnI)

<400>  105
ggtaccaata cccgggcaat ggatcctttg tcatcgtatc acatac                    46


<210>  106
<211>  36
<212>  DNA
<213>  Artificial sequence

<220>
<223>  primer for constructing plasmids expressing ArlR (forward, with
       tail XhoI SalI)

<400>  106
gtcgacattg ctcgaggtaa tatgaggtgt acaaat                               36


<210>  107
<211>  45
<212>  DNA
<213>  Artificial sequence

<220>
<223>  primer for constructing plasmids expressing SrrA(R) (reverse, with
       tail BamHI XmaI KpnI)

<400>  107
ggtaccaata cccgggcaat ggatccacta tttagccggc tcatc                     45


<210>  108
<211>  34
<212>  DNA
<213>  Artificial sequence

<220>
<223>  primer for constructing plasmids expressing SrrA(R) (forward, with
       tail XhoI SalI)

<400>  108
gtcgacattg ctcgagtgtg tgggaggtat gacc                                34


<210>  109
<211>  46
<212>  DNA
<213>  Artificial sequence

<220>
<223>  Primer for constructing plasmids expressing PhoP(R) (reverse,
       with tail BamHIXmaI KpnI)

<400>  109
ggtaccaata cccgggcaat ggatcctcat cattgttctt taggtc                   46


<210>  110
<211>  35
<212>  DNA
<213>  Artificial sequence

<220>
<223>  primer for constructing plasmids expressing PhoP(R) (forward, with
       tail XhoI SalI)

<400>  110
gtcgacattg ctcgagataa gttagggagg catac                               35


<210>  111
<211>  47
<212>  DNA
<213>  Artificial sequence

<220>
<223>  primer for constructing plasmids expressing YhcR (reverse, with
       tail BamHI XmaI KpnI)

<400>  111
ggtaccaata cccgggcaat ggatccttct aaatcaactt attttcc                  47


<210>  112
<211>  36
<212>  DNA
<213>  Artificial sequence

<220>
<223>  primer for constructing plasmids expressing YhcR (forward, with
       tail XhoI SalI)

<400>  112
gtcgacattg ctcgagattt aaggagataa cccatg                              36


<210>  113
<211>  48
<212>  DNA
<213>  Artificial sequence

<220>
<223>  primer for constructing plasmids expressing VraR (reverse, with

tail BamHI XmaI KpnI)

<400> 113
ggtaccaata cccgggcaat ggatccgaac tattgaatta aattatgt 48


<210> 114
<211> 36
<212> DNA
<213> Artificial sequence

<220>
<223> primer for constructing plasmids expressing VraR (forward, with
tail XhoI SalI)

<400> 114
gtcgacattg ctcgagaaat aaggaggatt cgtatg 36


<210> 115
<211> 49
<212> DNA
<213> Artificial sequence

<220>
<223> primer for constructing plasmids expressing AgrA(R) (reverse, with
tail BamHI XmaI KpnI)

<400> 115
ggtaccaata cccgggcaat ggatcctatc ttattatatt tttttaacg 49


<210> 116
<211> 36
<212> DNA
<213> Artificial sequence

<220>
<223> primer for constructing plasmids expressing AgrA(R) (forward, with
tail XhoI SalI)

<400> 116
gtcgacattg ctcgagcata aggatgtgaa tgtatg 36


<210> 117
<211> 46
<212> DNA
<213> Artificial sequence

<220>
<223> primer for constructing plasmids expressing KdpE(R) (reverse, with
tail BamHI XmaI KpnI)

<400> 117
ggtaccaata cccgggcaat ggatccatta tttctctttc cactgc 46


<210> 118
<211> 36
<212> DNA
<213> Artificial sequence

<220>
<223>  primer for constructing plasmids expressing KdpE(R) (forward, with
      tail XhoI SalI)

<400>  118
gtcgacattg ctcgagaatg aaggagacgt ataatg                              36


<210>  119
<211>  46
<212>  DNA
<213>  Artificial sequence

<220>
<223>  primer for constructing plasmids expressing HssR (reverse, with
      tail BamHI XmaI KpnI)

<400>  119
ggtaccaata cccgggcaat ggatccttta aacatgattc tccacc                   46


<210>  120
<211>  36
<212>  DNA
<213>  Artificial sequence

<220>
<223>  primer for constructing plasmids expressing HssR (forward, with
      tail XhoI SalI)

<400>  120
gtcgacattg ctcgaggata agggagttta tagcta                              36


<210>  121
<211>  46
<212>  DNA
<213>  Artificial sequence

<220>
<223>  primer for constructing plasmids expressing BraR (reverse, with
      tail BamHIXmaI KpnI)

<400>  121
ggtaccaata cccgggcaat ggatccccta tactttatat ccgaca                   46


<210>  122
<211>  36
<212>  DNA
<213>  Artificial sequence

<220>
<223>  primer for constructing plasmids expressing BraR (forward, with
      tail XhoI SalI)

<400>  122
gtcgacattg ctcgagttga aggaagaaga ttatag                              36


<210>  123
<211>  32
<212>  DNA

<213>  Artificial sequencep

<220>
<223>  rimer for constructing plasmids expressing SaeS (reverse, with
       tail XmaI AscI)

<400>  123
ggcgcgcccg ggatcggatt atgacgtaat gt                                    32


<210>  124
<211>  26
<212>  DNA
<213>  Artificial sequence

<220>
<223>  primer for constructing plasmids expressing SaeS (forward, with
       tail BamHI)

<400>  124
ggatccattt gaaaggagcc gataat                                           26


<210>  125
<211>  33
<212>  DNA
<213>  Artificial sequence

<220>
<223>  primer for constructing plasmids expressing NreS (reverse, with
       tail XmaI AscI)

<400>  125
ggcgcgcccg gggtatgttt caaattggaa tgt                                   33


<210>  126
<211>  26
<212>  DNA
<213>  Artificial sequence

<220>
<223>  primer for constructing plasmids expressing NreS (forward, with
       tail BamHI)

<400>  126
ggatccagat gaattagggt gtaagt                                           26


<210>  127
<211>  32
<212>  DNA
<213>  Artificial sequence

<220>
<223>  primer for constructing plasmids expressing WalK(S) (reverse, with
       tail XmaI AscI)

<400>  127
ggcgcgcccg ggccttatta ttcatcccaa tc                                    32


<210>  128

<211>  25
<212>  DNA
<213>  Artificial sequence

<220>
<223>  primer for constructing plasmids expressing WalK(S) (forward, with
       tail BamHI)

<400>  128
ggatccatga gtagaggtcg aaacg                                          25


<210>  129
<211>  32
<212>  DNA
<213>  Artificial sequence

<220>
<223>  primer for constructing plasmids expressing TCS3S (reverse, with
       tail XmaI AscI)

<400>  129
ggcgcgcccg ggtaccttaa acatctacat tc                                  32


<210>  130
<211>  26
<212>  DNA
<213>  Artificial sequence

<220>
<223>  primer for constructing plasmids expressing TCS3S (forward, with
       tail BamHI)

<400>  130
ggatcctttt tggagatgat tcaatg                                         26


<210>  131
<211>  32
<212>  DNA
<213>  Artificial sequence

<220>
<223>  primer for constructing plasmids expressing LytS (reverse, with
       tail XmaI AscI)

<400>  131
ggcgcgcccg ggtatttatt cctcctcttg tc                                  32


<210>  132
<211>  26
<212>  DNA
<213>  Artificial sequence

<220>
<223>  primer for constructing plasmids expressing LytS (forward, with
       tail BamHI)

<400>  132
ggatccaatt tactgaggtg ctatcg                                         26

<210>  133
<211>  32
<212>  DNA
<213>  Artificial sequence

<220>
<223>  primer for constructing plasmids expressing GraS (reverse, with
       tail XmaI-AscI)

<400>  133
ggcgcgcccg ggcgcatgtt taaaatgaca aa                                    32


<210>  134
<211>  26
<212>  DNA
<213>  Artificial sequence

<220>
<223>  primer for constructing plasmids expressing GraS (forward, with
       tail BamHI)

<400>  134
ggatcctagg aaaaggatat atggct                                           26


<210>  135
<211>  32
<212>  DNA
<213>  Artificial sequence

<220>
<223>  primer for constructing plasmids expressing TCS7S (reverse, with
       tail XmaI AscI)

<400>  135
ggcgcgcccg ggaaagatgt catgctattc ct                                    32


<210>  136
<211>  26
<212>  DNA
<213>  Artificial sequence

<220>
<223>  primer for constructing plasmids expressing TCS7S (forward, with
       tail BamHI)

<400>  136
ggatccaaag ggcggaataa aatatg                                           26


<210>  137
<211>  34
<212>  DNA
<213>  Artificial sequence

<220>
<223>  primer for constructing plasmids expressing ArlS (reverse, with
       tail XmaI AscI)

<400>  137

ggcgcgcccg gggattaaaa tatgatttta aacg                                    34


<210>  138
<211>  25
<212>  DNA
<213>  Artificial sequence

<220>
<223>  primer for constructing plasmids expressing ArlS (forward, with
       tail BamHI)

<400>  138
ggatcctggc gttgggtatg tgata                                             25


<210>  139
<211>  32
<212>  DNA
<213>  Artificial sequence

<220>
<223>  primer for constructing plasmids expressing SrrB(S) (reverse, with
       tail XmaI AscI)

<400>  139
ggcgcgcccg ggcaatttta ttctggtttt gg                                     32


<210>  140
<211>  26
<212>  DNA
<213>  Artificial sequence

<220>
<223>  primer for constructing plasmids expressing SrrB(S) (forward, with
       tail BamHI)

<400>  140
ggatccattt gaggttaaat ctaatg                                            26


<210>  141
<211>  35
<212>  DNA
<213>  Artificial sequence

<220>
<223>  primer for constructing plasmids expressing PhoR(S) (reverse, with
       tail XmaI AscI)

<400>  141
ggcgcgcccg ggttttattc tttataatct tttag                                  35


<210>  142
<211>  26
<212>  DNA
<213>  Artificial sequence

<220>
<223>  primer for constructing plasmids expressing PhoR(S) (forward, with
       tail BamHI)

<400>  142
ggatccattg gaaagaccta aagaac                                          26


<210>  143
<211>  32
<212>  DNA
<213>  Artificial sequence

<220>
<223>  primer for constructing plasmids expressing YhcS (reverse, with
       tail XmaI AscI)

<400>  143
ggcgcgcccg gggctatttt ataggaattg tg                                    32


<210>  144
<211>  26
<212>  DNA
<213>  Artificial sequence

<220>
<223>  primer for constructing plasmids expressing YhcS (forward, with
       tail BamHI)

<400>  144
ggatccaaat gaattggagc gatttg                                          26


<210>  145
<211>  32
<212>  DNA
<213>  Artificial sequence

<220>
<223>  primer for constructing plasmids expressing VraS (reverse, with
       tail XmaI AscI)

<400>  145
ggcgcgcccg ggctttaatc gtcatacgaa tc                                   32


<210>  146
<211>  26
<212>  DNA
<213>  Artificial sequence

<220>
<223>  primer for constructing plasmids expressing VraS (forward, with
       tail BamHI)

<400>  146
ggatccgaga cgtagaggtg atttat                                          26


<210>  147
<211>  32
<212>  DNA
<213>  Artificial sequence

<220>

<223>  primer for constructing plasmids expressing AgrC(S) (reverse, with
      tail XmaI AscI)

<400>  147
ggcgcgcccg ggggctagtt gttaataatt tc                                    32


<210>  148
<211>  26
<212>  DNA
<213>  Artificial sequence

<220>
<223>  Primer for constructing plasmids expressing AgrC(S) (forward,
      with tail BamHI)

<400>  148
ggatcctata agagaaagtg tgatag                                           26


<210>  149
<211>  32
<212>  DNA
<213>  Artificial sequence

<220>
<223>  primer for constructing plasmids expressing KdpD(S) (reverse, with
      tail XmaI AscI)

<400>  149
ggcgcgcccg ggcattatac gtctccttca tt                                    32


<210>  150
<211>  25
<212>  DNA
<213>  Artificial sequence

<220>
<223>  primer for constructing plasmids expressing KdpD(S) (forward, with
      tail BamHI)

<400>  150
ggatcctatc gaggtgaagg ttatg                                            25


<210>  151
<211>  33
<212>  DNA
<213>  Artificial sequence

<220>
<223>  primer for constructing plasmids expressing HssS (reverse, with
      tail XmaI AscI)

<400>  151
ggcgcgcccg ggagattaaa gtgaattatt tgg                                   33


<210>  152
<211>  25
<212>  DNA
<213>  Artificial sequence

<220>
<223>  primer for constructing plasmids expressing HssS (forward, with
      tail BamHI)

<400>  152
ggatcccaag gctataaggt ggaga                                          25


<210>  153
<211>  33
<212>  DNA
<213>  Artificial sequence

<220>
<223>  primer for constructing plasmids expressing BraS (reverse, with
      tail XmaI AscI)

<400>  153
ggcgcgcccg ggtttttatt catctggaaa ttg                                 33


<210>  154
<211>  26
<212>  DNA
<213>  Artificial sequence

<220>
<223>  primer for constructing plasmids expressing BraS (forward, with
      tail BamHI)

<400>  154
ggatccagta tagggtgaat gcaatg                                         26


<210>  155
<211>  29
<212>  DNA
<213>  Artificial sequence

<220>
<223>  OSA34: primer for generating a fragment with the ribosome-binding
      site and 5′ portion of yycF

<400>  155
tctagatatt aatgatttaa gaaaagagg                                      29


<210>  156
<211>  30
<212>  DNA
<213>  Artificial sequence

<220>
<223>  OSA35bis: primer for generating a fragment with the
      ribosome-binding site and 5′ portion of yycF

<400>  156
agatctccag tgtcttgtgc tggttgtgag                                     30


<210>  157
<211>  32

<212>  DNA
<213>  Artificial sequence

<220>
<223>  Primer Bapori-1mB for amplification of signal peptide and Bap-B
       domain of the bap gene

<400>  157
ggatccttta ttttgaggtg agtaaatatg gg                                    32


<210>  158
<211>  44
<212>  DNA
<213>  Artificial sequence

<220>
<223>  Primer Bap-65c for amplifying the signal peptide and the Bap-B
       doamin of the bap gene

<400>  158
ggctctttaa ttgaattaga tgaagcacta ttttgtactt ccgc                       44


<210>  159
<211>  44
<212>  DNA
<213>  Artificial sequence

<220>
<223>  Primer Bap-65c for amplifying the signal peptide and the Bap-B
       domain of the bap gene

<400>  159
gcggaagtac aaaatagtgc ttcatctaat tcaattaaag agcc                       44


<210>  160
<211>  27
<212>  DNA
<213>  Artificial sequence

<220>
<223>  Primer Bap-63ck for amplifying the signal peptide and the bap-B
       domain of the bap gene

<400>  160
ggtaccggtg ccttctggtg aatttgg                                          27


<210>  161
<211>  30
<212>  DNA
<213>  Artificial sequence

<220>
<223>  Primer K_ClfA for amplifying the R region of clumping factor A

<400>  161
ggtaccgaaa ttgaaccaat tccagaggat                                       30


<210>  162

<211>  26
<212>  DNA
<213>  Artificial sequence

<220>
<223>  Primer ClfA_7cE for amplifying the R region fo clumping factor A

<400>  162
gaattcttac accctatttt ttcgcc                                          26


<210>  163
<211>  27
<212>  DNA
<213>  Artificial sequence

<220>
<223>  Primer Snap-Fw for amplifying the SNAP-Tag

<400>  163
ggtaccatgg acaaagattg cgaaatg                                         27


<210>  164
<211>  27
<212>  DNA
<213>  Artificial sequence

<220>
<223>  Primer Snap-Rv for amplifying the SNAP-Tag

<400>  164
   ggtacctccc agacccggtt tacccag                                          27